# EUROPEAN PATENT APPLICATION

(11) **EP 1 958 964 A2**
(43) Date of publication of application: **20.08.2008**
(21) Application number: 08005922.3
(22) Date of filing: 24.02.2005
(51) Int. Cl.: C07K 14/82, C12N 15/11, C12N 15/63, A61K 48/00, C12N 5/10

(54) **RAB9A, RAB11A, and modulators thereof related to infectious disease**

(30) Priority: 24.02.2004 US 547328 P
(62) Divisional of application: 05758743.8
(71) Applicant: THE GOVERNMENT OF THE UNITED STATES OF AMERICA, as represented by the Secretary, Department of Health and Human Services, Atlanta, Georgia 30341 (US); VANDERBILT UNIVERSITY, Nashville, TN 37212 (US)
(72) Inventor: Hodge, Thomas, W., Roswell, GA 30076 (US); McDonald, Natalie J., Atlanta, GA 30319-3929 (US); Rubin, Donald, Nashville, TN 37215 (US); Shaw, Michael W., Decatur, GA 30033-2200 (US); Sanchez, Antony, Lilburn, GA 30047 (US); Murray, James L., Athens, GA 30605 (US)
(74) Representative: Walton, Seán Malcolm

(57) **Abstract**

Reducing infection of a cell by a pathogen comprising decreasing activity or expression of Rab11A or of a Rab9A modulator in the cell, for example to treat or prevent a pathogen infection. Exemplary pathogens are those that use a Iipid raft. The disclosure also relates to methods of identifying agents involved in pathogen infection, such as modulators of Rab9A and Rab11A.

SEQUENCE LISTING

<110> The Government of The United States of America
      Vanderbilt University

<120> RAB9A, RAB11A, AND MODULATORS THEREOF RELATED TO INFECTIOUS
      DISEASE

<130> SMW/FP6541783

<140> EP
<141> 2005-02-24

<150> EP 05758743.8
<151> 2005-02-24

<150> PCT/US2005/006396
<151> 2005-02-24

<150> US 60/547,328
<151> 2004-02-24

<160> 66

<170> PatentIn version 3.3

<210> 1
<211> 838
<212> DNA
<213> Cercopithecus aethiops


<220>
<221> misc_feature
<222> (1)..(838)
<223> n is a, c, t, or g.

<400> 1
gggnaaccga gcggntaaca ttttcacaca gaaantcgga aagtaaagcc aatcttagag      60

gctgcaggag gtttgggggc agtatctgat tcagacgctg gctaacgttt cacgatcgcg     120

ttcccttttt tcttccaact cggtaagtaa aaaggcaaga tgagaaattt acgtgctgaa     180

cttaataaat agttggtgga cgtattgcct tttttttttt ttttttggta agggatgaca     240

catctcgtga ctacagttct tttgaggaat aacttttctg ctagtttcca aatcggcacg     300

tgaccaaagt cttttcatag gattttagcg tcctgataaa aatcaatggg cagaatttga     360

ttgcttttta aaaaatgtgt ttgtcctttg gtctctggca ccattgtaat ggaaaatccc     420

tacattgcct gtactctcag aagctgtcca gtggagcaaa actagagata aagaaacctg     480

gaacgattca gttaggaact tttaagaagc cagcctttag tttttccttt agaagattat     540

gcagttatca tgattgcttc tctagaactt cagtgtgtta tttggattcc taaatctaag     600

acaatgctgn ggaagtctgg ggcttttagn attttngggt ctgctgnaga aaatcctcgt     660

ttatactaca aagtttctnt tttggaactt tnggaattgg gcattttttn nnttattatt     720

ngnatgntng antnanngge aaaactnagn naaccctttt nggtttgcct cnanccggtt 780

nttaaanaaa ngggaaaaan cctnanttta aanttttttc cacccttttt tnttttnt 838

<210> 2
<211> 820
<212> DNA
<213> Cercopithecus aethiops

<220>
<221> misc_feature
<222> (1)..(820)
<223> n is a, c, t, or g.

<400> 2
ttgggganct agcttgccaa ntctacaggt ggggtctttc agtggggggc tgtcctgtag 60

gttatagaat gtttagcagc aaaaattaaa aattaaataa caaaaataaa aataaaaaag 120

aatgtttagc agcatccctg gcctctaccc actagatgtc agcagcacct cccttgcccc 180

caggtgtgaa ccaaaaatgc ctgcagacat tgccaaatat ctcctaggag gacaaaattg 240

tcctctcttc cacttgagaa ctattactct aaaattaccc agatctgctt tgaatccccg 300

ctccacccca tcacaacctg ggtcatcttg gaaaacagac tgaaccttcc tatgcccccc 360

gcaaattcct caactgtaac atggagctct tgctgaagaa atgctatgaa aattaaatga 420

aatgatgtac gtacaggatt tacacgcaca gaatattcac cgcgccagag tgagtgctca 480

ataaatggtc agaaatgagg ggaggctaaa aaaaaataat ttcgagaact caaaaatctt 540

atctttaggc ctccagagta ctgtagtcta gacagaagaa atggttgaga tagaancaaa 600

agagatgaga gaggttggaa aagaagtgat agaactaagg tattattccc cttatctctt 660

aagaacccgg cttggagtca aagccaatag agggtctact tagttttgnc tattactcta 720

ctttcaaata taacgaaaat tgcccaaacc caaagtntcc aaaaaaaact ttnnnttnan 780

cggggatttc tncncggncn aaaatctaan nccccnctnc 820

<210> 3
<211> 19
<212> DNA
<213> Homo sapiens

<400> 3
gggaagagtt cacttatga 19

<210> 4
<211> 19
<212> DNA
<213> artificial

<220>
<223>  a sequence considered complementary to SEQ ID NO: 3.

<400>  4
cccttcccaa gtgaatact                                                    19


<210>  5
<211>  19
<212>  RNA
<213>  Artificial

<220>
<223>  Rab9A siRNA

<400>  5
gggaagaguu cacuuauga                                                    19


<210>  6
<211>  19
<212>  RNA
<213>  Artificial

<220>
<223>  Rab9A siRNA

<400>  6
ggaaguggau ggacauuuu                                                    19


<210>  7
<211>  19
<212>  RNA
<213>  Artificial

<220>
<223>  Rab9A siRNA

<400>  7
ucacaaagcu uccagaacu                                                    19


<210>  8
<211>  19
<212>  RNA
<213>  Artificial

<220>
<223>  Rab9A siRNA

<400>  8
guaacaagau ugacauaag                                                    19


<210>  9
<211>  21
<212>  RNA
<213>  Artificial

<220>

<223>  Rab9A siRNA

<400>  9
aaguuugaua cccagcucuu c                                                21

<210>  10
<211>  18
<212>  DNA
<213>  Artificial

<220>
<223>  primer

<400>  10
tcctcattgc gcccagac                                                    18

<210>  11
<211>  24
<212>  DNA
<213>  Artificial

<220>
<223>  primer

<400>  11
atcacagtgc caggctcatt acag                                             24

<210>  12
<211>  25
<212>  DNA
<213>  Artificial

<220>
<223>  Fam-labeled probe

<400>  12
tttcgtgtgg ccgcgagaca ctctt                                            25

<210>  13
<211>  19
<212>  DNA
<213>  Artificial

<220>
<223>  primer

<400>  13
catcttctca acaacccta                                                   19

<210>  14
<211>  19
<212>  DNA
<213>  Artificial

<220>
<223>  primer

<400>  14
ccatctccaa ggagaatta                                                    19


<210>  15
<211>  21
<212>  DNA
<213>  Artificial

<220>
<223>  Fam-labeled probe

<400>  15
caatggcagg aaaatcatca c                                                 21


<210>  16
<211>  19
<212>  RNA
<213>  Artificial

<220>
<223>  Rab11A siRNA

<400>  16
guaggugccu uauugguuu                                                    19


<210>  17
<211>  19
<212>  RNA
<213>  Artificial

<220>
<223>  Rab11A siRNA

<400>  17
gcaacaaugu gguuccuau                                                    19


<210>  18
<211>  19
<212>  RNA
<213>  Artificial

<220>
<223>  Rab11A siRNA

<400>  18
caagagcgau aucgagcua                                                    19


<210>  19
<211>  19
<212>  RNA
<213>  Artificial

<220>
<223>  Rab11A siRNA

<400>  19
ggaguagagu uugcaacaa                                                    19


<210>  20
<211>  19
<212>  RNA
<213>  Artificial

<220>
<223>  PIKfyve siRNA

<400>  20
ggaaauagcu auaauccua                                                    19


<210>  21
<211>  19
<212>  RNA
<213>  Artificial

<220>
<223>  PIKfyve siRNA

<400>  21
gaacccagcu ccaucauug                                                    19


<210>  22
<211>  19
<212>  RNA
<213>  Artificial

<220>
<223>  PIKfyve siRNA

<400>  22
guagaaugcu ggccgauua                                                    19


<210>  23
<211>  19
<212>  RNA
<213>  Artificial

<220>
<223>  PIKfyve siRNA

<400>  23
ggaaaucucc ugcucgaaa                                                    19


<210>  24
<211>  19
<212>  RNA
<213>  Artificial

<220>
<223>  p40 siRNA

<400>  24

ccacagcugu ucauauuua                                                    19

<210>  25
<211>  19
<212>  RNA
<213>  Artificial

<220>
<223>  p40 siRNA

<400>  25
ggauucagcu gacaaagua                                                    19

<210>  26
<211>  19
<212>  RNA
<213>  Artificial

<220>
<223>  p40 siRNA

<400>  26
gaaaccagcu auaugucuu                                                    19

<210>  27
<211>  19
<212>  RNA
<213>  Artificial

<220>
<223>  p40 siRNA

<400>  27
ggaaaucgaa auugucuac                                                    19

<210>  28
<211>  19
<212>  RNA
<213>  Artificial

<220>
<223>  TIP47 siRNA

<400>  28
gcaaggcguu gaucagaag                                                    19

<210>  29
<211>  19
<212>  RNA
<213>  Artificial

<220>
<223>  TIP47 siRNA

<400>  29
ggaacagagc uacuucgua                                                    19

<210> 30
<211> 19
<212> RNA
<213> Artificial

<220>
<223> TIP47 siRNA

<400> 30
uggaauaugu ggcccagaa 19


<210> 31
<211> 17
<212> RNA
<213> Artificial

<220>
<223> TIP47 siRNA

<400> 31
gcuugugucg ucuaagg 17


<210> 32
<211> 21
<212> RNA
<213> Artificial

<220>
<223> TIP47 siRNA

<400> 32
aacagagcua cuucguacgu c 21


<210> 33
<211> 17
<212> DNA
<213> Artificial

<220>
<223> primer

<400> 33
attaacaatg gcaggaa 17


<210> 34
<211> 26
<212> DNA
<213> Artificial

<220>
<223> primer

<400> 34
gtgtcccttc tcccaccaac taatga 26

<210>  35
<211>  16
<212>  DNA
<213>  Artificial

<220>
<223>  primer

<400>  35
gtaaaacgac ggccag                                                    16


<210>  36
<211>  17
<212>  DNA
<213>  Artificial

<220>
<223>  primer

<400>  36
caggaaacag ctatgac                                                   17


<210>  37
<211>  22
<212>  DNA
<213>  Artificial

<220>
<223>  primer

<400>  37
gcgatgtcta ggttaggaag aa                                             22


<210>  38
<211>  24
<212>  DNA
<213>  Artificial

<220>
<223>  primer

<400>  38
gctatgtcct tgggtagtaa gcct                                           24


<210>  39
<211>  20
<212>  DNA
<213>  Artificial

<220>
<223>  primer

<400>  39
gctctgagac aatgaacgct                                                20

<210>  40
<211>  21
<212>  DNA
<213>  Artificial

<220>
<223>  primer

<400>  40
aaagagataa tctggctgtg c                                                21

<210>  41
<211>  26
<212>  DNA
<213>  Artificial

<220>
<223>  primer

<400>  41
ggactttaag ggttacttgg gttgcc                                           26

<210>  42
<211>  25
<212>  DNA
<213>  Artificial

<220>
<223>  primer

<400>  42
cattttgatc atcatgtatg cttct                                            25

<210>  43
<211>  21
<212>  DNA
<213>  Artificial

<220>
<223>  primer

<400>  43
cagtacacct gccacaaagg a                                                21

<210>  44
<211>  21
<212>  DNA
<213>  Artificial

<220>
<223>  primer

<400>  44
gtgtgacctt ctctgcagac a                                                21

<210>  45

<211>  21
<212>  DNA
<213>  Artificial

<220>
<223>  primer

<400>  45
gacatggaga agatctggca c                                                21


<210>  46
<211>  21
<212>  DNA
<213>  Artificial

<220>
<223>  primer

<400>  46
tccagacgca ggatggcgtg a                                                21


<210>  47
<211>  40
<212>  RNA
<213>  Artificial

<220>
<223>  Rab9A antisense RNA

<400>  47
gcaugguaac aaaaugucca uccacuucca aaucuuuauu                            40


<210>  48
<211>  40
<212>  RNA
<213>  Artificial

<220>
<223>  Rab9A antisense RNA

<400>  48
guuacuuaag uucuggaagc uuugugaauc aucgacacua                            40


<210>  49
<211>  40
<212>  RNA
<213>  Artificial

<220>
<223>  Rab11A antisense

<400>  49
ggaugcuucu uguugcaaac ucuacuccaa uggugcucuu                            40


<210>  50
<211>  40

<212>  RNA
<213>  Artificial

<220>
<223>  Rab11A antisense

<400>  50
gcugauguua uagcucgaua ucgcucuugc ccugcugugu                              40


<210>  51
<211>  40
<212>  RNA
<213>  Artificial

<220>
<223>  PIKfyve antisense RNA

<400>  51
ggcugaucua uuucgagcag gagauuuccc agcauccucu                              40


<210>  52
<211>  40
<212>  RNA
<213>  Artificial

<220>
<223>  PIKfyve antisense RNA

<400>  52
agcaaaagca augauggagc uggguuccuu cucgcagacu                              40


<210>  53
<211>  40
<212>  RNA
<213>  Artificial

<220>
<223>  p40 antisense RNA

<400>  53
auucaggacu uguagacaau uucgauuucc ugauugguug                              40


<210>  54
<211>  40
<212>  RNA
<213>  Artificial

<220>
<223>  p40 antisense RNA

<400>  54
uccgccccca aagacauaua gcugguuucc aauggcugcc                              40


<210>  55
<211>  40
<212>  RNA

<213>  Artificial

<220>
<223>  TIP47 antisense RNA

<400>  55
ccgacaccuu agacgacaca agcuccuugg uguccgccag                              40


<210>  56
<211>  40
<212>  RNA
<213>  Artificial

<220>
<223>  TIP47 antisense RNA

<400>  56
ggagcccaga cguacgaagu agcucuguuc cugccgcugc                              40


<210>  57
<211>  46
<212>  RNA
<213>  Artificial

<220>
<223>  Rab9A ribozyme RNA

<400>  57
ggaaucgcac cucugaugag uccgugagga cgaaaccugc cguguc                       46


<210>  58
<211>  46
<212>  RNA
<213>  Artificial

<220>
<223>  Rab9A ribozyme RNA

<400>  58
uugugaauca uccugaugag uccgugagga cgaaacacua aaagua                       46


<210>  59
<211>  46
<212>  RNA
<213>  Artificial

<220>
<223>  Rab11A ribozyme RNA

<400>  59
cgaguaaauc gacugaugag uccgugagga cgaaacagga gauuac                       46


<210>  60
<211>  46
<212>  RNA
<213>  Artificial

<220>
<223>  Rab11A ribozyme RNA

<400>  60
uuguugcuug gacugaugag uccgugagga cgaaacaugu cauuuu                    46


<210>  61
<211>  46
<212>  RNA
<213>  Artificial

<220>
<223>  PIKfyve ribozyme RNA

<400>  61
gaucuaucca gucugaugag uccgugagga cgaaacaggu uaguaa                    46


<210>  62
<211>  46
<212>  RNA
<213>  Artificial

<220>
<223>  PIKfyve ribozyme RNA

<400>  62
uuccuucucg cacugaugag uccgugagga cgaaacugca augggc                    46


<210>  63
<211>  46
<212>  RNA
<213>  Artificial

<220>
<223>  p40 ribozyme RNA

<400>  63
ucaggacuug uacugaugag uccgugagga cgaaacaauu ucgauu                    46


<210>  64
<211>  46
<212>  RNA
<213>  Artificial

<220>
<223>  p40 ribozyme RNA

<400>  64
uccgccccca aacugaugag uccgugagga cgaaacauau agcugg                    46


<210>  65
<211>  46
<212>  RNA
<213>  Artificial

<220>
<223>  TIP47 ribozyme RNA

<400>  65
gacaccuuag accugaugag uccgugagga cgaaacacaa gcuccu                    46


<210>  66
<211>  46
<212>  RNA
<213>  Artificial

<220>
<223>  TIP47 ribozyme RNA

<400>  66
acagggagcc cacugaugag uccgugagga cgaaacguac gaagua                    46

## Description

### CROSS REFERENCE TO RELATED APPLICATIONS

This application claims the benefit of U.S. Provisional Application No. 60/547,328 filed February 24, 2004, and the benefit of PCT/US2003/037143 filed November 18, 2003, which claims the benefit of U.S. Provisional Application Nos. 60/427,464 filed November 18, 2002 and 60/482,604 filed June 25, 2003, all herein incorporated by reference.

### FIELD

The present disclosure relates to methods of reducing the activity of Rab9A or Rab11A, for example by reducing the activity of a modulator thereof that increases Rab9A or Rab 11A activity, to treat or reduce infection or other stage of the life cycle of a pathogen, such as a virus. The disclosure also relates to methods of identifying agents involved in pathogen infection, such as modulators of Rab9A and Rab11A.

### BACKGROUND

Infectious diseases affect the health of people and animals around the world, causing serious illness and death. Public health efforts have focused on behavioral modification and other public health efforts to reduce the incidences of infection, while treatment regimens for these diseases have focused on pharmaceuticals, such as antibiotics and anti-viral medications. However, educating people about modifying behavior can be difficult, and that approach alone rarely can significantly diminish the incidence of infection. Furthermore, modifying the behavior of domestic or wild animals would not result in diminished infections. Stopping the spread of infections in an animal population typically involves wholesale slaughter. Few vaccines are available or wholly effective, and they tend to be specific for particular conditions.

The rate of HIV (human immunodeficiency virus) infection is increasing. HIV and its associated acquired immune deficiency syndrome (AIDS) accounted for approximately 5% of all deaths in the United States in the year 2000, while over 313,000 persons were reported to be living with AIDS in that same year. Centers for Disease Control and Prevention, HIV/AIDS Surveillance Supplemental Report, 8(1):1-22 (2002). These increasing infection rates have occurred, even though the mode of HIV infection has been known for almost 20 years, and educational programs around the world have promoted behavioral modifications meant to reduce HIV infection. Incidence and death rates due to HIV disease have been decreasing since the mid-90's, in part due to aggressive antiviral therapies, which frequently have toxic side effects and strict dosage schedules. However, even with treatment, the patient is not cured of the disease, and to date, no effective vaccine therapy has been found.

In other diseases, such as infection by the Ebola virus, not only are treatments limited, but containment or prevention of infections is difficult because the life cycle of the virus is not well known. The natural reservoir for the Ebola virus, that is the place or population in nature where the virus resides between human outbreaks, has not yet been identified.

Additionally, different viral strains can rapidly evolve in response to drug usage, producing drug-resistant strains. For example, strains of the influenza virus resistant to amantadine and rimantadine have recently arisen. A recent study of 80 newly-infected people conducted by the AIDS Research Center at Rockefeller University in New York, found that as many as 16.3% of these individuals had strains of HIV associated with resistance to some treatments, and 3.8% appeared to be resistant to several currently available anti-HIV drugs. Thus, a need exists for alternative treatments for infectious disease and methods of identifying new drugs to combat infectious disease.

### SUMMARY

Several host nucleic acid sequences involved in viral infection were identified using gene trap methods. The identification of these host sequences and their encoded products permits the identification of sequences that can be targeted for therapeutic intervention. The genomics-based discovery of nucleic acids and proteins involved in, or even required for, infection provides a new paradigm for identifying and validating various aspects of infectious disease, including assessing individual or population resistance to infection and finding novel diagnostic and drug targets for infectious disease and altering the nucleotide sequence of the host nucleic acid sequence.

Once of those host sequences encodes a Ras oncogene family member, Rab9A (including the target sequences associated with SEQ ID NOS: 1-2, and the proteins encoded thereby, as well as variants, fusions, and fragments thereof that retain Rab9A biological activity). Rab11A is involved with trafficking of proteins from the trans-Golgi to the cell surface. Rab9A, Rab11A, and modulators thereof are shown herein to play a role in infection by a variety of pathogens. In particular examples, Rab9A, Rab11A, and modulators thereof, are involved in infection by pathogens that use similar pathways for morphogenesis of infectious particles, such as pathogens that can hijack a lipid raft. Rab9A, Rab11A, or a modulator thereof can mediate infection, and in some examples is required for infection by a pathogen.

Based on the observation that Rab9A, Rab11A, and modulators thereof are involved in pathogenic infection, provided herein are methods for decreasing infection of a host cell by a pathogen, such as a pathogen that can hijack a lipid raft, or treating such an infection, by interfering with the activity of Rab9A or Rab 11A, for example by decreasing the activity of one or more modulators thereof that increase Rab9A or Rab11A activity, or by increasing the activity of one or more modulators thereof that decrease Rab9A or Rab11A activity. Methods are disclosed herein for decreasing infection of a host cell by a pathogen, for example inhibiting infection of a host cell by a pathogen. In particular examples, decreasing infection does not require a 100% decrease in infection. For example, a decrease of at least 50%, at least 75%, at least 90%, or at least 98% reduction (for example as compared to an amount of infection in the absence of the therapeutic agent) can be sufficient.

In particular examples, the methods include decreasing (such as inhibiting) the biological activity of Rab9A or Rab11A in the host cell, for example by decreasing the activity of one or more modulators thereof that increase Rab9A or Rab11A activity, or by increasing the activity of one or more modulators thereof that decrease Rab9A or Rab11A activity. A decrease or elimination of such biological activity decreases (for example inhibits) infection of the host cell by the pathogen. The method can include decreasing activity of one or more Rab9A or Rab11A modulators, such as two or more, three or more, or one to four Rab9A or Rab11A modulators that increase Rab9A or Rab11A activity. Similarly, the method can include increasing activity of one or more Rab9A or Rab 11A modulators, such as two or more, three or more, or one to four Rab9A or Rab11A modulators that decrease Rab9A or Rab11A activity. In some examples, the activity of Rab9A, Rab11A, and one or more modulators thereof that increase Rab9A or Rab11A activity, is decreased.

Methods of decreasing the biological activity of a nucleic acid or protein sequence are known in the art. In one example, the method includes decreasing (and in some examples eliminating) an interaction between a protein of the pathogen and a Rab9A protein, Rab11A protein, or a modulator protein thereof. For example, by decreasing (and in some examples eliminating) expression of Rab9A or Rab11A (for example by decreasing expression of a modulator thereof that stimulates or enhances the activity of Rab9 or Rab 11A), the interaction between the pathogen protein and the Rab9A protein, Rab11A protein, or modulator protein thereof, will be reduced. In one example, protein expression is reduced by decreasing an amount of mRNA encoding Rab9A, Rab11A, or a modulator thereof that increases Rab9A or Rab11A activity, for example by contacting the mRNA with an antisense RNA, ribozyme, microRNA, triple helix molecule, or siRNA that recognizes the mRNA. Another method that can be used is administering an agent that decreases binding between a Rab9A protein, Rab11A protein, or a modulator protein thereof, to a pathogen protein, such as an antibody that specifically binds to Rab9A, Rab11A, or a modulator thereof. Such methods can be used to decrease, inhibit, or even prevent, infection of the host cell by the associated pathogen.

Methods of treating a pathogen infection in a subject are disclosed. In particular examples, the method includes administering to a subject having a pathogen infection a therapeutically effective amount of one or more agents that interfere with the activity of Rab9A or Rab11A (for example by administration of an agent that decreases the activity of or one or more modulators thereof that increase Rab9A or Rab11A activity, or by administration of an agent that increases the activity of a modulator that decreases Rab9A or Rab11A activity). In one example, the agent interferes with the interaction of a pathogen protein and a Rab9A protein, a Rab11A protein, or one or more modulator proteins of Rab9A or Rab11A. In another example, the method includes administering to a subject having a pathogen infection a therapeutically effective amount of one or more agents that decreases an amount of mRNA encoding Rab9A or Rab11A in a cell (for example an agent that decreases an amount of mRNA encoding a modulator that increases Rab9A or Rab11A activity, or an agent that increases an amount of mRNA encoding a modulator that decreases Rab9A or Rab11A activity). In some examples, such methods induce a prophylactic effect or a therapeutic effect in the subject.

Also provided are methods for identifying agents that can decrease infection of a host cell by a pathogen, such as a pathogen that hijacks a lipid raft. In particular examples, the method identifies a compound that decreases binding of a pathogen protein to a Rab9A protein, Rab11A protein, or a modulator protein thereof. The method can include contacting a Rab9A protein, Rab11A protein, or modulator protein thereof, with a pathogen protein and one or more test compounds, then determining whether binding of the pathogen protein to the Rab9A protein, the Rab11A protein, the Rab9A modulator, or the Rab11A modulator protein, is decreased in the presence of the test compound. A decrease in binding indicates that the test compound decreases pathogen infection.

Methods for identifying a modulator of Rab9A or Rab11A that is involved in infection by a pathogen, such as a pathogen that hijacks a lipid raft, are disclosed. In particular examples, the method includes decreasing expression of a putative Rab9A or a Rab11A modulator in a cell that expresses Rab9A or Rab11A, contacting the cell with a pathogen (such as a pathogen that hijacks a lipid raft), and then detecting an amount of infection by the pathogen. A decrease (and in some examples complete elimination) in infection indicates that the putative modulator of Rab9A or Rab11A is a modulator of Rab9A or Rab11B that increases Rab9A or Rab11A activity and is involved in infection by the pathogen, respectively. An increase (and in some examples no detectable change) in infection indicates that the putative modulator of Rab9A or Rab11A is a modulator of Rab9A or Rab11B that decreases Rab9A or Rab11A activity and is involved in infection by the pathogen, respectively.

Agents identified using the disclosed methods can be used therapeutically or prophylactically, for example administered to a subject to decrease infection, prevent a future infection, or treat an existing infection.

Cells and non-human mammals are provided that have decreased susceptibility to infection by a pathogen, such as a pathogen that hijacks a lipid raft. In particular examples, such cells and mammals include a functional deletion of a Rab9A gene, Rab11A gene, or a modulator gene thereof.

Also provided herein are methods of screening subjects for resistance or susceptibility to infection by characterizing the subject's Rab9A, Rab11A, Rab9A modulator, or Rab11A modulator nucleic acid or amino acid sequence.

Particular exemplary isolated nucleic acid sequences that can reduce expression of Rab9A, Rab11A, or modulators thereof (such as those that increase Rab9A or Rab11A activity) are disclosed herein. In one example, such an isolated nucleic acid sequence includes any nucleic acid sequence shown in SEQ ID NOS: 5-9, 16-32, or 47-66. However, variants, fragments, and fusions of such sequences are encompassed by this disclosure, as long as their ability to reduce mRNA expression is not decreased by more than 50%. Such isolated nucleic acid sequences can be part of a vector. Also provided by the present disclosure are cells including such isolated nucleic acid sequences and vectors, such as a mammalian cell.

The foregoing and other objects, features, and advantages of the disclosure will become more apparent from the following detailed description, which proceeds with reference to the accompanying figures.

### BRIEF DESCRIPTION OF THE DRAWINGS

**FIG. 1A** is a bar graph showing the relative amount of p24 in HIV-infected cells in the presence of various siRNAs. This figure demonstrates that Rab9A mRNA degradation with siRNA decreases HIV replication. Rab9A; KOX4 (similar to KOX4 (LOC131880) and LOC166140); KIAA 1259; F3 (tissue factor 3; thromboplastin); AXL (AXL receptor tyrosine kinase); SelB (the elongation factor for selenoprotein translation); FKBP38.
**FIG. 1B** is a bar graph showing the relative amount ofluciferase activity measured in cells, which corresponds to the amount of HIV infection (n=5).
**FIG. 1C** is a bar graph showing the amount of Rab9A mRNA present in cells following transfection with Rab9A siRNAs.
**FIG.** 2 is a bar graph showing the relative amount of p24 in HIV-infected cells in the presence of Rab9A, Rab11A, and Rab9A modulator siRNAs. This figure demonstrates that Rab9A, Rab11A, or Rab9A modulator mRNA degradation with siRNA decreases HIV replication.
**FIG. 3A** is a nucleotide sequence alignment between human Rab9A cDNA (Genbank accession no. NM_004251), and simian Rab9A cDNA (Genbank accession no. AY601640). Nonconserved nucleotides are indicated.
**FIG. 3B** is a bar graph showing the amount of Rab9A mRNA present in Vero cells following transfection with Rab9A siRNAs.
**FIG. 3C** is a bar graph showing the quantitation of Ebola and Marburg viral antigens secreted into culture supernatants (6 days post-infection) in the presence or absence of Rab9A siRNA.
**FIG. 4** is a bar graph showing the reduction in titers of MV released into culture medium following infection of siRNA transfected Vero cells. Data points represent average viral titers determined from three independent experiments. 1 x 10⁵ pfu/mL (day 2) and 1 x 10⁶ pfu/mL (day 5) were detected from lipofectamine-only transfectants.
**FIG. 5** is a schematic drawing showing a model of Rab9A, Rab11A, and Rab9A modulators, involvement in lipid raft formation.

### SEQUENCE LISTING

The nucleotide sequences described herein are shown using standard letter abbreviations for nucleotide bases. Only one strand of each nucleic acid sequence is shown, but the complementary strand is understood as included by any reference to the displayed strand. Additionally, the nucleic acid sequences shown in SEQ ID NOS: 1-2 inherently disclose the corresponding polypeptide sequences of coding sequences (resulting translations of the nucleotide sequences), even when those polypeptide sequences are not explicitly provided herein.

The siRNA sequences show the siRNA sequence of the sense strand; the opposite strand is hybridized to the sense strand to form the therapeutic siRNA molecules. These siRNA sequences (except SEQ ID NOS: 9 and 32) have UU as 3' overhangs on each strand (not shown) and 5'-phosphate on the anitsense strand only.

SEQ ID NO: 1 is a nucleic acid sequence associated with pathogen infection, and is the clone identified as Nucleotide Sequence MV3-E5, distal end. The human homolog is the (+) strand of GenBank Accession No. AC079383.17, Ras oncogene family member Rab9A.

SEQ ID NO: 2 is a nucleic acid sequence associated with pathogen infection, and is the clone identified as Nucleotide Sequence MV3-E5, proximal end. The human homolog is the (-) strand of GenBank Accession No. AC079383.17, Ras oncogene family member Rab9A.

SEQ ID NOS: 3-4 are exemplary sequences that are considered complementary.

SEQ ID NOS: 5-9 are exemplary Rab9A siRNA sequences.

SEQ ID NOS: 10-11 are primers used to detect human Rab9A cDNA.

SEQ ID NO: 12 is Fam-labeled probe used to detect human Rab9A cDNA.

SEQ ID NOS: 13-14 are primers used to detect simian Rab9A cDNA.

SEQ ID NO: 15 is Fam-labeled probe used to detect simian Rab9A cDNA.

SEQ ID NOS: 16-19 are exemplary Rab11A siRNA sequences.

SEQ ID NOS: 20-23 are exemplary human PIKfyve siRNA sequences.

SEQ ID NOS: 24-27 are exemplary human p40 siRNA sequences.

SEQ ID NOS: 28-32 are exemplary human TIP47 siRNA sequences.

SEQ ID NOS: 33-36 are primers used to clone simian Rab9A.

SEQ ID NOS: 37-38 are primers that can be used to detect RSV.

SEQ ID NOS: 39-40 are primers that can be used to detect mouse interferon-γ.

SEQ ID NOS: 41-42 are primers that can be used to detect mouse interleukin-10.

SEQ ID NOS: 43-44 are primers that can be used to detect mouse interleukin-12.

SEQ ID NOS: 45-46 are primers that can be used to detect mouse β-actin.

SEQ ID NOS: 47-48 are exemplary human Rab9A antisense RNA sequences.

SEQ ID NOS: 49-50 are exemplary human Rab11A antisense RNA sequences.

SEQ ID NOS: 51-52 are exemplary human PIKfyve antisense RNA sequences.

SEQ ID NOS: 53-54 are exemplary human p40 antisense RNA sequences.

SEQ ID NOS: 55-56 are exemplary human TIP47 antisense RNA sequences.

SEQ ID NOS: 57-58 are exemplary human Rab9A ribozyme RNA sequences.

SEQ ID NOS: 59-60 are exemplary human Rab11A ribozyme RNA sequences.

SEQ ID NOS: 61-62 are exemplary human PIKfyve ribozyme RNA sequences.

SEQ ID NOS: 63-64 are exemplary human p40 ribozyme RNA sequences.

SEQ ID NOS: 65-66 are exemplary human TIP47 ribozyme RNA sequences.

### DETAILED DESCRIPTION

### Abbreviations and Terms

The following explanations of terms and methods are provided to better describe the present disclosure and to guide those of ordinary skill in the art in the practice of the present disclosure. The singular forms "a," "an," and "the" refer to one or more than one, unless the context clearly dictates otherwise. For example, the term "comprising a modulator thereof" includes single or plural Rab9A or Rab11A modulators and is considered equivalent to the phrase "comprising at least one Rab9A or at least one Rab11A modulator" or to the phase "comprising one or more Rab9A or Rab11A modulators." The term "or" refers to a single element of stated alternative elements or a combination of two or more elements, unless the context clearly indicates otherwise. For example, the phrase "Rab9A, Rab11A, or a modulator thereof" refers to Rab9A, Rab11A, one or more Rab9A modulators, one or more Rab11A modulators, or combinations thereof. As used herein, "comprises" means "includes." Thus, "comprising A or B," means "including A, B, or A and B," without excluding additional elements.

Unless explained otherwise, all technical and scientific terms used herein have the same meaning as commonly understood to one of ordinary skill in the art to which this disclosure belongs. Although methods and materials similar or equivalent to those described herein can be used in the practice or testing of the present disclosure, suitable methods and materials are described below. The materials, methods, and examples are illustrative only and not intended to be limiting.

All sequences provided in the disclosed Genbank Accession numbers are incorporated herein by reference.
DNA = deoxyribonucleic acid
ds = double-stranded (for example, dsDNA)
PCR = polymerase chain reaction
RNA = ribonucleic acid
mRNA = messenger RNA
MOI = multiplicity of infection
RSV = respiratory syncytia virus
siRNA = short interfering or interrupting RNA
ss = single-stranded (for example, ssDNA)
µg = microgram
µl = microliter

**Activity:** Refers to the native biological activity of a molecule, such as a Rab9A, Rab11A, Rab9A modulator, or Rab11A modulator nucleic acid or protein sequence. Methods of interfering with the biological activity of a molecule are known in the art, and include, but are not limited to, decreasing expression of a protein or nucleic acid sequence (such as decreasing transcription or translation), as well as decreasing the interaction between a desired molecule and its target (such as a pathogen, for example a pathogen protein). Methods of increasing the biological activity of a molecule are also known in the art, and include, but are not limited to, increasing expression of a protein or nucleic acid sequence (such as increasing transcription or translation), as well as increasing the interaction between a desired molecule and its target.

**Antisense, Sense, and Antigene:** Antisense molecules are molecules that are specifically hybridizable or specifically complementary to either RNA or the plus strand of DNA. Sense molecules are molecules that are specifically hybridizable or specifically complementary to the minus strand of DNA. Antigene molecules are either antisense or sense molecules directed to a particular dsDNA target. These molecules can be used to interfere with gene expression, such as expression of Rab9A, Rab11A, or a modulator thereof.

**cDNA (complementary DNA):** A piece of DNA lacking internal, non-coding segments (introns) and transcriptional regulatory sequences. A cDNA also can contain untranslated regions (UTRs) that are responsible for translational control in the corresponding RNA molecule. cDNA can be produced using various methods, such as synthesis in the laboratory by reverse transcription from messenger RNA extracted from cells.

**Complementary:** Complementary binding occurs when the base of one nucleic acid molecule forms a hydrogen bond the base of another nucleic acid molecule. Normally, the base adenine (A) is complementary to thymidine (T) and uracil (U), while cytosine (C) is complementary to guanine (G). For example, the sequence 5'-ATCG-3' of one ssDNA molecule can bind to 3'-TAGC-5' of another ssDNA to form a dsDNA.

Nucleic acid molecules can be complementary to each other even without complete hydrogen-bonding of all bases of each molecule. By way of example only (and without limitation), the ssDNA: 5'-GGGAAGAGTTCACTTATGA-3' (SEQ ID NO: 3) is considered complementary to the ssDNA 3'-CCCTTCCCAAGTGAATACT-5' (SEQ ID NO: 4) even though there is a mismatched base pair (A-C rather than A-T or G-C) at the seventh position.

**Conservative substitution:** A substitution of an amino acid residue for another amino acid residue having similar biochemical properties. Typically, conservative substitutions have little to no impact on the biological activity of a resulting polypeptide. In a particular example, a conservative substitution is an amino acid substitution in a peptide that does not substantially affect the biological function of the peptide. A peptide can include one or more amino acid substitutions, for example 2-10 conservative substitutions, 2-5 conservative substitutions, 4-9 conservative substitutions, such as 2, 5 or 10 conservative substitutions.

For example, a conservative substitution in a Rab9A peptide is one that does not substantially affect the ability of Rab9A to confer resistance to infection by a pathogen, such as a pathogen that can hijack a lipid raft, for example HIV, Marburg, Measles, RSV, or Ebola virus.

A peptide can be produced to contain one or more conservative substitutions by manipulating the nucleotide sequence that encodes that peptide, for example using procedures known in the art, such as site-directed mutagenesis or PCR or peptide synthesis methods. An alanine scan can be used to identify which amino acid residues in a protein can tolerate an amino acid substitution. In one example, the biological activity of the protein is not decreased by more than 25%, for example not more than 20%, for example not more than 10%, when an alanine, or other conservative amino acid (such as those listed below), is substituted for one or more native amino acids.

Examples of amino acids which can be substituted for an original amino acid in a protein and which are regarded as conservative substitutions include, but are not limited to: Ser for Ala; Lys for Arg; Gln or His for Asn; Glu for Asp; Ser for Cys; Asn for Gln; Asp for Glu; Pro for Gly; Asn or Gln for His; Leu or Val for Ile; Ile or Val for Leu; Arg or Gln for Lys; Leu or Ile for Met; Met, Leu or Tyr for Phe; Thr for Ser; Ser for Thr; Tyr for Trp; Trp or Phe for Tyr; and Ile or Leu for Val.

Further information about conservative substitutions can be found in, among other locations in, Ben-Bassat et al., (J. Bacteriol. 169:751-7, 1987), O'Regan et al., (Gene 77:237-51, 1989), Sahin-Toth et al., (Protein Sci. 3:240-7, 1994), Hochuli et al., (Bio/Technology 6:1321-5, 1988) and in standard textbooks of genetics and molecular biology.

**Decrease:** To reduce the amount or activity of something, for example as compared to a control. When the term "decrease" is used herein, a 100% decrease is not required. Therefore, the term can refer to decreases of at least 20%, at least 40%, at least 50%, at least 60%, at least 75%, at least 80%, at least 90%, at least 95%, at least 98%, or even at least 100%. In particular examples, the amount of decrease is compared to a control, such as a sample or subject not receiving a therapeutic or prophylactic agent.

**Ebola virus:** A highly contagious hemorrhagic virus named after a river in the Democratic Republic of the Congo (formerly Zaire) in Africa, where it was first recognized. Ebola is one of two members of a family of RNA viruses called Filoviridae. There are four identified subtypes of Ebola virus. Ebola-Zaire, Ebola-Sudan, and Ebola-Ivory Coast have caused disease in humans. Ebola-Reston has caused disease in nonhuman primates, but not in humans.

Ebola hemorrhagic fever (Ebola HF) is a severe, often fatal disease in humans and nonhuman primates (for example, monkeys, gorillas, and chimpanzees) that is caused by Ebola virus infection. Early symptoms of Ebola infection can include red eyes and a skin rash. Antigen-capture enzyme-linked immunosorbent assay (ELISA) testing, IgM ELISA, PCR, and virus isolation can be used to diagnose a case of Ebola HF within a few days after the onset of symptoms. Subjects tested later in the course of the disease, or after recovery, can be tested for IgM and IgG antibodies. The disease also can be diagnosed by using immunohistochemistry testing, virus isolation, or PCR.

**Encodes:** Unless evident from its context, includes DNA sequences that encode a peptide, as the term is typically used, as well as DNA sequences that are transcribed into inhibitory antisense molecules.

**Enveloped RNA virus:** A virus whose genome includes RNA (such as a plus or minus RNA strand), and can derive an envelope from the host. The viral envelope contains the lipid and protein constituents of the membrane from which it is derived. In particular examples, the envelope is derived from the host cell plasma membrane (as in the case of HIV), from the host nuclear membrane (as in the case of herpesviruses), or from the host Golgi body (as in the case of vaccinia).

Particular examples of positive-strand RNA viruses that have an envelope include, but are not limited to: Togaviruses (examples of which include rubella); alphaviruses (such as Western equine encephalitis virus, Eastern equine encephalitis virus, and Venezuelan equine encephalitis virus); Flaviviruses (examples of which include Dengue virus, West Nile virus, Japanese encephalitis virus, and hepatitis C virus); and Coronaviruses (such as SARS).

A specific example of a positive-strand RNA virus is a retrovirus. Retroviruses genomes consist of two molecules of RNA, which are single stranded, (+) sense and have 5' cap and 3' poly-(A) (equivalent to mRNA). Particular examples of retroviruses include, but are not limited to: human immunodeficiency virus type 1 (HIV-1), HIV-2; equine infectious anemia virus; feline immunodeficiency virus (FIV); feline leukemia viruses (FeLV); simian immunodeficiency virus (SIV); and avian sarcoma virus.

Exemplary negative-strand RNA viruses that have an envelope include, but are not limited to: Orthomyxoviruses (such as influenza virus), Rhabdoviruses (such as Rabies virus), Paramyxoviruses (examples of which include measles virus, mumps virus, respiratory syncytial virus), and Filoviruses such as Marburg and Ebola.

**Expression:** With respect to a gene sequence, refers to transcription of the gene and, as appropriate, translation of the resulting mRNA transcript to a protein. Thus, expression of a protein coding sequence results from transcription and translation of the coding sequence.

**Functional deletion:** A mutation, partial or complete deletion, insertion, or other variation made to a gene sequence that inhibits production of the gene product or renders the gene product non-functional. For example, a functional deletion of a Rab9A gene in a cell results in a cells having non-functional Rab9A protein, which results in the cell having increased resistance to infection by a pathogen, such as a pathogen that hijacks a lipid raft.

**Gene:** A nucleic acid sequence that encodes a peptide under the control of a regulatory sequence, such as a promoter or operator. A gene includes an open reading frame encoding a peptide, as well as exon and (optionally) intron sequences. An intron is a DNA sequence present in a given gene that is not translated into protein and is generally found between exons. The coding sequence of the gene is the portion transcribed and translated into a peptide *(in vivo, in vitro* or *in situ*) when placed under the control of an appropriate regulatory sequence. The boundaries of the coding sequence can be determined by a start codon at the 5' (amino) terminus and a stop codon at the 3' (carboxyl) terminus. If the coding sequence is to be expressed in a eukaryotic cell, a polyadenylation signal and transcription termination sequence can be included 3' to the coding sequence.

Transcriptional and translational control sequences include, but are not limited to, DNA regulatory sequences such as promoters, enhancers, and terminators that provide for the expression of the coding sequence, such as expression in a host cell. A polyadenylation signal is an exemplary eukaryotic control sequence. A promoter is a regulatory region capable of binding RNA polymerase and initiating transcription of a downstream (3' direction) coding sequence. Additionally, a gene can include a signal sequence at the beginning of the coding sequence of a protein to be secreted or expressed on the surface of a cell. This sequence can encode a signal peptide, N-terminal to the mature polypeptide, which directs the host cell to translocate the polypeptide.

**Host Cell:** Any cell that can be infected with a pathogen, such as a virus. A host cell can be prokaryotic or eukaryotic, such as a cell from an insect, crustacean, mammal, bird, reptile, yeast, or a bacterium such as *E. coli.* Exemplary host cells include, but are not limited to, mammalian B-lymphocyte cells.

The host cell can be part of an organism (such as a human or veterinary subject), or part of a cell culture, such as a culture of mammalian cells or a bacterial culture. A host nucleic acid molecule is a nucleic acid molecule present in a host cell that expresses a host protein. Decreasing or inhibiting the interaction between a Rab9A, Rab11A, or a modulator thereof (such as a modulator that increases Rab9A or Rab11A activity), peptide or nucleic acid molecule and a pathogen (such as a pathogen protein), can occur *in vitro, in vivo,* and *in situ* environments.

**Human Immunodeficiency Virus (HIV):** A retrovirus that causes immunosuppression in humans and leads to a disease complex known as acquired immunodeficiency syndrome (AIDS). This immunosuppression results from a progressive depletion and functional impairment of T lymphocytes expressing the CD4 cell surface glycoprotein. Depletion of CD4 T cells results from the ability of HIV to selectively infect, replicate in, and ultimately destroy these T cells (for example see Klatzmann *et al., Science* 225:59, 1984). CD4 itself is an important component, and in some examples an essential component, of the cellular receptor for HIV.

HIV subtypes can be identified by particular number, such as HIV-1 and HIV-2. In the HIV life cycle, the virus enters a host cell in at least three stages: receptor docking, viral-cell membrane fusion, and particle uptake (D'Souza et al., JAMA 284:215, 2000). More detailed information about HIV can be found in Coffin et al., Retroviruses (Cold Spring Harbor Laboratory Press, 1997).

HIV can be diagnosed in a subject using routine methods, such as quantitative PCR methods to measure the amount of HIV virus present in an infected individual, and antibody assays (such as an ELISA assay or Western blot) to determine whether HIV antibodies are present in a subject's blood.

Early symptoms of HIV infection are usually what would be observed for any viral infection, such as one or more of the following: fever, headache, tiredness, and enlarged lymph nodes. Later symptoms of HIV infection, including development of AIDS, can include one or more of the following: lack of energy, weight loss, frequent fevers and sweats, persistent or frequent yeast infections (oral or vaginal), persistent skin rashes or flaky skin, pelvic inflammatory disease in women that does not respond to treatment, and short-term memory loss.

**Hybridization:** Hybridization of a nucleic acid molecule occurs when two complementary nucleic acid molecules undergo an amount of hydrogen bonding to each other. Hybridization conditions resulting in particular degrees of stringency will vary depending upon the nature of the hybridization method and the composition and length of the hybridizing nucleic acid sequences. Generally, the temperature of hybridization and the ionic strength (such as the Na+ concentration) of the hybridization buffer will determine the stringency of hybridization. Calculations regarding hybridization conditions for attaining particular degrees of stringency are discussed in Sambrook et al., (1989) Molecular Cloning, second edition, Cold Spring Harbor Laboratory, Plainview, NY (chapters 9 and 11) and Tijssen, Laboratory Techniques in Biochemistry and Molecular Biology-Hybridization with Nucleic Acid Probes Part I, Chapter 2 (Elsevier, New York, 1993).

The following is an exemplary set of hybridization conditions and is not limiting:

### Very High Stringency (detects sequences that share 90% identity)

- Hybridization:: 5x SSC at 65°C for 16 hours
- Wash twice:: 2x SSC at room temperature (RT) for 15 minutes each
- Wash twice:: 0.5x SSC at 65°C for 20 minutes each

### High Stringency (detects sequences that share 80% identity or greater)

- Hybridization:: 5x-6x SSC at 65°C-70°C for 16-20 hours
- Wash twice:: 2x SSC at RT for 5-20 minutes each
- Wash twice:: 1x SSC at 55°C-70°C for 30 minutes each

### Low Stringency (detects sequences that share greater than 50% identity)

- Hybridization:: 6x SSC at RT to 55°C for 16-20 hours

Wash at least twice: 2x-3x SSC at RT to 55°C for 20-30 minutes each.

**Infection:** The entry, replication, insertion, lysis or other event or process involved with the pathogensis of a virus or other infectious pathogen into a host cell. Thus, decreasing infection includes decreasing entry, replication, insertion, lysis, or other pathogensis of a virus or other pathogen into a cell or subject, or combinations thereof. Infection includes the introduction of an infectious agent, such as a non-recombinant virus, recombinant virus, plasmid, bacteria, prion, eukaryotic microbe, bacterium, fungus, protozoa, or other agent capable of infecting a host, such as the cell of a subject.

In another example, infection is the introduction of a recombinant vector into a host cell, for example via transduction, transformation, or transfection. For example, a recombinant vector can include an antisense molecule or siRNA that recognizes Rab9A (such as any target sequence associated with SEQ ID NOS: 1-2, or variants, fusions, or fragments thereof, as well as SEQ ID NOS: 1-2 themselves), Rab11A, or a modulator of Rab9A or Rab11A.

**Isolated:** An "isolated" biological component (such as a nucleic acid molecule or protein) has been substantially separated, produced apart from, or purified away from other biological components in the cell of the organism in which the component naturally occurs, such as other chromosomal and extrachromosomal DNA and RNA, and proteins. Nucleic acid molecules and proteins which have been "isolated" include, but are not limited to, nucleic acid molecules and proteins purified by standard purification methods, those prepared by recombinant expression in a host cell, as well as chemically synthesized nucleic acid molecules and proteins.

**Marburg virus (MBGV):** A highly contagious hemorrhagic virus named after Marburg, Germany where the first outbreak occurred in 1967. Marburg is one of two members of Filovirus in the family of RNA viruses called Filoviridae. There is little genetic variability among viruses belonging to the Marburg type.

Symptoms of Marburg infection can include one or more of the following: sudden onset of fever (typically lasting 7 days), maculopapular petechial rash, and hemorrhaging.

Marburg can be diagnosed using methods known in the art. Particular examples include, but are not limited to: Taqman-RT-PCR (for example see Weidmann et al., J. Clin. Virol. 30:94-9, 2004), real-time reverse transcription-PCR (for example see Drosten et al., J. Clin. Microbiol. 40:2323-30, 2002), as well as detecting Marburg antibodies in a sample obtained from a subject.

**Measles virus (MV):** Measles is a Morbillivirus in the family of viruses called Paramyxoviridae. Measles is one of the most highly contagious infectious diseases. The virus is transmitted by airborne droplets, and is easily spread from person to person. The virus enters the body through the upper respiratory tract.

Symptoms of measles infection can include one or more of the following: fever, runny nose, cough, and red, watery eyes, often with sensitivity to light.

Measles can be diagnosed using methods known in the art. Particular examples include, but are not limited to: detecting measles virus or measles antibodies in a sample obtained from a subject, such as isolating the virus from the throat, or by a blood test for antibodies.

**MicroRNA (miR):** A small non coding RNA sequence that directs post transcriptional regulation of gene expression through interaction with a homologous mRNA. MiRs can inhibit translation, or can direct cleavage of target mRNAs. Therefore, miRs can be used to decrease or inhibit expression of a gene. In particular examples, miRs are about 21-26 nucleotides in length.

**Nucleic acid:** A deoxyribonucleotide or ribonucleotide polymer in either single (ss) or double stranded (ds) form, and can include analogues of natural nucleotides that hybridize to nucleic acids in a manner similar to naturally occurring nucleotides. In some examples, a nucleic acid is a nucleotide analog.

Unless otherwise specified, any reference to a nucleic acid molecule includes the reverse complement of nucleic acid. Except where single-strandedness is required by the text herein (for example, a ssRNA molecule), any nucleic acid written to depict only a single strand encompasses both strands of a corresponding double-stranded nucleic acid. For example, depiction of a plus-strand of a dsDNA also encompasses the complementary minus-strand of that dsDNA. Additionally, reference to the nucleic acid molecule that encodes a specific protein, or a fragment thereof, encompasses both the sense strand and its reverse complement.

A fragment of a nucleic acid molecule includes at least 5 contiguous bases from a nucleic acid sequence, such as a Rab9A nucleic acid sequence (such as a sequence associated with SEQ ID NOS: 1-2), a Rab 11A nucleic acid sequence, or the nucleic acid sequence of a Rab11A or Rab9A modulator. In particular examples, a fragment of a nucleic acid molecule corresponds to at least 10 contiguous bases, at least 20 contiguous bases, at least 25 contiguous bases, at least 50 contiguous bases, at least 100 contiguous bases, at least 250 contiguous bases, or even at least 500 contiguous bases of a desired nucleic acid sequences. Fragments of the nucleic acids described herein can be used to generate siRNA, ribozyme, triple helix, microRNA and antisense molecules.

**Oligonucleotide.** A linear polynucleotide (such as DNA or RNA) sequence of at least 9 nucleotides, for example at least 15, at least 18, at least 24, at least 25, at least 30, at least 50, at least 100, at least 200 or even at least 500 nucleotides long. In particular examples, an oligonucleotide is 6-50 bases, for example 10-25 bases, such as 12-20 bases.

An oligonucleotide analog refers to moieties that function similarly to oligonucleotides, but have non-naturally occurring portions. For example, oligonucleotide analogs can contain non-naturally occurring portions, such as altered sugar moieties or inter-sugar linkages, such as a phosphorothioate oligodeoxynucleotide. Functional analogs of naturally occurring polynucleotides can bind to RNA or DNA, and include peptide nucleic acid (PNA) molecules.

**Open reading frame (ORF).** A series of nucleotide triplets (codons) coding for amino acids without any internal termination codons. These sequences are usually translatable into a peptide.

**Operably linked.** A first nucleic acid sequence is operably linked with a second nucleic acid sequence when the first nucleic acid sequence is placed in a functional relationship with the second nucleic acid sequence. For instance, a promoter is operably linked to a coding sequence if the promoter affects the transcription or expression of the coding sequence. Generally, operably linked DNA sequences are contiguous and, where necessary to join two protein coding regions, in the same reading frame.

**p40 (or Rab9p40):** The term p40 includes any p40 gene, cDNA, RNA, or protein from any organism and that is a p40 that can bind Rab9-GTP and stimulate endosome-to-trans-golgi transport (for example see Diaz et al., J. Cell Biol. 138:283-90, 1997). p40 is also referred to in the literature as Rab9p40. In particular examples, p40 also interacts with PIKfyve wherein the p40-PIKfyve interaction and the subsequent PIKfyve-catalyzed p40 phosphorylation anchor p40 to discrete membranes facilitating the late endosome-to-trans-golgi transport.

Examples of native p40 sequences include, but are not limited to: Genbank Accession Nos: NM_015040, NM_005833.2 and CR456837.1 (cDNA) as well as NP_005824 and CAG33118 (proteins). In one example, a p40 sequence includes a full-length wild-type (or native) sequence, as well as p40 variants, fragments, homologs or fusion sequences that retain the ability to facilitate late endosome-to-trans-golgi transport. In certain examples, p40 has at least 80% sequence identity, for example at least 85%, at least 90%, at least 95%, or at least 98% sequence identity to a native p40 sequence, such as a human p40 nucleotide or protein sequence.

In other examples, a p40 nucleic acid sequence can hybridize under high stringency conditions to a sequence set forth in GenBank Accession No. NM_005833.2 or CR456837.1, and retains p40 activity.

**Pathogen:** A disease-producing agent. Examples include, but are not limited to viruses, bacteria, protozoa, and fungi. In a particular example, a pathogen is one that can hijack a lipid raft.

Examples of viruses include, but are not limited to, parvovirus, papillomaviruses, filovirus, SARS (severe acute respiratory syndrome) virus, hantaviruses, influenza viruses (such as influenza A, B and C viruses), hepatitis viruses A to G, caliciviruses, astroviruses, rotaviruses, coronaviruses, (for example, human respiratory coronavirus), picornaviruses, (for example, human rhinovirus and enterovirus), human herpesvirus (such as, HSV-1-9, including zoster, Epstein-Bar, and human cytomegalovirus), human adenovirus, smallpox virus and hantaviruses. In a particular example, the virus is an enveloped RNA virus, such as HIV (including HIV-1 and HIV-2), Ebola virus, Marburg virus, respiratory syncytia virus (RSV), and measles virus.

Examples of bacteria include, but are not limited to, *Listeria (spp.), Mycobacterium spp* (such as *M tuberculosis), Rickettsia* (all types), *Ehrlichia, Chylamida, Campylobacterjujuni, Nocardia asteroides, Legionella spp.* (such as *L. pneumophila*), *Brucella spp.* (such as B. *abortus*), *Escherichia coli, Salmonella spp.* (such as S. *typhimurium*), *Shigella spp.* (such as S. *flexneri*), *Yersinia pestis, Pasteurella haemolytica, Pasteurella multocida, Actinobacillus pleuropneumoniae, Listeria monocytogenes, Cowdria ruminantium, Coxiella burnetti, Staphylococcus aureus, Bacillus anthracis, Vibrio cholerae, Campylobacter spp., Neiserria meningitidis, Neiserria gonorrhea, Pseudomonas* species, *Haemophilus influenzae,* and *Clostridium tetani.*

Examples of protozoan and fungal species include, but are not limited to, *Plasmodium falciparum, Toxoplasma gondii, Pneumocystis carinii, Trypanosoma cruzi, Leishmania donovani, Theileria annulata, Eimeria tenella, Histoplasma capsulatum, Cryptococcus neoformans, Blastomyces dermatitidis, Coccidioides immitis, Paracoccidioides brasiliensis, Penicillium marneffei,* and *Candida* species.

**Pathogen that hijacks a lipid raft:** A pathogen that can use a lipid raft for completion of at least one step in its replication cycle. Examples of pathogens that hijack lipid rafts include, but are not limited to, *Campylobacter jujuni, Vibrio cholerae,* SV40, *Legionella pneumophila, Aeromonas hydrophilia,* Echovirus 1, Echovirus 11, *Brucella* spp, *Clostridium* spp., Avian sarcoma and leukosis virus, FimH, Dr *Escherichia coli, Streptcoccus pyogenes,* Semiliki forest virus, *Salmonella typhimurium, Bacillus anthracis,* Ecotropic mouse leukaemia virus, *Shigella flexneri, Bacillus thuringiensis,* HTLV-1, *Chlamydia* spp., *Helicobacter pylori,* HIV-1, *Mycobacterium* spp., *Lysteria monocytogenes, Ebola, Marburg,* Measles virus, Herpes Simplex virus, influenza virus, respiratory syncytia virus (RSV), or Epstein-Barr virus.

**Pharmaceutical agent or drug:** A chemical compound or composition capable of inducing a desired therapeutic or prophylactic effect when administered to a subject, alone or in combination with another therapeutic agent(s) or pharmaceutically acceptable carriers. In a particular example, a pharmaceutical agent decreases or even inhibits infection of a cell, such as the cell of a subject, by a pathogen, such as a virus.

**PIKfyve (or PIP5K3):** The term PIKfyve includes any PIKfyve gene, cDNA, RNA, or protein from any organism and that is a PIKfyve that can catalyze the synthesis of phosphatidylinositol (PtdIns) 5-phosphate (P) and phosphatidylinositol 3,5-P(2). In certain examples, PIKfyve associates with and promotes the membrane attachment of the late endosome-to-trans-Golgi network transport factor Rab9 effector p40 (for example see Ikonomov et al. J. Biol. Chem. 278:50863-71,2003).

Examples of native PIKfyve sequences include, but are not limited to: Genbank Accession Nos: NM_015040, AY457063 and BC032389 (cDNA) as well as NP_055855.1, Q9Y2I7 and AAR19397 (proteins). In one example, a PIKfyve sequence includes a full-length wild-type (or native) sequence, as well as PIKfyve variants, fragments, homologs or fusion sequences that retain the ability to catalyze the synthesis of PtdIns 5-P and PtdIns 3,5-P(2). In certain examples, PIKfyve has at least 80% sequence identity, for example at least 85%, at least 90%, at least 95%, or at least 98% sequence identity to a native PIKfyve protein or nucleic acid sequence.

In particular examples, a PIKfyve nucleic acid sequence can hybridize under high stringency conditions to a sequence set forth in GenBank Accession No. AY457063 or BC032389, and retains PIKfyve activity.

**Polymorphism.** A polymorphism exists when two or more versions of a nucleic acid sequence exist within a population of subjects. For example, a polymorphic nucleic acid can be one where the most common allele has a frequency of 99% or less. Different alleles can be identified according to differences in nucleic acid sequences, and genetic variations occurring in more than 1% of a population (which is the commonly accepted frequency for defining polymorphism) are useful polymorphisms for certain applications.

The allelic frequency (the proportion of all allele nucleic acids within a population that are of a specified type) can be determined by directly counting or estimating the number and type of alleles within a population. Polymorphisms and methods of determining allelic frequencies are discussed in Hartl, D.L. and Clark, A.G., Principles of Population Genetics, Third Edition (Sinauer Associates, Inc., Sunderland Massachusetts, 1997), particularly in chapters 1 and 2.

**Preventing or treating** a **disease:** "Preventing" a disease refers to inhibiting the full development of a disease, for example preventing development of a pathogen infection. "Treatment" refers to a therapeutic intervention that ameliorates a sign or symptom of a disease or pathological condition related to a pathogen infection, such as inhibiting or decreasing pathogen infection.

**Protein coding sequence or** a **sequence that encodes** a **peptide:** A nucleic acid sequence that is transcribed (in the case of DNA) and is translated (in the case of mRNA) into a peptide *in vitro* or *in vivo* when placed under the control of appropriate regulatory sequences. The boundaries of the coding sequence are determined by a start codon at the 5' (amino) terminus and a translation stop codon at the 3' (carboxy) terminus. A coding sequence can include, but is not limited to, cDNA from procaryotic or eukaryotic mRNA, genomic DNA sequences from procaryotic or eukaryotic DNA, and even synthetic DNA sequences. A transcription termination sequence is usually located 3' to the coding sequence.

**Puritied:** The term purified does not require absolute purity; rather, it is a relative term. Thus, for example, a purified protein preparation is one in which the protein is more enriched than the protein is in its environment within a cell, such that the protein is substantially separated from cellular components (nucleic acids, lipids, carbohydrates, and other proteins) that may accompany it. In another example, a purified protein preparation is one in which the protein is substantially-free from contaminants, such as those that might be present following chemical synthesis of the protein.

In one example, a protein is purified when at least 60% by weight of a sample is composed of the protein, for example when at least 75%, at least 95%, or at least 99% or more of a sample is composed of the protein, such as a Rab9A peptide. Examples of methods that can be used to purify proteins, include, but are not limited to the methods disclosed in Sambrook et al. (Molecular Cloning: A Laboratory Manual, Cold Spring Harbor, New York, 1989, Ch. 17). Protein purity can be determined by, for example, polyacrylamide gel electrophoresis of a protein sample, followed by visualization of a single polypeptide band upon staining the polyacrylamide gel; high-pressure liquid chromatography; sequencing; or other conventional methods.

**Rab9A:** The term Rab9A includes any Rab9A gene, cDNA, RNA, or protein from any organism and that is a Rab9A that can transport late endosomes to trans-golgi and function as a ras-like GTPase. In some examples, Rab9A is involved in lipid raft formation.

Examples of native Rab9A nucleic acid sequences include, but are not limited to, target sequences associated with SEQ ID NOS: 1 and 2. Examples of Rab9A sequences include, but are not limited to: Genbank Accession Nos: BC017265.2 and NM_004251.3 (cDNA) as well as P51151 and AAH 17265 (proteins). The sequences provided under the GenBank Accession Nos. mentioned herein are hereby incorporated in their entireties by this reference. In one example, a Rab9A sequence includes a full-length wild-type (or native) sequence, as well as Rab9A variants, fragments, homologs or fusion sequences that retain the ability to transport late endosomes to trans-golgi. In certain examples, Rab9A has at least 80% sequence identity, for example at least 85%, at least 90%, at least 95%, or at least 98% sequence identity to a native Rab9A sequence. In particular examples, a Rab9A nucleic acid sequence can hybridize under high stringency conditions to a sequence set forth in GenBank Accession No. BC017265.2 or NM_004251.3, and retains Rab9A activity.

**Rab9A modulator:** A molecule, such as a nucleotide or protein sequence, that interacts directly or indirectly with Rab9A. In some examples a Rab9A modulator increases Rab9A activity, for example by increasing transcription or translation of Rab9A mRNA. In other examples a Rab9A modulator decreases Rab9A activity, for example by decreasing transcription or translation of Rab9A mRNA. In particular examples, a Rab9A modulator is a protein involved in trafficking proteins and other molecules from the late endosome to the trans-golgi via interaction with Rab9A.

Exemplary Rab9A modulators include, but are not limited to: a Rab9 effector such as p40 (referred to herein as p40); PIKfyve; GDI-displacement factor (GDF) (for example see Dirac-Svejstrup et al. EMBO J. 16: 465-72, 1997) such asYip3 (for example see Sivars et al. Nature 425:856-9, 2003; see GenBank Accession No. NM_006423 for an exemplary Yip3 nucleic acid and peptide sequence); tumor susceptibility gene 101 (TSG101; for example see Panchal et al. Proc. Natl. Acad. Sci. U S A. 100:15936-41, 2003; see GenBank Accession No. NM_006292 for an exemplary TSG101 nucleic acid and peptide sequence); TIP47 (see GenBank Accession No. NM_005817 for an exemplary TIP47 nucleic acid and peptide sequence); v-SNARE protein (see GenBank Accession No. XM-371718 for an exemplary v-SNARE nucleic acid and peptide sequence); mannose-6-phosphate receptor (see GenBank Accession No. NM_002355 for an exemplary mannose-6-phosphate receptor nucleic acid and peptide sequence); mapmodulin (for example see Itin et al., Mol. Biol. Cell. 10:2191-7, 1999; see GenBank Accession No. AF025684 for an exemplary mapmodulin nucleic acid and peptide sequence); Syntaxin 11, a t-SNARE protein (see GenBank Accession No. AF044309 for an exemplary t-SNARE nucleic acid and peptide sequence); a GDP dissociation inhibitor (see GenBank Accession No. NM_001493 for an exemplary GDP nucleic acid and peptide sequence); and a guanine-nucleotide exchange factor (GEF) (see GenBank Accession No. L13858 for an exemplary GEF nucleic acid and peptide sequence). In a specific example, a Rab9A modulator includes PIKfyve, p40, TIP47, or combinations thereof. In another specific example, a Rab9A modulator excludes TIP47.

**Rab11A:** The term Rab11A includes any Rab11A gene, cDNA, RNA, or protein from any organism and that is a Rab11A that can mediate transport from the trans-Golgi to the cell membrane. Rab11A is also expressed in recycling endosomes.

Examples of Rab11A sequences include, but are not limited to: Genbank Accession Nos: X56740 and L19260 (cDNA) as well as CAA40064 and AAA31491 (proteins). In one example, a Rab11A sequence includes a full-length wild-type (or native) sequence, as well as Rab11A variants, fragments, or fusion sequences that retain the ability to mediate transport from the trans-Golgi to the cell membrane. In certain examples, Rab11A has at least 80% sequence identity, for example at least 85%, at least 90%, at least 95%, or at least 98% sequence identity to a native Rab11 sequence. In particular examples, a Rab11A nucleic acid sequence can hybridize under high stringency to a sequence set forth in GenBank Accession No. X56740 or L19260, and retains Rab11A activity.

**Rab11A modulator:** A molecule, such as a nucleotide or protein sequence, that interacts directly or indirectly with Rab11A. In some examples a Rab9A modulator increases Rab9A activity, for example by increasing transcription or translation of Rab9A mRNA. In other examples a Rab9A modulator decreases Rab9A activity, for example by decreasing transcription or translation of Rab9A mRNA. In some examples, a Rab11A modulator is a protein involved in trafficking proteins and other molecules from the trans-golgi to the cell membrane via interaction with Rab11A.

Exemplary Rab11A modulators include, but are not limited to: Rab11 effectors, such as members of the family of Rab11-interacting proteins (FIPs). Specific examples include, but are not limited to, Sec15 (for example see Zhang et al. J. Biol. Chem, 279:43027-34, 2004; see GenBank Accession No. NM_019053 and NP_061926 for an exemplary Secl5 nucleic acid and peptide sequence, respectively), Rip11 (for example see Prekeris et al. Mol. Cell. 6:1437-48, 2000; see GenBank Accession No. AF334812 and AAK20892 for an exemplary Rip11 nucleic acid and peptide sequence, respectively), FIP2 (for example see Cullis et al. J. Biol. Chem. 277:49158-66, 2002; see GenBank Accession No. NM_014904 and NP_055719 for an exemplary FIP2 nucleic acid and peptide sequence, respectively), Rab-coupling protein (RCP) (for example see Lindsay et al. J. Biol. Chem. 277:12190-9, 2002; see GenBank Accession No. AF368294 for an exemplary RCP nucleic acid and peptide sequence), FIP3/eferin (for example see Prekeris et al. J. Biol. Chem. 276:38966-70, 2001; see GenBank Accession No. NM_014700 and NP_055515 for an exemplary FIP3 nucleic acid and peptide sequence, respectively), FIP4 (for example see Wallace et al., Biochem. Biophys. Res. Commun. 299:770-9, 2002; see GenBank Accession No. NM_032932 and NP_116321 for an exemplary FIP4 nucleic acid and peptide sequence, respectively), and FIP 1 (for example see Hales et al., J. Biol. Chem. 276:39067-75,2001; see GenBank Accession No. NM_001002814 for an exemplary FIP 1 nucleic acid and peptide sequence). All FIPs have a conserved C-terminal motif that is known as the Rab11/25-binding domain (RBD). Based on the presence of additional structural domains, FIPs are classified into three groups: class I FIPs (Rip11, FIP2, and RCP) containing a C2 domain, class II FIPs (FIP3/eferin and FIP4) containing EF-hand motifs, and the class III FIP (FIP1) with no homology to known protein domains.

**Recombinant:** A recombinant nucleic acid or protein is one that has a sequence that is not naturally occurring or has a sequence that is made by an artificial combination of two otherwise separated segments of sequence. This artificial combination can be accomplished, for example, by chemical synthesis or by the artificial manipulation of isolated segments of nucleic acids or proteins, for example, by genetic engineering techniques.

**RNA interference (RNAi):** A post-transcriptional gene silencing mechanism mediated by double-stranded RNA (dsRNA). Introduction of dsRNA into cells, such as siRNA compounds, induces targeted degradation of RNA molecules with homologous sequences. RNAi compounds can be used to modulate transcription, for example, by silencing genes, such as Rab9A, Rab11, and modulators thereof. In certain examples, an RNAi molecule is directed against a target gene, such as Rab9A, Rab11A, a Rab9A modulator, or a Rab11A modulator, and is used to decrease a pathogen infection.

**Sequence identity:** The similarity between nucleic acid or amino acid sequences is expressed in terms of the similarity between the sequences. Sequence identity is frequently measured in terms of percentage identity (or similarity or homology); the higher the percentage, the more similar the two sequences are. Homologs or variants of a protein or nucleic acid sequence, and their corresponding cDNA and protein sequences, will possess a relatively high degree of sequence identity when aligned using standard methods.

Methods of alignment of sequences for comparison are well known in the art. Various programs and alignment algorithms are described in: Smith and Waterman, Adv. Appl. Math. 2:482, 1981; Needleman and Wunsch, J. Mol. Biol. 48:443, 1970; Pearson and Lipman, Proc. Natl. Acad Sci. U.S.A. 85:2444, 1988; Higgins and Sharp, Gene 73:237-44, 1988; Higgins and Sharp, CABIOS 5:151-3, 1989; Corpet et al., Nucl. Acids Res. 16:10881-90, 1988; Pearson and Lipman, Proc. Natl. Acad. Sci. U.S.A. 85:2444, 1988; and Altschul et al., Nature Genet. 6:119-29,1994.

The NCBI Basic Local Alignment Search Tool (BLAST^{™}) (Altschul et al., J. Mol. Biol. 215:403-10, 1990) is available from several sources, including the National Center for Biotechnology Information (NCBI, Bethesda, MD) and on the Internet, for use in connection with the sequence analysis programs blastp, blastn, blastx, tblastn and tblastx.

Variants of a peptide, such as a Rab9A, Rab11A, or a modulator thereof, are typically characterized by possession of at least 70% sequence identity counted over the full length alignment with the amino acid sequence using the NCBI Blast 2.0, gapped blastp set to default parameters. For comparisons of amino acid sequences of greater than about 30 amino acids, the Blast 2 sequences function is employed using the default BLOSUM62 matrix set to default parameters, (gap existence cost of 11, and a per residue gap cost of 1). When aligning short peptides (fewer than around 30 amino acids), the alignment is performed using the Blast 2 sequences function, employing the PAM30 matrix set to default parameters (open gap 9, extension gap 1 penalties). Proteins with even greater similarity to the reference sequences will show increasing percentage identities when assessed by this method, such as at least 80%, at least 90%, at least 95%, at least 98%, or even at least 99% sequence identity. When less than the entire sequence is being compared for sequence identity, homologs and variants will typically possess at least 80% sequence identity over short windows of 10-20 amino acids, and may possess sequence identities of at least 85%, at least 90%, at least 95%, or at least 98% depending on their similarity to the reference sequence. Methods for determining sequence identity over such short windows are described at the website that is maintained by the National Center for Biotechnology Information in Bethesda, Maryland. One of skill in the art will appreciate that these sequence identity ranges are provided for guidance only; it is entirely possible that strongly significant homologs could be obtained that fall outside of the ranges provided.

Similar methods can be used to determine the sequence identity between two or more nucleic acids. To compare two nucleic acid sequences, the BLASTN options can be set as follows: -i is set to a file containing the first nucleic acid sequence to be compared (such as C:\seq1.txt);-j is set to a file containing the second nucleic acid sequence to be compared (such as C:\seq2.txt); -p is set to blastn; -o is set to any desired file name (such as C:\output.txt); -q is set to -1; -r is set to 2; and all other options are left at their default setting. For example, the following command can be used to generate an output file containing a comparison between two sequences: C:\Bl2seq -i c:\seq1.txt -j c:\seq2.txt -p blastn -o c:\output.txt -q -1-r 2.

Once aligned, the number of matches is determined by counting the number of positions where an identical nucleotide or amino acid residue is presented in both sequences. The percent sequence identity is determined by dividing the number of matches either by the length of the sequence set forth in the identified sequence, or by an articulated length (for example, 100 consecutive nucleotides or amino acid residues from a sequence set forth in an identified sequence), followed by multiplying the resulting value by 100. For example, a nucleic acid sequence that has 1166 matches when aligned with a test sequence having 1154 nucleotides is 75.0 percent identical to the test sequence (for example, 1166÷1554*100=75.0). The percent sequence identity value is rounded to the nearest tenth. For example, 75.11, 75.12, 75.13, and 75.14 are rounded down to 75.1, while 75.15, 75.16, 75.17, 75.18, and 75.19 are rounded up to 75.2. The length value will always be an integer. In another example, a target sequence containing a 20-nucleotide region that aligns with 20 consecutive nucleotides from an identified sequence as follows contains a region that shares 75 percent sequence identity to that identified sequence (for example, 15÷20*100=75).

1                    20
Target Sequence: AGGTCGTGTACTGTCAGTCA
| || ||| |||| |||| |
Identified Sequence:ACGTGGTGAACTGCCAGTGA

The nucleic acids disclosed herein include nucleic acids have nucleotide sequences that are at least 70%, at least 75%, at least 80%, at least 85%, at least 90%, at least 95%, at least 98%, or at least 99% identical to the nucleotide sequence of a Rab9A, Rab11A, Rab9A modulator, or a Rab11A modulator nucleic acid sequence. In particular examples, a nucleic acid sequence is substantially similar to a Rab9A, Rab11A, Rab9A modulator, or a Rab11A modulator nucleotide sequence. A first nucleic acid is "substantially similar" to a second nucleic acid if, when the first nucleic acid is optimally aligned (with appropriate nucleotide deletions or gap insertions) with the second nucleic acid (or its complementary strand) and there is nucleotide sequence identity of at least about 90%, for example at least about 95%, at least 98% or at least 99% identity. An alternative indication that two nucleic acid molecules are closely related is that the two molecules hybridize to each other under stringent conditions.

Nucleic acid sequences that do not show a high degree of identity may nevertheless encode similar amino acid sequences, due to the degeneracy of the genetic code. It is understood that changes in nucleic acid sequence can be made using this degeneracy to produce multiple nucleic acid molecules that all encode substantially the same protein.

**Short interfering or interrupting RNA (siRNA):** Double-stranded RNAs that can induce sequence-specific post-transcriptional gene silencing, thereby decreasing or even inhibiting gene expression. In some examples, siRNA molecules are about 19-23 nucleotides in length, such as 21-23 nucleotides. In particular examples, siRNA molecules are at least 21 nucleotides, for example at least 23 nucleotides in length. In one example, an siRNA molecule is an siRNA duplex composed of21-nucleotides (nt) sense and 21-nt antisense strands, paired in a manner to have a 2-nt 3' overhang on both strands. In another example, an siRNA is 19 nt in length having two dT overhangs at the N- and C-terminal ends.

In one example, siRNA triggers the specific degradation of homologous RNA molecules, such as mRNAs, within the region of sequence identity between both the siRNA and the target RNA. For example, WO 02/44321 discloses siRNAs capable of sequence-specific degradation of target mRNAs when base-paired with 3' overhanging ends. The direction of dsRNA processing determines whether a sense or an antisense target RNA can be cleaved by the produced siRNA endonuclease complex. Thus, siRNAs can be used to modulate transcription, for example, by silencing genes, such as Rab9A, Rab11A, a Rab9A modulator, or a Rab11A modulator. The effects of siRNAs have been demonstrated in cells from a variety of organisms, including *Drosophila, C. elegans,* insects, frogs, plants, fungi, mice and humans (for example, WO 02/44321; Gitlin et al., Nature 418:430-4, 2002; Caplen et al., Proc. Natl. Acad Sci. 98:9742-9747,2001; and Elbashir et al., Nature 411:494-8, 2001).

**Specific binding agent.** An agent that binds substantially only to a defined target. For example, a protein-specific binding agent binds substantially only the specified protein and a nucleic acid molecule specific binding agent binds substantially only the specified nucleic acid sequence.

As used herein, the term "protein [X] specific binding agent" includes anti-[X] protein antibodies (such as polyclonal or monoclonal antibodies and functional fragments thereof) and other agents (such as soluble receptors) that bind substantially only to the [X] protein. In this context, [X] refers to any specific or designated protein, for instance Rab9A, Rab11A, a Rab9A modulator, or a Rab11A modulator.

Anti-[X] protein antibodies can be produced using standard procedures such as those described in Harlow and Lane (Using Antibodies: A Laboratory Manual, Cold Spring Harbor Laboratory Press: Cold Spring Harbor, 1998). Exemplary antibodies include, but are not limited to, polyclonal antibodies, monoclonal antibodies, humanized antibodies, Fab fragments, F(ab')2 fragments, single chain antibodies, and chimeric antibodies. For example, polyclonal antibodies can be produced by immunizing a host animal by injection with the desired peptide (such as Rab9A, Rab11A, a Rab9A modulator, or a Rab11A modulator). The production of monoclonal antibodies can be accomplished by a variety of methods, such as the hybridoma technique (Kohler and Milstein, Nature 256:495-7, 1975), the human B-cell technique (Kosbor et al., Immunology Today 4:72, 1983), or the EBV-hybridoma technique (Cole et al., in Monoclonal Antibodies and Cancer Therapy, Alan R. Liss, Inc., pp. 77-96, 1983). Additionally, chimeric antibodies can be produced (for example, see Morrison et al., J. Bacteriol. 159:870, 1984; Neuberger et al., Nature 312:604-8, 1984; and Takeda et al., Nature 314:452-4, 1985), as well as single-chain antibodies (for example, see U.S. Pat. Nos: 5,476,786; 5,132,405; and 4,946,778).

The determination that a particular agent binds substantially only to the specified protein readily can be made by using or adapting routine procedures. For example, Western blotting can be used to determine that a given protein binding agent, such as an anti-[X] protein monoclonal antibody, binds substantially only to the [X] protein. Other assays include, but are not limited to, competitive and non-competitive homogenous and heterogeneous enzyme-linked immunosorbent assays (ELISA) as symmetrical or asymmetrical direct or indirect detection formats; "sandwich" immunoassays; immunodiffusion assays; in situ immunoassays (for example, using colloidal gold, enzyme or radioisotope labels); agglutination assays; complement fixing assays; immunoelectrophorectic assays; enzyme-linked immunospot assays (ELISPOT); radioallergosorbent tests (RAST); fluorescent tests, such as used in fluorescent microscopy and flow cytometry; Western, grid, dot or tissue blots; dip-stick assays; halogen assays; or antibody arrays (for example, see O'Meara and Tovey, Clin. Rev. Allergy Immunol., 18:341-95, 2000; Sambrook et al., 2001, Appendix 9; Simonnet and Guilloteau, in: Methods of Immunological Analysis, Masseyeff et al. (Eds.), VCH, New York, 1993, pp. 270-388).

A specific binding agent also can be labeled for direct detection (see Chapter 9, Harlow and Lane, Antibodies: A Laboratory Manual. 1988). Suitable labels include (but are not limited to) enzymes (such as alkaline phosphatase (AP) or horseradish peroxidase (HRP)), fluorescent labels, colorimetric labels, radioisotopes, chelating agents, dyes, colloidal gold, ligands (such as biotin), and chemiluminescent agents.

Shorter fragments of antibodies can also serve as specific binding agents. For instance, Fabs, Fvs, and single-chain Fvs (SCFvs) that bind to a specified protein would be specific binding agents. These antibody fragments include: (1) Fab, the fragment containing a monovalent antigen-binding fragment of an antibody molecule produced by digestion of whole antibody with the enzyme papain to yield an intact light chain and a portion of one heavy chain; (2) Fab', the fragment of an antibody molecule obtained by treating whole antibody with pepsin, followed by reduction, to yield an intact light chain and a portion of the heavy chain; two Fab' fragments are obtained per antibody molecule; (3) (Fab')2, the fragment of the antibody obtained by treating whole antibody with the enzyme pepsin without subsequent reduction; (4) F(ab')2, a dimer of two Fab' fragments held together by two disulfide bonds; (5) Fv, a genetically engineered fragment containing the variable region of the light chain and the variable region of the heavy chain expressed as two chains; and (6) single chain antibody ("SCA"), a genetically engineered molecule containing the variable region of the light chain, the variable region of the heavy chain, linked by a suitable polypeptide linker as a genetically fused single chain molecule. Methods of making these fragments are routine. For example, construction of Fab expression libraries permits the rapid and easy identification of monoclonal Fab fragments with the desired specificity for a protein described herein.

**Subject:** Living multi-cellular vertebrate organisms, including human and veterinary subjects. Particular examples of veterinary subjects include domesticated animals (such as cats and dogs), livestock (for example, cattle, horses, pigs, sheep, and goats), laboratory animals (for example, mice, rabbits, rats, gerbils, guinea pigs, and non-human primates), as well as birds, reptiles, and fish.

**Target sequences associated with SEQ ID NO:** When used herein, this phrase refers to any nucleic acid sequence, amino acid sequence, or combination of nucleic acid and amino acid sequences, that are involved in pathogen infection, and therefore serve as targets for decreasing or inhibiting pathogen infection, and which are or include a specified SEQ ID NO, are involved in the expression of the SEQ ID NO, or are peptide sequences that are expressed by such specified SEQ ID NO. Although a target sequence may refer to a SEQ ID NO of a sequence obtained from a particular species, the target sequences also include homologues of the sequence from other related species, such as other mammals. For example, the phrase "target sequences associated with SEQ ID NO. X" can refer to the entire gene sequence of which the particular SEQ ID NO X is a part, the appropriate coding sequence, a promoter sequence associated with the gene, or the corresponding protein sequence, as well as variants, fragments, homologues, and fusions thereof that retain the activity of the native sequence.

For example, when using the phrase "sequences associated with SEQ ID NOS: 1-2," this term encompasses Rab9A genomic sequences, endogenous promoter sequences that promote the expression of Rab9A, Rab9A coding sequences, and Rab9A proteins, as well as variants, fragments, and fusions thereof that retain the activity of a native Rab9A sequence.

Although sequences are provided herein that encode (or are included within sequences that encode) host proteins that are involved in pathogen infection, it should be understood that the ultimate goal is to interfere with the activity of the protein that has been identified to be involved in pathogenesis. Such interference can be at either the level of the nucleic acid molecule that encodes the protein (for example by reducing or otherwise disrupting expression of the protein), or at the level of the protein itself (for example by interfering with the activity of the protein, or its interaction with the pathogen). The disclosure of specific techniques for achieving these goals in particular species should not be interpreted to limit the method to these particular techniques.

**Target sequence of** a **nucleic acid:** A portion of a nucleic acid molecule that, upon hybridization to a therapeutically effective oligonucleotide or oligonucleotide analog, results in reduction or even inhibition of infection by an infectious agent. An antisense or a sense molecule can be used to target a portion of dsDNA, since either can interfere with the expression of that portion of the dsDNA. The antisense molecule can bind to the plus strand, and the sense molecule can bind to the minus strand. Thus, target sequences can be ssDNA, dsDNA, and RNA.

**Therapeutically active molecule:** An agent, such as a protein, antibody or nucleic acid molecule, that has a desired therapeutic effect. An example of such an agent is one that can decrease the activity of Rab9A, Rab11A, a Rab9A modulator, or a Rab11A modulator involved in pathogen infection, for example as measured by clinical response (such as a decrease in infection by a pathogen, such as an inhibition of infection). Therapeutically active agents also include organic or other chemical compounds that mimic the effects of the therapeutically effective peptide, antibody, or nucleic acid molecule.

**Therapeutically Effective Amount:** An amount of a pharmaceutical preparation that alone, or together with a pharmaceutically acceptable carrier or one or more additional therapeutic agents, induces the desired response. The preparations disclosed herein are administered in therapeutically effective amounts.

In one example, a desired response is to decrease or inhibit infection of a cell by a pathogen, such as a cell of a subject. Infection does not need to be completely inhibited for the pharmaceutical preparation to be therapeutically effective. For example, a pharmaceutical preparation can decrease infection by a desired amount, for example by at least 20%, at least 50%, at least 60%, at least 70%, at least 80%, at least 90%, at least 95%, at least 98%, or even at least 100%, as compared to an amount of infection in the absence of the pharmaceutical preparation. This decrease or inhibition can result in halting or slowing the progression of, or inducing a regression of a pathological condition caused by the pathogen infection, or which is capable of relieving signs or symptoms caused by the condition.

In another or additional example, it is an amount sufficient to partially or completely alleviate symptoms of pathogen infection within a host subject. Treatment can involve only slowing the progression of the infection temporarily, but can also include halting or reversing the progression of the infection permanently.

Effective amounts of the therapeutic agents described herein can be determined in many different ways, such as assaying for a reduction in the rate of infection of cells or subjects, a reduction in the viral load within a host, improvement of physiological condition of an infected subject, or increased resistance to infection following exposure to the virus. Effective amounts also can be determined through various *in vitro, in vivo* or *in situ* assays, including the assays described herein.

The disclosed therapeutic agents can be administered in a single dose, or in several doses, for example daily, during a course of treatment. However, the effective amount of can be dependent on the source applied (for example a nucleic acid molecule isolated from a cellular extract versus a chemically synthesized and purified nucleic acid), the subject being treated, the severity and type of the condition being treated, and the manner of administration.

**TIP47 (tail-interacting protein of 47 kDa):** The term TIP47 includes any TIP47 gene, cDNA, RNA, or protein from any organism and that is a TIP47 that can transport mannose-6-phosphate receptors from endosomes to the TGN. TIP47 binds with high specificity to the cytosolic domains of the cation-dependent mannose-6-phosphate receptor (CD-MPR) and cation-independent MPR (CI-MPR). In addition, TIP47 binds directly to Rab9A, which enhances TIP47's affinity for MPR cytoplasmic domains. Also referred to in the literature as or mannose-6-phosphate receptor binding protein 1, M6PRBP 1.

Examples of TIP47 sequences include, but are not limited to: Genbank Accession Nos:
NM_005817 and BC007566 (cDNA) as well as NP_005808 and AAH05818 (proteins). In one example, a TIP47 sequence includes a full-length wild-type (or native) sequence, as well as TIP47 variants, fragments, or fusion sequences that retain the ability to transport mannose-6-phosphate receptors from endosomes to the TGN. In certain examples, TIP47 has at least 80% sequence identity, for example at least 85%, at least 90%, at least 95%, or at least 98% sequence identity to a native TIP47 sequence.

In particular examples, a TIP47 nucleic acid sequence can hybridize under high stringency to a sequence set forth in GenBank Accession No. NM_005817 or BC007566, and retains TIP47 activity.

**Transduce, Transform, or Transfect:** To introduce a nucleic acid molecule into a cell. These terms encompass all techniques by which a nucleic acid molecule can be introduced into a cell, including but not limited to, transfection with viral vectors, transformation with plasmid vectors, and introduction of naked DNA by electroporation, lipofection, and particle gun acceleration. A transfected or transformed cell is a cell into which has been introduced a nucleic acid molecule by molecular biology techniques. In particular examples, the nucleic acid molecule becomes stably replicated by the cell, for example by incorporation of the nucleic acid molecule into the cellular genome, or by episomal replication.

**Transgene:** An exogenous nucleic acid sequence, for example supplied by a vector. In one example, a transgene includes a nucleic acid that encodes or specifically hybridizes to a Rab9A, Rab11A, or a modulator of Rab9A or Rab11A.

**Variants, fragments or fusions:** The disclosed nucleic acid sequences, such as target sequences associated with SEQ ID NO: 1 or 2, and the proteins encoded thereby, (as well as Rab9A, Rab11A, Rab9A modulator, and Rab11A modulator proteins and nucleic acid sequences) include variants, fragments, and fusions thereof that retain the native biological activity (such as playing a role in pathogen infection). DNA sequences which encode for a protein or fusion thereof, or a fragment or variant of thereof can be engineered to allow the protein to be expressed in eukaryotic cells or organisms, bacteria, insects, or plants. To obtain expression, the DNA sequence can be altered and operably linked to other regulatory sequences. The final product, which contains the regulatory sequences and the therapeutic protein, is referred to as a vector. This vector can be introduced into a cell. Once inside the cell the vector allows the protein to be produced.

One of ordinary skill in the art will appreciate that the DNA can be altered in numerous ways without affecting the biological activity of the encoded protein. For example, PCR can be used to produce variations in the DNA sequence which encodes a protein. Such variants can be variants optimized for codon preference in a host cell used to express the protein, or other sequence changes that facilitate expression.

**Vector:** A nucleic acid molecule as introduced into a host cell, thereby producing a transformed host cell. A vector can include nucleic acid sequences that permit it to replicate in a host cell, such as an origin of replication, and can also include one or more selectable marker genes and other genetic elements. An insertional vector is capable of inserting itself into a host nucleic acid. Vectors include, but are not limited to, viral, plasmid, cosmid, and artificial chromosome vectors.

**Wild-type.** A naturally occurring, non-mutated version of a nucleic acid or protein sequence. Among multiple alleles, the allele with the greatest frequency within the population is usually (but not necessarily) the wild-type. The term "native" can be used as a synonym for "wild-type."

### Methods of Decreasing Infection

Rab9A was identified as a host nucleic acid sequence involved in viral infection using gene trap methods. Rab9A is a GTPase that resides in a late endosome microdomain together with mannose 6-phosphate receptors (MPRs) and the tail-interacting protein of 47 kDa (TIP47). Rab9A traffics proteins and other molecules from the late endosome to the TGN. For example, Rab9A can transport newly synthesized lysosomal enzymes from the Golgi complex to endosomes, and then return to the Golgi. Rab 11A is involved with trafficking of proteins from the TGN to the cell surface.

It is demonstrated herein that in addition to Rab9A, Rab11A and modulators of Rab9A (including TIP47, PIKfyve, and p40) are involved in viral infection. However, the role of Rab9A, Rab11A, as well as modulators of Rab9A and Rab11A, are not limited to viral infection, as such proteins (and their corresponding nucleic acid sequences) are involved generally in infection by pathogens (such as viruses, bacteria (for example members of the genus *Pseudomonas*), protozoa, and fungi), such as those pathogens that use similar pathways for infection.

Based on this disclosure, methods are provided for decreasing the biological activity of Rab9A or Rab11A in a host cell, such as a mammalian cell, to decrease infection of the host cell by a pathogen. Decreased infection can occur, *in vitro, ex vivo* or *in vivo.* In some examples, the biological activity of Rab9A or Rab 11A is decreased by decreasing the activity of a Rab9A or Rab11A modulator that increases Rab9A or Rab11A activity, respectively. Alternatively (or in addition), the biological activity of Rab9A or Rab11A is decreased by increasing the activity of a Rab9A or Rab11A modulator that decreases Rab9A or Rab11A activity, respectively.

In a particular example, Rab9A includes the target sequences associated with SEQ ID NOS: 1-2, such as a host nucleic acid sequence that shares at least 90% sequence identity, such as at least 95% sequence identity, to a target nucleic acid sequence associated with SEQ ID NO: 1 or 2. The method can include decreasing, and in some examples inhibiting, the biological activity of Rab9A, Rab11A, at least one Rab9A modulator, two or more Rab9A modulators, three or more Rab9A modulators, one to five Rab9A modulators, at least one Rab11A modulator, two or more Rab11A modulators, three or more Rab11A modulators, one to five Rab11A modulators, or combinations thereof. Decreasing or inhibiting the activity of Rab9A, Rab11A, or modulator thereof that increases Rab9A or Rab11A activity, can block a component of the life cycle of a pathogen, such as blocking a signal pathway leading to transcription or translation of the viral genome, or assembly of viral sub-parts.

Methods of decreasing the biological activity of a nucleic acid or protein sequence are known in the art. Although particular examples of such methods are provided herein for illustrative purposes, the disclosure is not limited to such methods. In particular examples, decreasing the biological activity of Rab9A or Rab11A (for example by decreasing the activity of a modulator thereof that increases Rab9A or Rab 11A activity), does not require a 100% reduction. For example, decreases of at least 20%, at least 40%, at least 50%, at least 60%, at least 75%, at least 80%, at least 90%, at least 95%, or at least 99%, as compared to a control (such as an amount of activity in a cell not treated with a therapeutic agent), can be sufficient.

### Pathogens

Pathogens include, but are not limited to, viruses, bacteria, protozoa, and fungi. In one example, pathogens include those that hijack a lipid raft. Lipid rafts are liquid-ordered microdomains enriched in sphingolipds and cholesterol, and are involved in biosynthetic traffic, signal transduction, and endocytosis. Some pathogens take advantage of ("hijack") rafts for completion of some steps of their replication cycle, such as entry into their cell host, assembly, and budding. Examples of pathogens that hijack lipid rafts include, but are not limited to, *Campylobacter jujuni, Vibrio cholerae,* SV40, *Legionella pneumophila, Aeromonas hydrophilia,* Echovirus 1, Echovirus 11, *Brucella* spp, *Clostridium* spp., Avian sarcoma and leukosis virus, FimH, Dr *Escherichia coli, Streptcoccus pyogenes,* Semiliki forest virus, *Salmonella typhimurium, Bacillus anthracis,* Ecotropic mouse leukaemia virus, *Shigella flexneri, Bacillus thuringiensis,* HTLV-1, *Chlamydia* spp., *Helicobacterpylori,* HIV-1, *Mycobacterium* spp., *Lysteria monocytogenes, Ebola, Marburg,* Measles, Herpes Simplex vitus, influenza virus, RSV, or Epstein-Barr virus.

In one example, a pathogen that hijacks a lipid raft is an enveloped RNA virus. Enveloped RNA viruses include enveloped positive-strand RNA viruses, enveloped negative-strand RNA viruses, and retroviruses. Exemplary positive-strand RNA viruses that have an envelope include, but are not limited to: Togaviruses (examples of which include rubella); alphaviruses (such as Western equine encephalitis virus, Eastern equine encephalitis virus, and Venezuelan equine encephalitis virus); Flaviviruses (examples of which include Dengue virus, West Nile virus, Japanese encephalitis virus, and hepatitis C virus); and Coronaviruses (such as SARS). Exemplary negative-strand RNA viruses that have an envelope include, but are not limited to: Orthomyxoviruses (such as influenza virus), Rhabdoviruses (such as Rabies virus), Paramyxoviruses (examples of which include measles virus, mumps virus, respiratory syncytial virus) and Filoviruses, such as Marburg and Ebola.

Examples of retroviruses include, but are not limited to: human immunodeficiency virus type 1 (HIV-1), HIV-2; equine infectious anemia virus; feline immunodeficiency virus (FIV); feline leukemia viruses (FeLV); simian immunodeficiency virus (SIV); and avian sarcoma virus.

In a particular examples, the pathogen is human immunodeficiency virus (HIV)-1, HIV-2, Ebola virus, Marburg virus, RSV, or measles virus.

### Rab9A modulators

Rab9A modulators include nucleic acid and protein sequences that interact directly or indirectly with Rab9A. In some examples, modulators increase Rab9A activity, for example by increasing transcription or translation of Rab9A mRNA. In other examples, modulators decrease Rab9A activity, for example by decreasing transcription or translation of Rab9A mRNA. Some Rab9A modulators directly bind to Rab9A, and enhance the biological activity of Rab9A. In particular examples, a Rab9A modulator modulates Rab9A expression by transcriptional activation or by binding to Rab9A. In some examples, a Rab9A modulator is a protein involved in trafficking proteins or other molecules from a late endosome to the TGN by interaction with Rab9A. Therefore, a Rab9A modulator can be a protein that binds to, or is involved in a complex with Rab9A, and is involved in the trafficking of proteins and other molecules from the late endosome to the TGN.

Exemplary Rab9A modulators include, but are not limited to: a Rab9 effector such as p40 (referred to herein as p40); PIKfyve; a GDI-displacement factor (GDF) such asYip3; tumor susceptibility gene 101 (TSG101); TIP47; v-SNARE; mannose-6-phosphate receptor; t-SNARE; a GDP dissociation inhibitor, a guanine-nucleotide exchange factor (GEF), mapmodulin, or combinations thereof. In a specific example, a Rab9A modulator is PIKfyve, p40, or TIP47.

### Rab11A modulators

Rab11A modulators include nucleic acid and protein sequences that interact directly or indirectly with Rab11A. In some examples, modulators increase Rab11A activity, for example by increasing transcription or translation of Rab11A mRNA. In other examples, modulators decrease Rab11A activity, for example by decreasing transcription or translation of Rab11A mRNA. For example, some Rab11A modulators directly bind to Rab11A, thereby enhancing the biological activity of Rab11A. In particular examples, a Rab11A modulator modulates Rab11A expression by transcriptional activation or by binding to Rab11A. In some examples, a Rab11A modulator is a protein involved in trafficking proteins or other molecules from the trans-golgi to the cell membrane by interaction with Rab11A. Therefore, a Rab11A modulator can be a protein that binds to, or is involved in a complex with Rab11A, and is involved in the trafficking of proteins and other molecules from the trans-golgi to the cell membrane.

Exemplary Rab11A modulators include, but are not limited to: Sec15, Rip11, FIP1, FIP2, FIP3/eferin, FIP4, Rab-coupling protein (RCP), or combinations thereof..

### Methods for decreasing activity

One particular method that can be used to decrease the biological activity of a Rab9A or Rab11A nucleic acid or protein sequence (for example by decreasing the activity of modulator that increases the activity of Rab9A or Rab11A) is to decrease or disrupt transcription or translation of an mRNA encoding Rab9A or Rab11A (or a modulator thereof), or combinations thereof, in the cell.

In one example, transcription or translation of at least one (such as at least 2, at least 3, or at least 4) of a Rab9A, Rab11A, a Rab9 modulator, or a RAb11A modulator is decreased. For example, transcription or translation of Rab9A can be decreased or inhibited. In another example, transcription or translation of Rab11A is decreased. In a particular example, transcription or translation of one or more Rab9A modulators is decreased. In another example, transcription or translation of one or more Rab11A modulators is decreased. In another example, transcription or translation of Rab9A and at least one (such as at least 2, at least 3, or at least 4) Rab9A modulator is decreased. In yet another example, transcription or translation of Rab11A and at least one (such as at least 2, at least 3, or at least 4) Rab11A modulator is decreased. In another example, transcription or translation of Rab9A and at least one (such as at least 2, at least 3, or at least 4) Rab11A modulator is decreased. In yet another example, transcription or translation of Rab11A and at least one (such as at least 2, at least 3, or at least 4) Rab9A modulator is decreased. In a specific example, transcription or translation of Rab9A, Rab11A and at least one (such as at least 2, at least 3, or at least 4) Rab9A modulator is decreased. In another specific example, transcription or translation of Rab9A, Rab11A, and at least one (such as at least 2, at least 3, or at least 4) Rab11A modulator is decreased. In yet another specific example, transcription or translation of Rab9A, Rab11A, and at least one (such as at least 2, at least 3, or at least 4) Rab11A modulator, and at least one (such as at least 2, at least 3, or at least 4) Rab9A modulator is decreased.

Based on publicly available Rab9A, Rab11A, Rab9A modulator, and Rab11A modulator nucleic acid sequences, including variants, fusions and fragments of such sequences, methods that can be used to interrupt or alter transcription of such nucleic acid molecules include, but are not limited to, site-directed mutagenesis (including mutations caused by a transposon or an insertional vector), providing a DNA-binding protein that binds to the coding region of the protein (thus blocking or interfering with RNA polymerase or another protein involved in transcription), disrupting expression of Rab9A, Rab11A, a Rab9A modulator, or a Rab11A modulator, coding sequence (for example by functionally deleting the coding sequence, such as by a mutation, insertion, or deletion), altering the amino acid sequence or overall shape of Rab9A, Rab11A, a Rab9A modulator protein, or a Rab11A modulator protein, degrading Rab9A, Rab11A, a Rab9A modulator protein, or a Rab11A modulator protein, or combinations thereof.

Various inactive and recombinant DNA-binding proteins, and their effects on transcription, are discussed in Lewin, *Genes VII.* Methods that can be used to interrupt or alter translation of a nucleic acid molecule include, but are not limited to, using an antisense RNA, ribozyme or an siRNA that binds to a messenger RNA transcribed by the nucleic acid encoding Rab9A, Rab11A, a Rab9A modulator, or a Rab11A modulator. Such methods can be used to decrease or inhibit expression of a Rab9A, a Rab11A, a Rab9A modulator, or a Rab11A modulator nucleic acid molecule, to reduce pathogen infection.

For example, the amount mRNA can be decreased in the cell by contacting the mRNA with a molecule that binds to a Rab9A, Rab11A, Rab9A modulator, or a Rab11A modulator, messenger RNA, such as an antisense RNA, ribozyme, triple helix molecule, miR,or siRNA that is specific for the mRNA, for example by administering to the cell the antisense RNA, ribozyme, triple helix molecule, miR, or siRNA. In one example, antisense RNA, triple helix molecule, ribozyme, miR, or siRNA molecules are contacted with the cell under conditions that permit the molecule to be introduced into the cell. In a particular example, an expression vector that transcribes one or more antisense RNA, ribozyme, triple helix molecule, miR, or siRNA sequences that recognize a Rab9A, Rab11A, Rab9A modulator, or Rab11A modulator mRNA sequence is used to transform cells.

Particular siRNA, antisense, and ribozyme molecules are disclosed herein. For example, any of SEQ ID NOS: 5-9, 47-48, and 57-58 (or combinations thereof, such as at least 2, at least 3, or at least 4 of these sequences) can be used to decrease an amount of Rab9A mRNA in the cell. Similarly, any of SEQ ID NOS: 20-32, 51-56 and 61-66 (or combinations thereof, such as at least 2, at least 3, or at least 4 of these sequences) can be used to decrease an amount of one or more Rab9A modulator mRNAs in the cell. In addition, any of SEQ ID NOS: 16-19, 49-50, and 59-60 (or combinations thereof, such as at least 2, at least 3, or at least 4 of these sequences) can be used to decrease an amount of Rab11A mRNA in the cell. However, this disclosure is not limited to the use of particular molecules that decrease mRNA.

In particular examples, decreasing the biological activity of Rab9A or Rab11A includes decreasing the interaction between Rab9A, Rab11A, a Rab9A modulator, or a Rab11A modulator nucleic acid or peptide sequences, and a pathogen protein. Methods for decreasing or inhibiting the interaction between a pathogen protein and a Rab9A, Rab11A, a Rab9A modulator, or a Rab11A modulator protein or nucleic acid sequence are known. The pathogen and Rab9A, Rab11A, Rab9A modulator, or Rab11A modulator proteins or nucleic acid molecules can be part of an *in vitro* solution, an *in vivo* expression system, or *in situ* with a host tissue or subject. The pathogen protein can be part of a larger molecule or complex, such as an envelope protein on the envelope of a mature virus or a fragment of a viral envelope. The Rab9A, Rab11A, Rab9A modulator, or Rab11A modulator protein also can be part of a larger molecule or complex, such as a Rab9A, Rab11A, Rab9A modulator, or Rab11A modulator peptide expressed as part of a fusion protein or contained as one subunit of a larger protein, such as a transport protein, cell receptor, structural protein, or an enzyme. A Rab9A, Rab11A, Rab9A modulator, or Rab11A modulator nucleic acid molecule can be part of a larger molecule, complex, organism or microorganism such as a nucleic acid sequence contained within a host genome, a recombinant vector, or a transgenic organism or microorganism (including both extrachromosomal molecules or genomic insertions).

In one example, the pathogen protein is a virus (such as an enveloped RNA virus) and decreasing the interaction of the virus and Rab9A, Rab11A, Rab9A modulator, or Rab11A modulator protein decreases or inhibits infection of a host cell by the virus. In particular example, decreasing such an interaction includes decreasing the integration of the viral nucleic acid (such as a viral genome) into the host nucleic acid (such as a host genome).

In one example, interaction is decreased or inhibited between one or more pathogen proteins and at least one (such as at least 2, at least 3, or at least 4) of a Rab9A, Rab11A, a Rab9 modulator, or RAb11A modulator nucleic acid molecule or peptide. For example, interaction can be decreased or inhibited between one or more pathogen proteins and a Rab9A nucleic acid molecule or peptide. In another example, interaction is decreased or inhibited between one or more pathogen proteins and a Rab11A nucleic acid molecule or peptide. In a particular example, interaction is decreased or inhibited between one or more pathogen proteins and one or more Rab9A modulator nucleic acid molecules or peptides. In another example, interaction is decreased or inhibited between one or more pathogen proteins and one or more Rab11A modulator nucleic acid molecules or peptides. In another example, interaction is decreased or inhibited between one or more pathogen proteins and a Rab9A nucleic acid molecule or peptide, as well as between one or more pathogen proteins and at least one (such as at least 2, at least 3, or at least 4) Rab9A modulator nucleic acid molecule or peptide. In yet another example, interaction is decreased or inhibited between one or more pathogen proteins and a Rab11A nucleic acid molecule or peptide, as well as between one or more pathogen proteins and at least one (such as at least 2, at least 3, or at least 4) Rab11A modulator nucleic acid molecule or peptide. In another example, interaction is decreased or inhibited between one or more pathogen proteins and a Rab9A nucleic acid molecule or peptide, as well as between one or more pathogen proteins and at least one (such as at least 2, at least 3, or at least 4) Rab11A modulator nucleic acid molecule or peptide. In yet another example, interaction is decreased or inhibited between one or more pathogen proteins and a Rab11A nucleic acid molecule or peptide, as well as between one or more pathogen proteins and at least one (such as at least 2, at least 3, or at least 4) Rab9A modulator nucleic acid molecule or peptide. In a specific example, interaction is decreased or inhibited between one or more pathogen proteins and a Rab9A nucleic acid molecule or peptide, as well as between one or more pathogen proteins and at least one (such as at least 2, at least 3, or at least 4) Rab9A modulator nucleic acid molecule or peptide, as well as between one or more pathogen proteins and a Rab11A modulator nucleic acid molecule or peptide. In another specific example, interaction is decreased or inhibited between one or more pathogen proteins and a Rab9A nucleic acid molecule or peptide, as well as between one or more pathogen proteins and at least one (such as at least 2, at least 3, or at least 4) Rab11A modulator nucleic acid molecule or peptide, as well as between one or more pathogen proteins and a Rab11A modulator nucleic acid molecule or peptide. In yet another specific example, interaction is decreased or inhibited between one or more pathogen proteins and a Rab9A nucleic acid molecule or peptide, as well as between one or more pathogen proteins and at least one (such as at least 2, at least 3, or at least 4) Rab11A and Rab9A modulator nucleic acid molecules or peptides, as well as between one or more pathogen proteins and a Rab11A modulator nucleic acid molecule or peptide. Decreasing or inhibiting the interactions of one or more of Rab9A, Rab11A, a Rab9 modulator, or a RAb11A modulator nucleic acid molecule or peptide with one or more pathogen proteins can have additive or exponentially increasing effects.

Methods that can be used to disrupt or decrease such an interaction include those described above for decreasing expression of Rab9A, Rab11A, a Rab9A modulator, or a Rab11A modulator. For example, exemplary host Rab9A peptides are encoded by target sequences associated with SEQ ID NOS: 1-2. Disrupting the expression of a nucleic acid sequence including any target sequence associated with SEQ ID NOS: 1-2 can reduce or prevent production of the corresponding Rab9A peptide, and without access to the Rab9A peptide, a pathogen (such as a pathogen that hijacks a lipid raft) cannot infect the host cell. Even if expression of the Rab9A, Rab11A, Rab9A modulator, or Rab11A modulator nucleic acid molecule is not completely disrupted, pathogen infection can still be reduced or even inhibited. For example, interference with a host protein encoded by any target sequence associated with SEQ ID NOS: 1-2 reduces the number of Rab9A proteins within that host cell available for recognition by a pathogen, thus inhibiting infection by the pathogen.

In particular examples, the interaction between Rab9A, Rab11A, one or more Rab9A modulators, or one or more Rab11A modulator nucleic acid or peptide sequences and a pathogen protein is decreased by disrupting or decreasing expression of the Rab9A, Rab11A, or modulator thereof. Decreased expression of Rab9A, Rab11A, or modulator thereof will result in a decreased amount of nucleic acid molecule or protein available for interacting with the pathogen protein. For example, the methods for described above for decreasing an amount of Rab9A, Rab11A, or modulator thereof mRNA in the cell can be used. Such methods will decrease or even inhibit transcription of an mRNA encoding Rab9A, Rab 11A, or modulator thereof. In particular examples, mRNA transcription is decreased or inhibited by inserting a transposon or insertional vector into a Rab9A, Rab11A, or modulator thereof, coding region.

Another example of a method that can be used to decrease the interaction between Rab9A, Rab11A, one or more Rab9A modulators, or one or more Rab11A modulator nucleic acid or peptide sequences, and a pathogen protein, is to administer an agent that decreases, inhibits, or disrupts the interaction (for example, a binding interaction) between a Rab9A modulator and Rab9A, between a Rab11A modulator and Rab11A, or an interaction between Rab9A, a Rab9A modulator, Rab11A, or a Rab11A modulator, and a pathogen protein. Agents that recognize Rab9A, Rab11A, a Rab9A modulator, or a Rab11A modulator protein can prevent a pathogen or portion thereof, such as a pathogen protein (such as a viral protein) from binding to Rab9A, Rab11A, Rab9A modulator, or Rab11A modulator, thereby decreasing or inhibiting infection by the pathogen. For example, a monoclonal or polyclonal antibody that binds to a Rab9A, or a Rab9A modulator protein can block the binding of HIV, Ebola, RSV, Marburg, or measles virus to Rab9A, thus blocking infection of that cell. Similarly, a monoclonal or polyclonal antibody that binds to a Rab11A, or a Rab11A modulator protein can block the binding of HIV, Ebola, RSV, Marburg, or measles virus to Rab11A, thus blocking infection of that cell.

Examples of such agents include, but are not limited to, an anti-protein binding agent that specifically binds to Rab9A, Rab11A, a Rab9A modulator, or a Rab11A modulator, such as an antibody, peptide, or chemical. Antibodies that specifically bind to Rab9A are known in the art (for example see Soldati et al., Mol. Biol. Cell 4:425-34, 1993 and # 552101CB from Voigt Global Distribution LLC), as are antibodies that bind Rab9A modulators, such as TIP47 (for example see Diaz and Pfeffer, Cell 93:433-43, 1998), PIKfyve (for example see Sbrissa et al., J. Biol. Chem. 274:21589-97, 1999), and M6PR (for example see Lombardi et al., EMBO J. 12:677-82, 1993). Rab11A antibodies are also publicly available (#R5903 from Sigma, St. Louis, MO; and available from Santa Cruz Biotechnology, Santa Cruz, CA), and antibodies that recognize a Rab 11A modulators, such as FIP 1 (for example see Preker et al., Cell 81:379-89, 1995), as well as FIP2 and FIP3/eferin (Meyers et al., J. Biol. Chem., 277:49003-10, 2002), are known in the art. Such anti-protein binding agents can decrease the interaction between a Rab9A protein, Rab11A protein, a Rab9 modulator protein, or a Rab11 modulator protein, and the pathogen.

In examples where a Rab9A or a Rab11A modulator peptide is a cell receptor or part of a cell receptor, decreasing or inhibiting expression of the peptide, or altering the three-dimensional structure of the peptide, can reduce or inhibit the interaction between the receptor and a pathogen protein. Similarly, decreasing, inhibiting or preventing expression of a Rab9A or Rab 11A modulator ligand peptide (or altering the structure of such a ligand) can decrease or inhibit an interaction between the pathogen protein and the ligand.

### Methods of Treating or Preventing Infection

The methods disclosed herein for decreasing the biological activity of Rab9A or Rab11A (for example by decreasing the activity of a Rab9A or Rab11A modulator that increases Rab9A or Rab11A activity, respectively, or by increasing the activity of a Rab9A or Rab11A modulator that decreases Rab9A or Rab11A activity, respectively) can also be used to treat or prevent infection by a pathogen, such as a pathogen that hijacks a lipid raft. In particular examples, decreasing the biological activity of Rab9A, Rab11A, or a modulator thereof that increases Rab9A or Rab11A activity, does not require a 100% reduction. For example, decreases of at least 20%, at least 40%, at least 50%, at least 60%, at least 75%, at least 80%, at least 90%, at least 95%, or at least 99%, as compared to a control (such as an amount of Rab9A or Rab11A activity in a cell not treated with a therapeutic agent), can be sufficient to treat or prevent an infection.

For example, functional deletion in a cell of a Rab9A gene, a Rab11A gene, or a modulator gene that increases Rab9A or Rab11A activity, can result in cells having a non-functional Rab9A, non-functional Rab11A, or a non-functional Rab9A or Rab11A modulator that increases Rab9A or Rab11A activity, which results in a cell, such as a cell in a subject, having increased resistance to infection by a pathogen. Such resistance can be used to provide a prophylactic effect in the subject, thereby reducing or even preventing infection of the subject by a pathogen. In other examples, functional deletion of a Rab9A gene, a Rab11A gene, a Rab9A modulator gene, or a Rab11A modulator gene, results in a cell, such as a cell in a subject, having a decreased amount of pathogen (such as pathogen nucleic acid sequences, peptides, or entire pathogens). Such a decrease can be used to provide a therapeutic effect in the subject, thereby reducing or even eliminating one or more symptoms of infection in the subject.

Methods for treating an existing pathogen infection in a subject, or for providing a prophylactic effect to a subject who is susceptible to a pathogen infection, are disclosed. In particular examples, the method includes administering to the subject a therapeutically effective amount of an agent that decreases the biological activity of Rab9A or Rab 11A (for example by decreasing the activity of a Rab9A or Rab11A modulator that increases Rab9A or Rab11A activity, respectively, or by increasing the activity of a Rab9A or Rab 11A modulator that decreases Rab9A or Rab11A activity, respectively). Any mode of administration can be used. The agent that decreases the biological activity of Rab9A, Rab111A (or modulator thereof that increases Rab9A or Rab11A activity) is administered at a therapeutically effective dose. However, multiple administrations may be required to achieve the therapeutically effective dose. The agent that decreases the biological activity of Rab9A or Rab11A nucleic acid or protein sequence (or a modulator of Rab9A or a Rab11A) can be administered to the subject alone or in combination with other agents. Therapeutic amounts of such agents can be administered to a subject for the treatment of a pathogen infection or as a prophylactic measure prior to exposure of the subject to the pathogen. After the agent has taken effect, the subject can be monitored for one or more symptoms associated with infection.

In one example, the agent interferes with the interaction between a pathogen and a Rab9, a Rab11A, a Rab9A modulator, or a Rab11A modulator protein or nucleic acid sequence. For example, decreasing or even inhibiting the interaction between a pathogen and a Rab9A, a Rab11A, a Rab9A modulator, or a Rab 1A modulator protein or nucleic acid sequence can decrease, inhibit, or even prevent infection of a subject the pathogen, or otherwise decrease or inhibit the progression or clinical manifestation of the infection. In addition, decreasing the interaction of a pathogen and a Rab9A, a Rab11A, a Rab9A modulator, or a Rab11A modulator protein or nucleic acid sequence can reduce or alleviate one or more symptoms associated with infection, such as a fever.

In one example, an agent that decreases or disrupts expression of Rab9A or Rab11A coding sequence (for example by decreasing expression of a modulator that increases Rab9A or Rab11A activity) is contacted with a cell, for example by administration to a subject. Such an agent can be used for prophylactic or therapeutic purposes. For example, antisense oligonucleotides, ribozymes, triple helix molecules, miRs, and siRNA molecules that recognize Rab9A, Rab11A, or a modulator thereof, can be administered to the subject to disrupt expression of Rab9A, Rab11A, or a modulator thereof, respectively. In a particular example, an expression vector including antisense RNA, ribozyme, triple helix molecule, miR, or siRNA molecules that target Rab9A, Rab11A, or a modulator thereof nucleic acid sequence is introduced into the bone marrow of a subject. Uptake of the vector and expression of the antisense RNA, ribozyme, triple helix molecule, miR, or siRNA within cells infected by a pathogen, such as a pathogen that can hijack a lipid raft (such as HIV, RSV, Marburg, measles, or Ebola), offers a prophylactic or therapeutic effect by decreasing expression of Rab9A, Rab11A, or a modulator thereof that increases Rab9A or Rab11A activity, within those cells, thus decreasing or even inhibiting infection by the pathogen. In particular examples, expression of the antisense RNA, ribozyme, triple helix molecule, miR, or siRNA is under control of a promoter, such as an inducible promoter. The vector, or other nucleic acid molecule, can be introduced into a subject by any standard molecular biology method and can be included in a composition that includes a pharmaceutically acceptable carrier.

Examples of other molecules which can be used to decrease the interaction between a pathogen and a Rab9A, Rab11A, Rab9A modulator, or a Rab11A modulator protein or nucleic acid sequence (such as binding between such sequences) include, but are not limited to specific binding agent, such as such as an antibody, peptide, or other compound that recognizes Rab9A, Rab 11A or a modulator thereof. For example, an agent that interferes with the interaction between Rab9A and a pathogen or pathogen protein (such as an HIV-protein) provides a prophylactic or therapeutic effect against infection by a pathogen (such as HIV) when provided to a cell or administered to a subject.

In particular examples, peptides or peptide analogs having a structure that mimics a Rab9A peptide, a Rab11a peptide, a Rab9A modulator peptide, or a Rab11A modulator peptide, can be used for prophylactic or therapeutic uses. For example, such peptides or peptide analogs recognized by a pathogen can be administered to a subject as a pharmaceutical composition. These polypeptides interact with a pathogen already infecting that subject, or provide a prophylactic defense mechanism against infection if the subject is at risk of exposure to a pathogen. For example, peptides structurally similar to Rab9A are recognized by HIV. If such polypeptides are administered to an HIV-positive subject, the viruses already present in the subject interact with those peptides in addition to that subject's T-cell receptors, thus inhibiting the rate at which HIV infects T-cells. The administered peptides act as "decoys" to block HIV from interacting with T-cell receptors.

### Identification of Rab9A and Rab11A Modulators

The present disclosure provides methods of identifying Rab9A and Rab11A modulators involved in infection by a pathogen, such as a pathogen that hijacks a lipid raft. In particular examples, the method includes reducing (such as inhibiting) the expression of a putative Rab9A (or Rab11A) modulator in a cell that expresses Rab9A (or Rab11A), or reducing the biological activity of the putative Rab9A (or Rab11A) modulator in a cell that expresses Rab9A (or Rab11A). The cell is contacted with a desired pathogen (such as a pathogen that hijacks a lipid raft), and an amount of infection by the pathogen determined. A decrease (and in some examples elimination) in infection indicates that the putative modulator of Rab9A (or Rab11A) is a Rab9A (or Rab11A) modulator involved in infection by a pathogen. In particular examples, a decrease in infection of at least 40%, at least 50%, at least 60%, at least 75%, at least 80%, at least 90%, at least 95%, or at least 100%, as compared to a control (such as infection of a cell not having reduced expression of a putative Rab9A (or Rab11A) modulator), indicates that the putative Rab9 modulator is a Rab9 modulator involved in infection by a pathogen. In one example, the pathogen is a virus, such as an enveloped RNA virus, for example HIV, Ebola, Marburg, RSV, or measles virus.

The method can further include associating the amount of infection with an amount of Rab9A (or Rab11A) (such as an amount of Rab9A (or Rab11A) nucleic acid molecule, for example cDNA or mRNA, or protein in the cell) or an amount of Rab9A (or Rab11A) biological activity in the cell, wherein the presence of a decrease in infection and a decrease in the amount of Rab9A (or Rab11A) or Rab9A activity (or Rab11A activity) indicates that the putative modulator of Rab9A (or Rab11A) is a modulator of Rab9A (or Rab11A) involved in infection by the pathogen.

One of skill in the art can also assess the effect(s) of inhibiting a Rab9A (or Rab11A) modulator on Rab9A (or Rab11A) biological activity, for example by observing cellular changes that are associated with Rab9A (or Rab11A) inhibition. For example, a cell can be exposed to a test agent, and the agent's effect on Rab9A (or Rab11A) activity can be assessed by measuring differences in vesicle transport from late endosomes to the trans-Golgi (or from the trans-Golgi to the cell membrane for Rab11A). Methods for visualizing this process are known in the art (for example, see Barbero et al. J. Cell. Biol. 156:511-8, 2002, herein incorporated by reference). In another example, the binding of Rab9A (or Rab11A) to another molecule that is known to stimulate or inhibit Rab9A (or Rab11A) activity is measured. The interaction between Rab9A (or Rab11A) and other proteins can be direct or indirect. An example of a direct interaction is the binding of Rab9A (or Rab11A) to another protein. In yet another example, expression of Rab9A (or Rab11A) in the presence of the test agent is measured, for example by determining a level of Rab9A (or Rab11A) mRNA in a cell and assessing whether Rab9A (or Rab11A) expression is decreased by the test agent.

### Screening for Agents that Decrease Infection

Based on the observation that Rab9A, Rab11A, and Rab9A modulators (and similar results are expected with Rab11A modulators) are involved in pathogen infection, methods are disclosed for identifying agents that decrease or inhibit the binding of a pathogen (such as a pathogen protein) to a Rab9A, Rab11A, Rab9A modulator, or a Rab11A modulator nucleic acid molecule or protein. Such agents can be used to decrease pathogen infection, as well as to treat or prevent pathogen infection using the methods described herein.

In some examples, a Rab9A molecule, Rab11A molecule, or modulator molecule thereof, such as one or more of a Rab9A, Rab11A, Rab9A modulator, or Rab11A modulator protein or nucleic acid molecule, is contacted with a pathogen or portion thereof (such as a pathogen protein). Particular examples of pathogens include, but are not limited to, a virus, a viral envelope, a viral protein (such as a viral envelope protein), a bacterium, a bacterial protein, a fungus, a fungal protein, a protozoa, or a protozoa protein. One or more test agents are contacted with the one or more of Rab9A, Rab11A, a Rab9A modulator, or a Rab11A modulator protein or nucleic acid molecules, and the pathogen, before, during or after contacting a Rab9A molecule, a Rab11A molecule, or modulator molecule thereof, and the pathogen. In particular examples, the Rab9A molecule, Rab11A molecule, or modulator molecule thereof, is in a cell, and contacting the Rab9A molecule, Rab11A molecule, or modulator molecule thereof, with the pathogen and a test agent includes exposing the cell to the pathogen and to the test agent. Subsequently, it is determined whether binding of the pathogen to the Rab9A molecule, Rab11A molecule, or modulator molecule thereof, is decreased in the presence of the test agent, wherein a decrease in binding is an indication that the test agent decreases the binding of the pathogen to Rab9A, Rab11A, or a modulator of Rab9A or Rab11A, and therefore is an agent that can be used to decrease, treat, or even prevent infection by a pathogen. In particular examples, the Rab9A, Rab11A, or modulator of Rab9A or Rab11A molecule (such as a protein), the pathogen (such as a pathogen protein), or both, includes a detectable label, and determining whether binding is decreased includes detecting an amount of label present.

A decrease in binding can be determined by a comparison to a reference standard, such as an amount of binding reported in the scientific literature, or to a control (such as an amount of binding in the absence of the test agent). Any suitable compound or composition can be used as a test agent. Particular examples include, but are not limited to: organic or inorganic chemicals, including aromatics, fatty acids, and carbohydrates; peptides, such as monoclonal antibodies, polyclonal antibodies, and other specific binding agents; and nucleic acid molecules. The pathogen or pathogen protein can be obtained from any suitable pathogen, such as HIV, RSV, Marburg, Ebola, measles virus, E. *coli, Pseudomonas,* and so forth.

For example, the binding of Rab9A, Rab11A, or a modulator thereof, to HIV (or an HIV envelope glycoprotein) can be determined in the presence of a test agent. A decrease in binding activity between the Rab9A, Rab11A, or a Rab9 or a Rab11A modulator, and HIV, indicates that the test agent decreases the binding of HIV to Rab9A, Rab11A, or a modulator thereof, and the agent is a candidate for use as an anti-HIV agent.

Therapeutic agents identified with the disclosed approaches can be used as lead compounds to identify other agents having even greater anti-pathogen activity, such as antiviral or antibacterial activity. For example, chemical analogs of identified chemical entities, or variant, fragments of fusions of peptide agents, can be tested for their ability to decrease pathogen infection using the disclosed assays. The compounds identified utilizing these methods can be used to reduce infection in cells either *in vitro, ex vivo* or *in vivo.* Candidate agents can also be tested for safety in animals and then used for clinical trials in animals or humans.

### Transgenic Cells and Non-Human Mammals

Transgenic animal models, including recombinant and knock-out animals, that include a functionally deleted Rab9A, Rab11A, or a modulator thereof that increases Rab9A or Rab11A activity, can be generated using methods known in the art. Transgenic animals, methods of making and using transgenic animals, are known (for example see WO 01/43540; WO 02/19811; U.S. Pub. Nos: 2001-0044937 and 2002-0066117; and U.S. Pat. Nos: 5,859,308; 6,281,408; and 6,376,743; and the references cited therein).

Such animals will have decreased suseptibility to infection by a pathogen, such as a pathogen that hijacks a lipid raft. Exemplary transgenic non-human mammals include, but are not limited to, non-human primates, mice, rats, chickens, cows, and pigs. In certain examples, a transgenic non-human mammal has a knock-out of any target sequence associated with SEQ ID NO: 1 or 2, and has a decreased susceptibility to infection by a pathogen that uses a lipid raft. The disclosed knock-out animals are useful for reducing the transmission of pathogens (such as viruses) from animals to humans. In addition, animal pathogens that utilize the same targets provided herein can be decreased in the animals.

Expression of the sequence used to knock-out or functionally delete the Rab9A, Rab11A, or a modulator thereof that increases Rab9A or Rab11A activity, can be regulated by choosing the appropriate promoter sequence. For example, constitutive promoters can be used to ensure that the functionally deleted gene is not expressed by the animal. In contrast, an inducible promoter can be used to control when the transgenic animal does or does not express the gene of interest. Exemplary inducible promoters include tissue-specific promoters and promoters responsive or unresponsive to a particular stimulus (such as light, oxygen, chemical concentration, such as a tetracycline inducible promoter).

For example, a transgenic mouse including a Rab9A gene, Rab11A gene, or a modulator gene thereof that increases Rab9A or Rab11A activity, or a mouse having a disrupted Rab9A gene, Rab11A gene, or a modulator gene thereof that increases Rab9A or Rab11A activity, can be examined during exposure to various pathogens. Comparison data can provide insight into the life cycles of pathogens. Moreover, knock-out animals (such as primates) that are otherwise susceptible to an infection (for example SIV) can be made to determine the resistance to infection conferred by disruption of the gene.

Cells including a functionally deleted Rab9A gene, Rab 11A gene, or a modulator gene thereof that increases Rab9A or Rab11A activity, are resistant to infection by a pathogen, such as a pathogen that hijacks a lipid raft. Such cells may therefore include cells having decreased susceptibility to infection by an enveloped RNA virus, such as HIV, Ebola, Marburg, measles, RSV, or any of the other pathogens described herein, including bacteria and fungi. The cells of the present disclosure also include hematopoietic stem cells with a functionally deleted Rab9A gene, Rab11A gene, Rab9A modulator gene, or Rab11A modulator gene.

### Screening for Resistance to Infection

Also provided herein are methods of screening host subjects for resistance or susceptibility to infection by characterizing a host Rab9A, Rab11A, Rab9A modulator, or Rab11A modulator nucleic acid or amino acid sequence, or combinations thereof. For example, a Rab9A modulator (or Rab9A, Rab11A, or a Rab11A modulator) nucleic acid molecule can be isolated from a subject, sequenced, and compared to the wild-type (or native) sequence for the Rab9A modulator (or Rab9A, Rab11A, or a Rab11A modulator, respectively). The greater the similarity between that subject's Rab9A modulator (or Rab9A, Rab11A, or a Rab11A modulator) nucleic acid sequence and the wild-type Rab9A modulator (or Rab9A, Rab11A, or a Rab11A modulator, respectively) sequence, the more susceptible that subject is to infection, while a decrease in similarity between that subject's Rab9A modulator (or Rab9A, Rab11A, or a Rab11A modulator) nucleic acid and the wild-type Rab9A (or Rab9A, Rab11A, or a Rab11A modulator, respectively) modulator sequence, the more resistant that subject may be to infection.

Assessing the genetic characteristics of a population can provide information about the susceptibility or resistance of that population to pathogen infection, such as viral infection. For example, polymorphic analysis of alleles in a particular human population, such as the population of a particular city or geographic area, can indicate how susceptible that population is to infection. A higher percentage of alleles substantially similar to wild-type sequences indicates that the population is more susceptible to infection, while a large number of polymorphic alleles that are substantially different than wild-type sequences indicates that a population is more resistant to infection. Such information can be used, for example, in making public health decisions about vaccinating susceptible populations.

The present disclosure also provides a method of screening a cell for a variant of Rab9A, Rab11A, or modulators thereof, which is associated with decreased susceptibility to infection by a pathogen. A variant of Rab9A, Rab11A, a Rab9A modulator, or a Rab11A modulator, can be a gene with a functional deletion or alteration in the gene, for example, a mutation, an insertion, duplication or the like, such that the amount or activity of the gene product is altered. These cells containing a variant of a Rab9A gene, Rab11A gene, a Rab9A modulator gene, or a Rab11A modulator gene, can be contacted with a pathogen to determine if cells that have a naturally occurring variant of a Rab9A gene, Rab11A gene, a Rab9A modulator gene, or a Rab11A modulator gene, differ in their resistance to infection by a pathogen, such as a pathogen that hijacks a lipid raft. For example, cells from an animal, such as a chicken, can be screened for a variant form of Rab9A, Rab11A, or a modulator thereof. If a naturally occurring variant is found and chickens possessing a variant form of the gene in their genome are less susceptible to infection, these chickens can be selectively bred to establish flocks that are resistant to infection. Similarly, other animals can be screened for a variant form of Rab9A, Rab11A or a modulator thereof.

If a naturally occurring variant is found and animals possessing a variant form of the gene in their genome are less susceptible to infection, these animals can be selectively bred to establish populations that are resistant to infection. These animals include, but are not limited to, cats, dogs, livestock (for example, cattle, horses, pigs, sheep, goats, and so forth), laboratory animals (for example, mouse, rabbit, rat, gerbil, guinea pig, and so forth) and avian species (for example, chickens, turkeys, ducks, pheasants, pigeons, doves, and so forth).

### Example 1

### Rab9A siRNA Molecules Decrease HIV Replication

This example describes methods used to express siRNAs that recognize Rab9A (such as a target sequence associated with SEQ ID NOS: 1-2), KOX4 (similar to KOX4 (LOC131880) and LOC166140); KIAA 1259; F3 (tissue factor 3; thromboplastin); AXL (AXL receptor tyrosine kinase); SelB (the elongation factor for selenoprotein translation); FKBP38 (a chaperone that shuttles Bcl-2 to the mitochondria), to determine if such expression can decrease HIV replication and infection.

SMARTpool siRNA oligonucleotides directed against human Rab9A mRNA at positions 55-73 (GGGAAGAGUUCACWAUGA; SEQ ID NO: 5), 147-165 (GGAAGUGGAUGGACAUUUU; SEQ ID NO: 6), 274-292 (UCACAAAGCUUCCAGAACU; SEQ ID NO: 7), and 368-386 (GUAACAAGAUUGACAUAAG; SEQ ID NO: 8) were from Dharmacon (Lafayette, CO). These sequences show the siRNA sequence of the sense strand; the opposite strand is hybridized to the sense strand to form the therapeutic siRNA molecules. These siRNA sequences have UU as 3' overhangs on each strand and 5'-phosphate on the anitsense strand only. However, the disclosure is not limited to these positions. For example, one skilled in the art will appreciate that siRNA sequences can be used that recognize the Rab9A sequence fragment AAGUUUGAUACCCAGCUCUUC (SEQ ID NO: 9). siRNA sequences were also used that recognized KOX4; KIAA 1259; F3; AXL; SelB; and FKBP38.

Four siRNA sequences for each gene (Rab9A, KOX4, KIAA1259, F3, AXL SelV, and FKBP38) were separately pooled. Each of the pools of siRNAs, hybridized to its appropriate complement sequence, were used to transfect JC53 (HeLa cells modified to accept HIV). Cells were obtained from American Type Culture Collection (ATCC, Mannassas, VA). GFP siRNA sequences were used as a negative control (Qiagen, Inc.).

Cells (20,000 to 250,000) were incubated in serum free media for 24 hours. Cocktails were made by mixing the appropriate duplex siRNAs (50-100 pmoles) with lipofectamine 2000 (4-16 µl) and RNAse Inhibitor (1-4 µl) in a solution of Optimem (serum free medium) in a total volume of 200-2000 µl. The lipofectamine was allowed to incubate at room temperature for 5 minutes before the addition of siRNA. Aliquots (50-500 µl) of the cocktail were added to the cells which were incubated at 37°C for 48 hours. The cells were then infected with HIV and the incubation continued for 3-7 days. Following transfection, several assays were conducted to confirm transfection efficiency, and to determine the resistance of the cells to infection.

Quantitation of p24 levels in HIV infected JC53-BL cells was determined using the Coulter HIV-1 p24 Antigen Neutralization Kit according to the manufacturers recommendation. Briefly, two days post-transfection with siRNA, JC53-BL cells were infected overnight with the X4-tropic HIV LAV (MOI = 1). Subsequent to LAV infections, HIV p24 assays were performed by seeding 25,000 cells per well into 24-well plates, and p24 production was assayed from supernatants on day 3 post-infection using the HIV-1 p24 Antigen ELISA Test System (Beckman/Coulter/Immunotech, Brea, CA). Luciferase assays were performed in quadruplicate by measuring luciferase activity in detergent lysates one day post-infection with LAV (MOI = 1) using the Steady-Glo Luciferase assay system (Promega, Madison, WI) and an EL 312e Microplate Bio-kinetics Reader (Bio-Tek Instruments, Winooski, VT). The luciferase assays permit quantitation of HIV infection of JC53-BL cells ± transfection with the siRNA, whereas p24 assays measure secretion of infectious virus (and virus-like particles) into the supernatant.

The relative amount of Rab9A mRNA in the presence or absence of siRNA molecules was determined as follows. Total mRNA was isolated from parental and siRNA-transfected JC53-BL cells (human Rab9A) or Vero cells (simian Rab9A) using the RNeasy Mini and QIAshredder Kits, 2 or 5 days following the indicated siRNA transfections. The mRNA was reverse transcribed to cDNA using random hexamers (Applied Biosystems, Foster City, CA) at 37°C for 60 minutes. The relative levels of Rab9A message originally present was determined by real time PCR. Real time quantitative PCR was performed with 3 µL of a 1:10 dilution of JC53-BL or Vero cDNA in a 25 µL reaction volume, and quantitated in an Mx4000 Multiplex Quantitative PCR System (Stratagene). Human Rab9A cDNA was detected using the forward primer (5'-TCCTCATTGCGCCCAGAC-3'; SEQ ID NO: 10), the reverse primer (5'-ATCACAGTGCCAGGCTCATTACAG-3'; SEQ ID NO: 11), and the Fam-labeled probe (5'-TTTCGTGTGGCCGCGAGACACTCTT-3'; SEQ ID NO: 12) at final concentrations of 200 nM). Simian Rab9A cDNA was detected using the forward primer (5'-CATCTTCTCAACAACCCTA-3'; SEQ ID NO: 13), the reverse primer (5'-CCATCTCCAAGGAGAATTA-3'; SEQ ID NO: 14), and Fam-labeled probe (5'-CAATGGCAGGAAAATCATCAC-3'; SEQ ID NO: 15). The constitutively expressed gene hypoxanthine-guanine phosphoribosyltransferase (HGPRT) was detected using a human HGPRT TaqMan assay kit (Applied Biosystems). Assay conditions were 10 min at 95°C, followed by 50 cycles of 95°C for 15 seconds and 52°C for 30 seconds. Rab9A mRNA detected in untreated and siRNA-transfected cells was normalized to detected HGPRT mRNA expression levels.

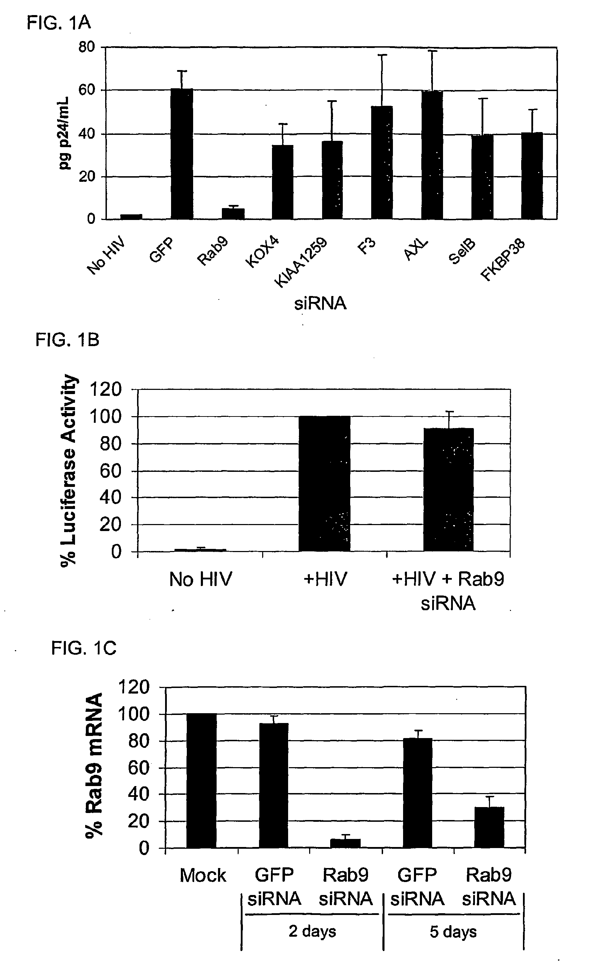

As shown in FIG. 1A, among the siRNAs tested, Rab9A siRNA showed the most pronounced effect in blocking HIV-1 replication (~90% inhibition of p24 production), compared to cells transfected with an irrelevant siRNA control against green fluorescence protein (GFP).

Untransfected and Rab9A siRNA-transfected JC53-BL cells showed similar luciferase activities (FIG. 1B), indicating that they are infected at similar rates. This indicates that the observed defect in the HIV-1 life cycle following Rab9A inhibition occurred downstream of viral entry and early proviral gene expression. The specificity of Rab9A siRNA in inhibiting both HIV-1 replication and Rab9A expression is seen when compared to control siRNA transfections against GFP (FIGS. 1A and 1C).

Although cytoplasmic double stranded RNA > 30 bp induces the interferon response, leading to nonspecific shutdown of protein synthesis and apoptosis, shorter siRNAs like the ones used herein (21 bp) generally do not. It is unlikely that viral inhibition with Rab9A siRNA was due to the interferon response given the lack of strong HIV-1 inhibition by other siRNAs of the same size, but directed against GFP or different cellular messages (FIG. 1A). Further, both JC53-BL and 293T cell lines stably expressing small hairpin RNA against the same Rab9A targets had - 80% Rab9A mRNA knockdown and showed ~70% inhibition of HIV-1 replication, without any discernable effects on viability or growth rate kinetics during 1 month in culture.

### Example 2

### Rab9A Modulators and Rab11A siRNA Molecules Decrease HIV Replication

This example describes methods used to express siRNAs that recognize the Rab9A modulators, TIP47, p40, and PIKfyve, as well as Rab11A, to determine if such expression can decrease HIV replication. The Rab9A siRNAs are described in Example 1. siRNA sequences were also used that recognized TIP47, p40, PIKfyve, and Rab11A (as well as Rin2 and IPO9 as controls). All siRNAs were from Dharmacon (Lafayette, CO).

The following SMARTpool siRNA oligonucleotides directed against human Rab11A mRNA (Accession Number: NM_004663) were used: GUAGGUGCCUUAUUGGUUU (SEQ ID NO: 16); GCAACAAUGUGGUUCCUAU SEQ ID NO: 17); CAAGAGCGAUAUCGAGCUA (SEQ ID NO: 18), and GGAGUAGAGUUUGCAACAA (SEQ ID NO: 19).

The following SMARTpool siRNA oligonucleotides directed against human PIKfyve mRNA (Accession Number: NM_015040) were used: GGAAAUAGCUAUAAUCCUA (SEQ ID NO: 20); GAACCCAGCUCCAUCAWG (SEQ ID NO: 21); GUAGAAUGCUGGCCGAWA (SEQ ID NO: 22); and GGAAAUCUCCUGCUCGAAA (SEQ ID NO: 23).

The following SMARTpool siRNA oligonucleotides directed against human p40 mRNA (Accession Number: NM_005833) were used: CCACAGCUGUUCAUAUUUA (SEQ ID NO: 24); GGAWCAGCUGACAAAGUA (SEQ ID NO: 25); GAAACCAGCUAUAUGUCUU; (SEQ ID NO: 26); and GGAAAUCGAAAUUGUCUAC (SEQ ID NO: 27).

The following SMARTpool siRNA oligonucleotides directed against human TIP47 mRNA (Accession Number: NM_005817) were used: GCAAGGCGUUGAUCAGAAG (SEQ ID NO: 28); GGAACAGAGCUACUUCGUA (SEQ ID NO: 29); UGGAAUAUGUGGCCCAGAA (SEQ ID NO: 30); and GCUUGUGUCGUCUAAGG (SEQ ID NO: 31).

All of these sequences show the siRNA sequence of the sense strand; the opposite strand is hybridized to the sense strand to form the therapeutic siRNA molecules. These siRNA sequences have UU as 3' overhangs on each strand and 5'-phosphate on the anitsense strand only.

However, the present disclosure is not limited to these siRNA sequences. For example, TIP47 can also be targeted with the sequence AACAGAGCUACUUCGUACGUC (SEQ ID NO: 32).

The siRNA sequences were transfected into JC53 cells, and p24 levels quantitated using the methods described in Example 1.

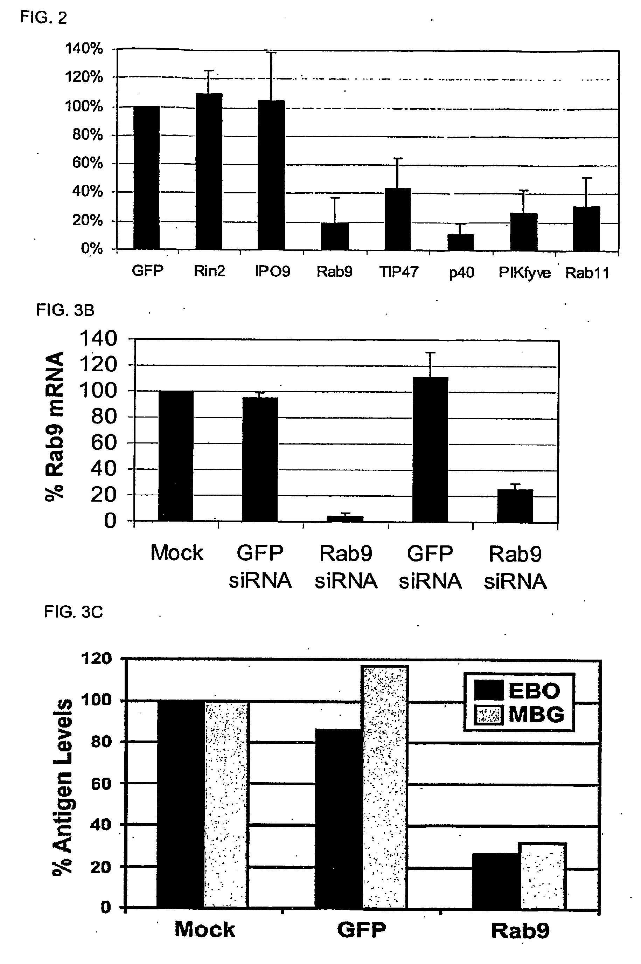

As shown in FIG. 2, Rab9A, Rab11A, and Rab9A modulator siRNAs were able to block HIV-1 replication (about 60-90% inhibition of p24 production), compared to cells transfected with an irrelevant siRNA control against green fluorescence protein (GFP). In contrast, Rin2 and IPO2 sirRNAs had no effect on HIV-1 replication.

### Example 3

### Rab9A siRNA Molecules Decrease Ebola, Marburg, RSV, and Measles Replication

This example describes methods used to demonstrate that Rab9A is utilized in the replication of other viruses, such as other enveloped RNA viruses, for example Ebola, Marburg, measles virus, and RSV.

### Ebola virus and Marburg virus infections and assays

Vero cells (an African green monkey kidney cell line) were transfected with Rab9A siRNA in 6-well plates as described in Example 1. Two days after Vero cells were transfected, cultures were transported to a maximum containment laboratory and infected with low-passage stocks of Ebola virus (Zaire species, 1976 Mayinga strain) and Marburg virus (1967 Voege strain). Dilutions of viral stocks were made in Dulbecco's Minimal Essential Medium containing 10% fetal bovine serum. Wells were inoculated with 0.1 ml diluted virus stock, containing 2,500 plaque forming units (pfu) of Ebola virus or either 2,500 or 25,000 pfu of Marburg virus. Cultures were incubated for 6-7 days, and tissue culture fluids were clarified by low speed centrifugation (1,500 rpm, 5 minutes). Supernatant fluids were removed and aliquoted, and stored either in liquid nitrogen or inactivated by gamma irradiation on dry ice (>3 Mrad).

Cells were harvested by scraping into 1 ml phosphate buffered saline (PBS; 0.01 M, pH 7.2), pelleted in a microfuge tube at 5,000 rpm, and spotted and dried onto 12-well glass slides. Slides were gamma-irradiated (along with supernatant fluids), then fixed with acetone for 10 min. Indirect fluorescent antibody staining of cells was performed using rabbit polyclonal antisera reactive against either Ebola virus or Marburg virus proteins as the primary antibody, and goat anti-rabbit IgG conjugated with FITC (Kirkegaard & Perry Laboratories, Inc.) as the secondary antibody. Cells were viewed for specific staining using an Olympus BX51 microscope fitted with a HQ-41001/FITC filter set (Chroma Technology Corp.) and images capture using a Q-Color5 Imaging System with Peltier cooling (Olympus).

Detection of virus antigen in supernatant fluids was performed using solid-phase antigen capture assays to detect the VP40 protein of Ebola virus or the NP and VP40 proteins of Marburg virus. Assays were performed 6 days post-infection, essentially as described previously (Ksiazek et al., J. Clin. Microbiol. 30:947, 1992).

### Measles Virus in vitro assays

Vero cells were transfected as described in Example 1 and infected 2 days post-transfection with the Edmonston (Edm) strain of Measles Virus (MV) at an MOI of 0.1. To measure MV production, the culture supernatant was removed daily and stored at -70°C. Each time, the cells were replenished with fresh medium for the next 6 days. Subsequently, the titer of released virus was determined by a plaque assay of the virus on a Vero monolayer.

Syncytia formation was detected on days 2 and 6 following MV infection. Infected cells were mounted on chamber slides and stained for MV-nucleoprotein using the primary antibody mab-KK2 (Chemicon International, Temecula, CA), and an alexa fluor-conjugated goat antimouse secondary antibody (Molecular Probes, Eugene, OR).

### RSV in vitro assays

Rab9A siRNA molecules were used to decrease infection by RSV in an *in vitro* system. Vero E6 cells (2 x 10⁵ cells/well) were cultured in 24-well plates in Dulbecco's modified Eagles Medium (DMEM) containing 10% fetal bovine sera (FBS) 24 hours prior to siRNA testing. Mirus-Transit-TKO transfection reagent was used for all transfections. Cocktails were made by mixing the appropriate duplex siRNAs (50-100 pmoles) with DMEM. Two microliters of Transit TKO was added to 50 µl of DMEM/10% FBS and incubated at room temperature for 10 minutes. The cells were washed with DMEM to remove FBS in the media, and the siRNA mixture was added to cells and rocked for 3 minutes. The cells were incubated for 6 hours at 37°C, 5% CO₂. At 6 hours post-transfection, the cells were removed from incubator and the siRNA mixture removed from cells by washing with DMEM.

RSV strain A2 was diluted to 100-200 PFU/100 µl in DMEM, and 100 µl added per well of 24-well plate and incubate for 1 hour at 37°C, 5% CO₂. At one hour post-infection, the cells were removed from the incubator, 1 ml of 2% complete methylcellulose was overlayed over the cells, and the cells incubated at 37°C, 5% CO₂ for 5 days. At day 5 post-infection, the methylcellulose overlay was removed and RSV plaques enumerated by immunostaining with anti-F protein (clone 131-2A) monoclonal antibody.

### Cloning simian Rab9A cDNA

cDNA was prepared from Vero cells as described above, and simian Rab9A cDNA was amplified using the forward primer (5'-ATTAACAATGGCAGGAA-3'; SEQ ID NO: 33) and reverse primer (5'-GTGTCCCTTCTCCCACCAACTAATGA-3'; SEQ ID NO: 34), based on an observed homology between the human and murine genes at these regions. Simian Rab9A cDNA was cloned using the Topo TA Cloning Kit (InVitrogen), and sequenced using the forward primer (5'-GTAAAACGACGGCCAG-3'; SEQ ID NO: 35) and reverse primer (5'-CAGGAAACAGCTATGAC-3'; SEQ ID NO: 36), annealing to the cloning vector.

### Results

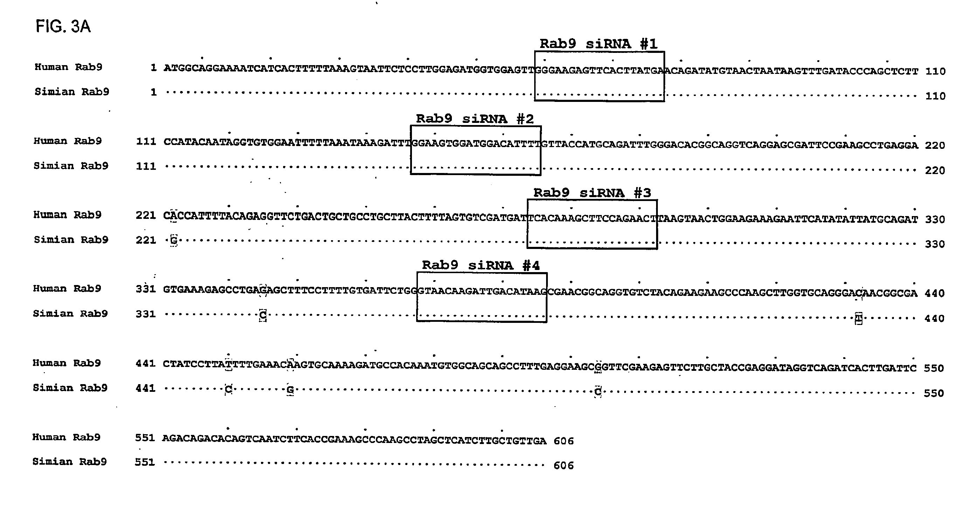

The simian Rab9A gene was cloned to aid in designing appropriate primers for quantitation of mRNA expression. Sequence analysis revealed that simian Rab9A shows an overall homology of 98% to human Rab9A mRNA (FIG. 3A) and is 100% homologous at the chosen siRNA target sites. Two days post-transfection, total mRNA was isolated and reverse transcribed as described in Example 1. Treatment of Vero cells with Rab9A siRNA effectively reduced Rab9A mRNA expression by ~90%, while GFP siRNA treated cells showed mRNA levels similar to parental cells (FIG. 3B).

A dramatic decrease in the immunofluorescence staining of virus protein was observed in EBO-infected cells (and to a lesser degree in MBG-infected cells) transfected with Rab9A siRNA, compared to control cells treated with GFP siRNA. These results were corroborated in antigen capture assays (FIG. 3C), which show a ~70-75% decrease in virus antigen released into the culture medium.

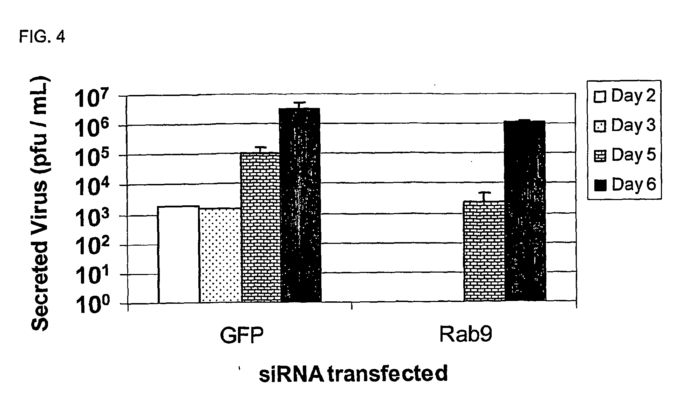

MV replication was also reduced by targeting Rab9A mRNA for degradation with siRNA. Both GFP and Rab9A siRNA transfectants were readily infectable based on FACS detection of the viral proteins nucleoprotein and hemagglutinin in ~80-90% of cells 2 days post-infection with the Edm strain of MV (MOI = 0.1). However, there was a delay in the downstream events of viral particle assembly and syncytia formation in the Rab9A siRNA transfectants compared to GFP siRNA transfectants. Syncytia formation and secretion of viral particles into the culture supernatant was undetectable 2 days following infection in Rab9A siRNA transfectants, and that MV production in Rab9A transfectants was approximately ~90% lower than in GFP siRNA transfectants 5 days after infection (FIG. 4). That the delay in viral production kinetics is overcome in Rab9A transfectants by day 6 after infection may reflect the transient nature of mRNA knock down using siRNA (FIG. 2C).

It was also observed that Rab9A siRNA decreased RSV infection by 70-95%.

Taken together, the results shown in Examples 1-3 demonstrate that targeting Rab9A mRNA, Rab11A mRNA, or mRNA of a modulator thereof, for degradation with small interfering RNA, dramatically decreases the replication of HIV-1, filoviruses (MBG and Ebola (EBO)), RSV, and Measles (MU), indicating that Rab9A, Rab11A, modulators thereof, can be used target in the development of broad-spectrum antiviral drugs.

### Example 4

### Rab9A siRNA Decreases Lipid Raft Formation

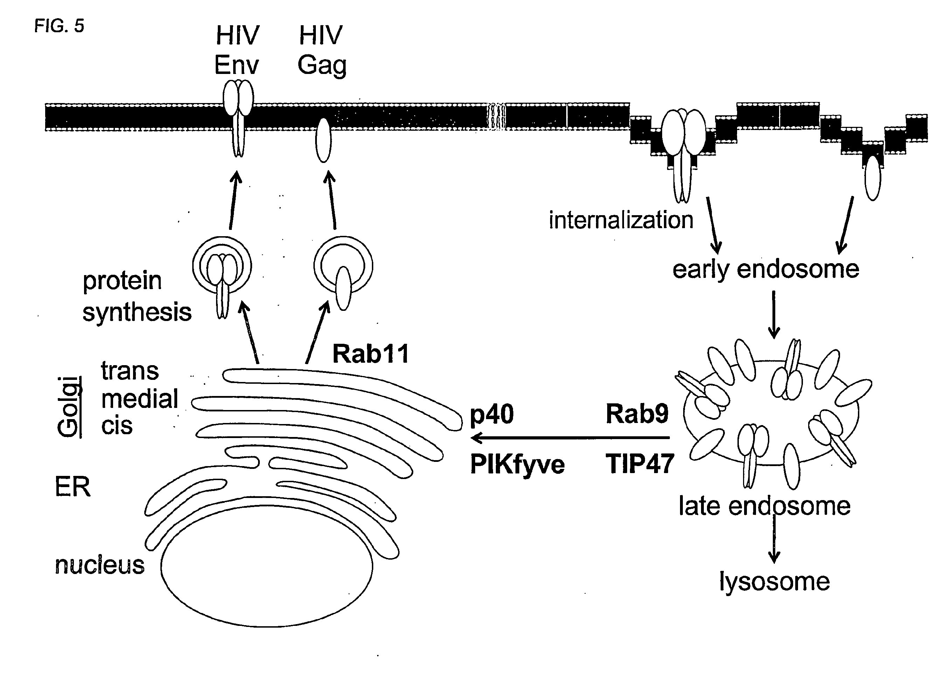

As described in the examples above, siRNA molecules that recognize Rab9A decrease viral infection, such as enveloped RNA viruses. Rab9A transports late endosomes to trans-golgi. Based on these results, Rab9A is shown to play a role in lipid raft formation (FIG. 5).

Lipid rafts are liquid-ordered microdomains enriched in sphingolipds and cholesterol, and are involved in biosynthetic traffic, signal transduction, and endocytosis. Viruses take advantage of ("hijack") rafts for completion of some steps of their replication cycle, such as entry into their cell host, assembly, and budding. Without wishing to be bound to a particular theory, it is proposed that Rab9A promotes proper subcellular localization of pathogen proteins (such as viral proteins) or lipid raft components, such as those that are involved in viral assembly and budding. For example, Rab7, Rab9A, and Rab11A regulate normal sphingolipid and cholesterol trafficking (the "glue" that holds lipid rafts together). In addition, Rab7 or Rab9A overexpression corrects sphingolipid and cholesterol trafficking defects in the Niemann-Pick C sphingolipid storage disease. Therefore, reducing or inhibiting expression of Rab7, Rab9A, and Rab9A modulators that increase Rab9A activity, can disregulate cholesterol and sphingolipid trafficking, with consequential impairments in lipid raft formation and infection (such as viral replication).

As shown in Example 1, depletion of Rab9A, which mediates late endosome to trans-Golgi transport, dramatically reduces HIV-1 replication. These results indicate that viral egress in HeLa cells arises from viral assembly at the late endosome, followed by Rab9A-dependent transit of preformed Gag-Env complexes to the trans-Golgi network en route to the plasma membrane, where budding is completed.

The VP40 matrix proteins of EBO and MBG viruses have variable partitioning between the plasma membrane and intracellular viral inclusions (Martin-Serrano et al., Nat. Med 7:1313, 2001; Kolesnikova et al., J. Virol. 78:2382, 2004). MBG VP40 associates with the late endosome, and vesicular transport is used to transfer VP40 to the cell surface (Kolesnikova et al., J. Virol. 78:2382,2004). Given that IUV-1 and MBG are believed to assemble at the late endosome and HIV-1 and EBO utilize endosomal sorting machinery for budding (Martin-Serrano et al., Nat. Med 7:1313, 2001), the results described above indicate a conserved dependence by HIV-1, EBO, and MBG upon Rab9A for transport to the cell membrane via the trans-Golgi network. Thus, reducing or inhibiting Rab9A or Rab11A activity, for example by decreasing its expression (or by decreasing expression of a modulator thereof that is required for or enhances Rab9A or Rab11A activity), can result in the redirection of viral proteins into the lysosome for their degradation.

Examples of pathogens that hijack lipid rafts include, but are not limited to those shown in Table 2. In the absence of functional Rab9A and lipid rafts (or a decrease in the number of rafts), viruses may not be able to bud or be infectious. Therefore, the use of agents that decrease activity of Rab9A or a Rab9A modulator can be used to decrease infection by other pathogens, as well as toxins such as anthrax, that hijack lipid rafts, such as those shown in Table 2.

**Table 2: Exemplary pathogens that hijack lipid rafts.**

| **Bacteria** | | | |
|---|---|---|---|
| Intracellular survival | Toxin binding/oligomerization | **Viruses** | **Protozoa** |
| *Campylobacter jujuni* | *Vibrio cholerae* | SV40 | *Toxoplasma gondii* |
| *Legionella pneumophila* | *Aeromonas hydrophilia* | Echovirus 1 and 11 | *Plasmodium falciparum* |
| *Brucella spp* | *Clostridium spp.* | Avian sarcoma and leukosis virus | |
| *FimH and Dr Escherichia coli* | *Streptcoccus pyogenes* | Semiliki forest virus | |
| *Salmonella typhimurium* | *Bacillus anthracis* | Ecotropic mouse leukaemia virus | |
| *Shigella flexneri* | *Bacillus thuringiensis* | HTLV-1 | |
| *Chlamydia spp.* | *Helicobacter pylori* | HIV-1 | |
| *Mycobacterium spp.* | *Lysteria monocytogenes* | Ebola and Marburg viruses | |
| | | Measles virus | |
| | | Herpes Simplex virus | |
| | | Influenza virus | |
| | | Epstein-Barr virus | |
| | | RSV | |

### Example 5

### Treatment of an RSV infected animal with Rab9A siRNA

This example describes methods that can be used to treat an animal having a pathogen infection or to protect the animal from protection in the future (prophylaxis). Although the example particularly describes methods using RSV (respiratory syncytia virus) and Rab9A siRNA molecules, one skilled in the art will appreciate that similar methods can be used to treat other pathogen infections with other molecules (such as Rab11A or Rab9A modulator siRNAs, or a Rab9A, Rab11A, Rab9A modulator, or Rab11A modulator antisense molecule) in other subjects, such as a human subject.

RSV is an enveloped RNA virus in which rodent models are available. RSV infection causes bronchiolitis in infants and children, which can be fatal, especially in immunocompromised patients. Mouse models for RSV include the BALB/c mouse, as well as a BALB/c mouse by pretreated with cyclophosphamide (for example see Kong et al., Virol J. 2:3, 2005). The cotton rat (*Sigmodon hispidus)* is also a host model for RSV (Harlan Sprague Dawley, Indianapolis, IN).

To inhibit (including prevent) RSV infection in a mouse, the following methods can be used. Rab9A (or a Rab9A modulator) siRNA (1-5 mg/kg) is administered to the lungs of a mouse via intranasal administration (such as 6 to 16 week old BALB/c mice; Jackson Laboratory, Bar Harbor, ME), and the mouse infected subsequently (or at the same time as siRNA administration) intranasally with 10⁵ - 10⁷ PFU/mouse (such s 5 x 10⁵ PFU) in a volume of 50 µl. To treat RSV infection in a mouse, the following methods can be used. The mouse is first infected intranasally with RSV and subsequently, Rab9 siRNA is administered to the lungs intranasally (however, systemic administration could also be used). If desired, the BALB/c mice can be administered cyclophosphamide (Sigma, St. Louis, MO) intraperitoneally (i.p.) at a single dose of 100 mg per kg five days prior to RSV infection. The A2 strain of human RSV (American Type Culture Collection, Manassas, VA) can be propagated in HEp-2, Vero E6, A549 or primary epithelial cell lines. Alternatively, the RSV strain B1 can be used. Animal subjects can be anesthetized prior to such treatments. Similar methods can be used to infect a cotton rat (such as 2 x 10⁵ PFU of RSV in 100 µl).

In a particular example, the following method is used. Female 6-8 week old BALB/c mice (Harlan Sprague) are anesthetized by intraperitoneal administration of 2,2,2-tribromoethanol (Avertin) and intranasally administered 5-100 µg siRNA in a total volume of 50 µl at time 4 hours prior to infection for prophylaxis, or at 12, 24, 48, or 72 hours post-infection for treatment. Anesthetized mice are intranasally infected with 10⁶ plaque forming units of RSV strain A2 or B 1. Prior to removal of lungs at desired time points (such as 1-7 days post-infection), mice are anesthetized with Avertin and exsanguinated by severing the right caudal artery. Blood is collected and sera isolated. Lungs are removed and collected in 1.0 ml of Dulbecco's phosphate buffered saline (D-PBS, Invitrogen) and stored at -70°C for simultaneous analysis of virus titers. Virus titers/gram lung tissue are assayed by standard immunostaining plaque assay (for example see J. Immunol. 164:5913-21, 2000).

### Example of Prophylaxis Method:

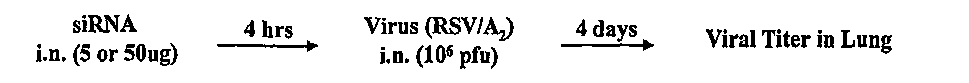

### Example of Treatment Method:

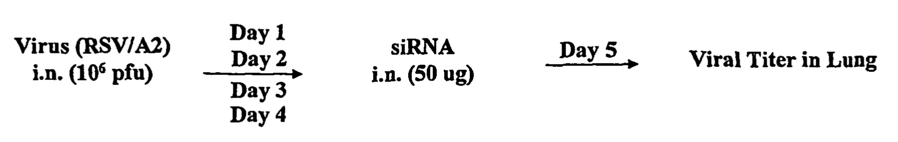

The animals are subsequently examined for viral titers and pathology of RSV infection, such as weight, lung condition, serum antibody titers, interferon levels, and so forth. Reduction of viral titer or one or more symptoms of RSV infection, as compared to a control (such as a mouse or other subject infected with RSV but did not receive the therapeutic molecules), indicates that the siRNA molecules can prevent or treat an RSV infection.

For example, animals can be monitored for weight loss 0, 5,10 and 17 days after RSV infection. Mice can be sacrificed five days after infection and their lungs removed for determination of RSV titers, cytokine levels and histopathology.

To determine RSV titers, an RSV plaque assay can be used. For example, HEp-2 cells (5 x 10⁵/well) in 6-well plates are infected with 5 x 10⁵ pfu RSV per well for 2 hours at 37°C. The RSV is removed and the wells overlaid with 1.5 ml of growth medium containing 0.8% methylcellulose. The cells are then incubated at 37°C for 72 hours, after which the overlay is removed. Following incubation, the cells are fixed in cold 80% methanol for 3 hours, blocked with 1% horse serum in PBS at 37°C for 30 min, then incubated with anti-RSV monoclonal antibody (NCL-RSV 3, Vector Laboratories, Burlingame, CA) diluted 1:400 for 1 hour at 37°C. Secondary antibody staining and substrate reactions can be performed using the Vectastain ABC Kit (Vector Laboratories) and diaminobenzidine in H₂O₂ (Pierce, Rockford, IL) used as a chromagen. The plaques can be enumerated by microscopy.

To determine airway hyperresponsiveness (AHR), the following methods can be used. AHR can be measured in unrestrained mice using a whole body plethysmograph (Buxco, Troy, NY) and expressed as enhanced pause (Penh). Mice are exposed for 5 minutes to nebulized PBS and subsequently to increasing concentrations (6, 12, 25 and 50 mg/ml) of nebulized methacholine (MCh; Sigma, St, Louis, MO) in PBS using an ultrasonic nebulizer. After nebulization, recordings are taken for 5 minutes. Penh values can be averaged and expressed as a percentage of baseline Penh values obtained following PBS exposure.

Immunohistochemical analysis of the lungs can be performed as follows. Briefly, lungs are rinsed with intratracheal injections of PBS then perfused with 10 % neutral buffered formalin. Lungs are removed, paraffin-embedded, sectioned at 20 µm and stained with hematoxylin and eosin. A semi-quantitative evaluation of inflammatory cells in the lung sections can be determined. Whole lung homogenates can be prepared using a TissueMizer and assayed for cytokines IL-10, IL-12 and IFN-γ by ELISA (R & D Systems, Minneapolis MN), following the manufacturer's directions. Results can be expressed as cytokine amount in picograms per gram of lung (pg/g).

RSV and cytokines in the lungs can be detected using RT-PCR. Briefly, total cellular RNA can be isolated from lung tissue using TRIZOL reagent (Life Technologies, Gaithersburg, MD). Forward and reverse primers that can be used are as follows: RSV-N forward: 5'-GCG ATG TCT AGG TTA GGA AGA A-3' (SEQ ID NO: 37); reverse: 5'-GCT ATG TCC TTG GGT AGT AAG CCT-3' (SEQ ID NO: 38); mouse IFN-γ Forward: 5'-GCT CTG AGA CAA TGA ACG CT-3' (SEQ ID NO: 39); reverse: 5'-AAA GAG ATA ATC TGG CTG TGC-3' (SEQ ID NO: 40); mouse IL-10 forward: 5'-GGA CTT TAA GGG TTA CTT GGG TTG CC-3'(SEQ ID NO: 41); reverse: 5'-CAT TTT GAT CAT CAT GTA TGC TTC T-3' (SEQ ID NO: 42); mouse IL-12 forward: 5'-CAG TAC ACC TGC CAC AAA GGA -3' (SEQ ID NO: 43); reverse: 5'-GTG TGA CCT TCT CTG CAG ACA -3' (SEQ ID NO: 44); and β-actin forward: 5'-GAC ATG GAG AAG ATC TGG CAC-3' (SEQ ID NO: 45); reverse: 5'-TCC AGA CGC AGG ATG GCG TGA -3' (SEQ ID NO: 46). Reactions are denatured at 95°C for 1 min, annealed at 56°C for 30 sec, and extended at 72°C for 1 min for 25-35 cycles. The PCR amplicon products can be separated by agarose gel electrophoresis and quantified.

Cell enumeration of bronchoalveolar lavage (BAL) fluid can be performed as follows. Briefly, BAL is centrifuged and the cell pellet suspended in 200 µl ofPBS and counted using a hemocytometer. The cell suspensions are then centrifuged onto glass slides, for example by using a cytospin centrifuge at 1000 rpm for 5 minutes at room temperature. Cytocentrifuged cells are air dried and stained with a modified Wright's stain (Leukostat, Fisher Scientific, Atlanta, GA) which allows differential counting of monocytes and lymphocytes.

### Example 6

### Molecules for Disruption of Gene Expression

This example describes siRNA, antisense, ribozyme, microRNA, and triple helix molecules, that can be used to reduce or disrupt expression of Rab9A, Rab11A, one or more modulators thereof that increases Rab9A or Rab11A activity, or combinations thereof, thereby decreasing the biological activity of Rab9A or Rab11A (or in some examples a modulator thereof). Such agents are useful for decreasing infection by a pathogen, treating a pathogen infection, or preventing future infection by a pathogen. For example, the agents can be used to treat a subject having a pathogen infection, or susceptible to a pathogen infection. Techniques for the production and use of such molecules are well known to those of skill in the art. For example, nucleic acid sequences can synthesized by use of an automated DNA synthesizer. Methods for using these molecules are described in Example 7.

The amount of siRNA, antisense, ribozyme, microRNA, or triple helix molecule that is effective in the treatment of a particular disease or condition (the therapeutically effective amount) depends on the nature of the disease or condition, and can be determined by standard clinical techniques. For example, it can be useful to use compositions to achieve sustained release of such nucleic acid molecules. In another example, liposomes containing the desired therapeutic molecule are targeted via antibodies to specific cells.

In particular examples, at least one of the particularly disclosed siRNA molecules (SEQ ID NOS: 5-9 and 16-32), antisense RNAs (SEQ ID NOS: 48-56), ribozyme RNAs (SEQ ID NOS: 57-66), or combinations thereof, is administered to a subject to treat or inhibit infection by a pathogen. In one example, the amount of disclosed siRNA, antisense, or ribozyme RNA administered (for example in a single dose) is 1-10 mg nucleic acid molecule/kg of subject, such as 1-5 mg/kg, or 3-7 mg/kg.

### siRNA Molecules

As shown in Examples 1-3, siRNA molecules can be used to decrease or inhibit expression of Rab9A, Rab11A, or a modulator thereof that increases Rab9A or Rab11A activity, for example to decrease the ability of a pathogen to infect a cell, such as infection by an enveloped RNA virus. Exemplary siRNA compounds are provided herein for several different genes, such as Rab9A, Rab11A, p40, TIP47, and PIKfyve. However, the disclosure is not limited to these particular siRNA molecules. Based on publicly available Rab9A, Rab11A, Rab9A modulator, and Rab11A modulator sequences, one skilled in the art can generate other siRNA molecules using known methods. For example, siRNA sequences that recognize Rab9A, Rab11A, Rab9A modulator, and Rab11A modulator sequences can be designed and prepared by commercial entities, such as Sequitur, Inc. (Natick, MA).

Although only 21-mers are shown in SEQ ID NOS: 5-8 and 16-31, this disclosure is not limited to siRNA compounds of a particular length. A siRNA molecule can be any length, such as at least 19 nucleotides, at least 20 nucleotides, at least 21 nucleotides, at least 22 nucleotides, at least 23 nucleotides, at least 24 nucleotides, at least 25 nucleotides, at least 26 nucleotides, at least 27 nucleotides, or at least 30 nucleotides.

Using the methods described herein (For example see Examples 1, 3, 5 and 7), siRNA compounds can be used to decrease infection of a cell by a pathogen, treat an existing infection, prevent future infection, or combinations thereof. For example, an siRNA compound shown in any of SEQ ID NOS: 5-9 and 16-32 is incubated with its reverse complement, allowing hybridization of the two molecules. In particular examples, two or more, such as three or more, or four or more, siRNA compounds are introduced into a cell. For example, the duplex molecule is contacted with a cell, such as a cell of a subject in whom decreased viral infection is desired, under conditions that allow the duplex to enter the cell. In particular examples, the duplex is administered ex vivo or in vitro to a cell, or administered directly to a subject. In another example, an siRNA is part of a vector, and the vector administered ex vivo or in vitro to a cell, or administered directly to a subject. In one example, the vector is the pSilencer^{™} 4.1-CMV vector (Ambion, Austin, TX).

### Antisense Methods

Antisense oligonucleotides can be designed and generated using methods known in the art. For example, a Rab9A, Rab11A, Rab9A modulator, or Rab11A modulator mRNA sequence is examined. Regions of the sequence containing multiple repeats, such as TTTTTTTT, are not as desirable because they will lack specificity. Several different regions can be chosen. Of those, antisense oligonucleotides are selected by the following characteristics: those having the best conformation in solution; those optimized for hybridization characteristics; and those having less potential to form secondary structures. Antisense molecules having a propensity to generate secondary structures are less desirable.

An antisense molecule that recognizes Rab9A, Rab11A, or a modulator thereof that increases Rab9A or Rab11A activity, includes a sequence complementary to at least a portion of a Rab9A, Rab11A, Rab9A modulator, or a Rab11A modulator RNA transcript of a gene. However, absolute complementarity is not required. An antisense sequence can be complementary to at least a portion of an RNA, meaning a sequence having sufficient complementarily to be able to hybridize with the RNA, forming a stable duplex; in the case of double-stranded antisense nucleic acids, a single strand of the duplex DNA may thus be tested, or triplex formation can be assayed. The ability to hybridize depends on the degree of complementarity and the length of the antisense nucleic acid. Generally, the longer the hybridizing nucleic acid, the more base mismatches with an RNA it may contain and still form a stable duplex (or triplex, as the case may be). One skilled in the art can ascertain a tolerable degree of mismatch by use of standard procedures to determine the melting point of the hybridized complex.

In a particular example, an antisense molecule that is specific for Rab9A, Rab11A, Rab9A modulator, or Rab11A modulator includes at least 80% sequence identity, such as at least 90% sequence identity, to at least a fragment of a Rab9A, Rab11A, Rab9A modulator, or Rab11A modulator gene sequence, such as an mRNA sequence. In one example, the relative ability of an antisense molecule to bind to its complementary nucleic acid sequence is compared by determining the Tₘ of a hybridization complex of the antisense molecule and its complementary strand. The higher the Tₘ the greater the strength of the binding of the hybridized strands.

Plasmids or vectors including antisense sequences that recognize Rab9A, Rab11A, Rab9A modulator, or Rab11A modulator can be generated using standard methods. For example, cDNA fragments or variants coding for a Rab9A, Rab11A, Rab9A modulator, or Rab11A modulator protein involved in infection are PCR amplified, for example using Pfu DNA polymerase (Stratagene). The resulting sequence is cloned in antisense orientation a vector, such as pcDNA vectors (InVitrogen, Carlsbad, CA). The nucleotide sequence and orientation of the insert can be confirmed by sequencing using a Sequenase kit (Amersham Pharmacia Biotech). Such vectors can be administered to a cell in a therapeutic amount, such as administered to a subject, to decrease pathogen infection.

Generally, the term "antisense" refers to a nucleic acid molecule capable of hybridizing to a portion of a Rab9A, Rab11A, Rab9A modulator, or Rab11A modulator RNA sequence (such as mRNA) by virtue of some sequence complementarity. The antisense nucleic acids disclosed herein can be oligonucleotides that are double-stranded or single-stranded, RNA or DNA or a modification or derivative thereof, which can be directly administered to a cell (for example by administering the antisense molecule to the subject), or which can be produced intracellularly by transcription of exogenous, introduced sequences (for example by administering to the subject a vector that includes the antisense molecule under control of a promoter).

Antisense nucleic acids are polynucleotides, for example nucleic acid molecules that are at least 6 nucleotides in length, at least 10 nucleotides, at least 15 nucleotides, at least 20 nucleotides, at least 40 nucleotides, at least 100 nucleotides, at least 200 nucleotides, such as 6 to 100 nucleotides. However, antisense molecules can be much longer. In particular examples, the nucleotide is modified at one or more base moiety, sugar moiety, or phosphate backbone (or combinations thereof), and can include other appending groups such as peptides, or agents facilitating transport across the cell membrane (Letsinger et al., Proc. Natl. Acad Sci. USA 1989, 86:6553-6; Lemaitre et al., Proc. Natl. Acad. Sci. USA 1987, 84:648-52; WO 88/09810) or blood-brain barrier (WO 89/10134), hybridization triggered cleavage agents (Krol et al., BioTechniques 1988, 6:958-76) or intercalating agents (Zon, Pharm. Res. 5:539-49, 1988).

Examples of modified base moieties include, but are not limited to: 5-fluorouracil, 5-bromouracil, 5-chlorouracil, 5-iodouracil, hypoxanthine, xanthine, acetylcytosine, 5-(carboxyhydroxylmethyl) uracil, 5-carboxymethylaminomethyl-2-thiouridine, 5-carboxymethylaminomethyluracil, dihydrouracil, beta-D-galactosylqueosine, inosine, N∼6-sopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, methoxyarninomethyl-2-thiouracil, beta-D-mannosylqueosine, 5'-methoxycarboxymethyluracil, 5-methoxyuracil, 2-methylthio-N6-isopentenyladenine, uracil-5-oxyacetic acid, pseudouracil, queosine, 2-thiocytosine, 5-methyl-2-thiouracil, 2-thiouracil, 4-thiouracil, 5-methyluracil, uracil-5-oxyacetic acid methylester, uracil-S-oxyacetic acid, 5-methyl-2-thiouracil, 3-(3-amino-3-N-2-carboxypropyl) uracil, and 2,6-diaminopurine.

Examples of modified sugar moieties include, but are not limited to: arabinose, 2-fluoroarabinose, xylose, and hexose, or a modified component of the phosphate backbone, such as phosphorothioate, a phosphorodithioate, a phosphoramidothioate, a phosphoramidate, a phosphordiamidate, a methylphosphonate, an alkyl phosphotriester, or a formacetal or analog thereof.

In a particular example, an antisense molecule is an α-anomeric oligonucleotide. An α-anomeric oligonucleotide forms specific double-stranded hybrids with complementary RNA in which, contrary to the usual β-units, the strands run parallel to each other (Gautier et al., Nucl. Acids Res. 15:6625-41, 1987). The oligonucleotide can be conjugated to another molecule, such as a peptide, hybridization triggered cross-linking agent, transport agent, or hybridization-triggered cleavage agent. Oligonucleotides can include a targeting moiety that enhances uptake of the molecule by host cells. The targeting moiety can be a specific binding molecule, such as an antibody or fragment thereof that recognizes a molecule present on the surface of the host cell.

In a specific example, antisense molecules that recognize a Rab9A or Rab11A nucleic acid molecule (or a modulator sequence thereof that increases Rab9A or Rab11A activity), include a catalytic RNA or a ribozyme (for example see WO 90/11364; WO 95/06764; and Sarver et al., Science 247:1222-5, 1990). Conjugates of antisense with a metal complex, such as terpyridylCu (II), capable of mediating mRNA hydrolysis, are described in Bashkin et al. (Appl. Biochem Biotechnol. 54:43-56, 1995). In one example, the antisense nucleotide is a 2'-0-methylribonucleotide (Inoue et al., Nucl. Acids Res. 15:6131-48, 1987), or a chimeric RNA-DNA analogue (Inoue et al., FEBS Lett. 215:327-30, 1987).

Although the following exemplary antisense molecules are provided, the disclosure is not limited to these particular molecules.

Exemplary Rab9A antisense RNAs include, but are not limited to 5'-GCAUGGUAACAAAAUGUCCAUCCACUUCCAAAUCUUUAUU- 3' (SEQ ID NO: 47) that recognizes human Rab9A mRNA nucleotide positions 136-175 and 5'-GUUACUUAAGUUCUGGAAGCUUUGUGAAUCAUCGACACUA-3' (SEQ ID NO: 48) that recognizes human Rab9A mRNA nucleotide positions 261-300.

Exemplary Rab11A antisense RNAs include, but are not limited to 5'-GGAUGCUUCUUGUUGCAAACUCUACUCCAAUGGUGCUCUU- 3' (SEQ ID NO: 49) that recognizes human Rab11A mRNA nucleotide positions 121-160 and 5'-GCUGAUGUUAUAGCUCGAUAUCGCUCUUGCCCUGCUGUGU-3' (SEQ ID NO: 50) that recognizes human Rab11A mRNA nucleotide positions 197-236.

Exemplary PIKfyve antisense RNAs include, but are not limited to 5'-GGCUGAUCUAUUUCGAGCAGGAGAUUUCCCAGCAUCCUCU- 3' (SEQ ID NO: 51) that recognizes human PIKfyve mRNA nucleotide positions 879-918 and 5'-AGCAAAAGCAAUGAUGGAGCUGGGUUCCUUCUCGCAGACU-3' (SEQ ID NO: 52) that recognizes human PIKfyve mRNA nucleotide positions 4983-5022.

Exemplary p40 antisense RNAs include, but are not limited to 5'-AUUCAGGACUUGUAGACAAUUUCGAUUUCCUGAUUGGUUG- 3' (SEQ ID NO: 53) that recognizes human p40 mRNA nucleotide positions 321-360 and 5'-UCCGCCCCCAAAGACAUAUAGCUGGUUUCCAAUGGCUGCC-3' (SEQ ID NO: 54) that recognizes human p40 mRNA nucleotide positions 434-473.

Exemplary TIP47 antisense RNAs include, but are not limited to 5'-CCGACACCUUAGACGACACAAGCUCCUUGGUGUCCGCCAG-3' (SEQ ID NO: 55) that recognizes human TIP47 mRNA nucleotide positions 352-391 and 5'-GGAGCCCAGACGUACGAAGUAGCUCUGUUCCUGCCGCUGC-3' (SEQ ID NO: 56) that recognizes human TIP47 mRNA nucleotide positions 684-723.

### Ribozymes

Ribozymes are enzymatic RNA molecules capable of catalyzing the specific cleavage of RNA. The mechanism ofribozyme action involves sequence specific hybridization of the ribozyme molecule to complementary target RNA, followed by a endonucleolytic cleavage. Methods of using ribozymes to decrease or inhibit RNA expression are known in the art (for example see Kashani-Sabet, J. Investig. Dermatol. Symp. Proc., 7:76-78, 2002).

Ribozyme molecules include one or more sequences complementary to a Rab9A, Rab11A, Rab9A modulator, or Rab11A modulator mRNA and include the well-known catalytic sequence responsible for mRNA cleavage (see U.S. Pat. No. 5,093,246, herein incorporated by reference).

Methods of designing and generating ribozyme molecules are known in the art. Briefly, specific ribozyme cleavage sites within a Rab9A, Rab11A, Rab9A modulator, or Rab11A modulator RNA target are identified by scanning the RNA sequence for ribozyme cleavage sites that include: GUA, GUU and GUC. Once identified, RNA sequences of between 15 and 50 ribonucleotides (such as at least 20 ribonucleotides, at least 40 ribonucleotides, or at least 46 ribonucleotides) corresponding to the region of the target gene containing the cleavage site can be evaluated for predicted structural features, such as secondary structure, that may render the oligonucleotide sequence unsuitable. The suitability of candidate targets cam also be evaluated by testing their accessibility to hybridization with complementary oligonucleotides, using ribonuclease protection assays.

In particular examples, ribozymes are administered directly to a subject. In another example, a ribozyme is encoded on an expression vector, from which the ribozyme is synthesized in a cell (as in WO 9523225, and Beigelman et al. Nucl. Acids Res. 1995, 23:4434-42). Such a cell or the vector can be administered to a subject.

In a specific example, a vector that contains a riboyzme gene directed against Rab9A, Rab11A, or a modulator thereof, placed behind a promoter (such as an inducible promoter), is transfected into the cells of a subject, for example a subject susceptible to infection by a pathogen that utilizes lipid rafts (such as HIV, Ebola, Marburg, RSV, or measles). Expression of this vector in a cell will decrease or inhibit Rab9A, Rab11A, Rab9A modulator, or Rab11A modulator RNA expression in the cell. In one example, the vector is the pSilencer^{TM} 4.1-CMV vector (Ambion).

In a particular example, a vector includes self-cleaving tandem ribozymes (for example, 5 ribozymes encoded on a single RNA transcript). Once the tandem hammerhead transcript is synthesized, it recognizes ribozyme cleavage sites in cis within the newly synthesized transcript identical to the ribozyme target site on the cell's endogenously expressed mRNA. The tandem ribozyme then cleaves itself, liberating free ribozymes to cleave the target mRNA in the cell.

Although the following exemplary hammerhead ribozyme molecules are provided, the disclosure is not limited to these particular molecules.

Exemplary Rab9A ribozyme RNAs include, but are not limited to 5'-GGAAUCGCACCUCUGAUGAGUCCGUGAGGACGAAACCUGCCGUGUC-3' (SEQ ID NO: 57) that recognizes human Rab9A mRNA nucleotide positions 183-207 and 5'-UUGUGAAUCAUCCUGAUGAGUCCGUGAGGACGAAACACUAAAAGUA -3' (SEQ ID NO: 58) that recognizes human Rab9A mRNA nucleotide positions 255-279.

Exemplary Rab11A ribozyme RNAs include, but are not limited to 5'-CGAGUAAAUCGACUGAUGAGUCCGUGAGGACGAAACAGGAGAUUAC - 3' (SEQ ID NO: 59) that recognizes human Rab11A mRNA nucleotide positions 74-98 and 5'-UUGUGCUGGACUGAUGAGUCCGUGAGGACGAAACAUGUCAUUUU -3' (SEQ ID NO: 60) that recognizes human Rab11A mRNA nucleotide positions 557-581.

Exemplary PIKfyve ribozyme RNAs include, but are not limited to 5'-GAUCUAUCCAGUCUGAUGAGUCCGUGAGGACGAAACAGGUUAGUAA - 3' (SEQ ID NO: 61) that recognizes human PIKfyve mRNA nucleotide positions 923-947 and 5'-UUCCUUCUCGCACUGAUGAGUCCGUGAGGACGAAACUGCAAUGGGC -3' (SEQ ID NO: 62) that recognizes human PIKfyve mRNA nucleotide positions 4974-4998.

Exemplary p40 ribozyme RNAs include, but are not limited to 5'-UCAGGACUUGUACUGAUGAGUCCGUGAGGACGAAACAAUUUCGAUU - 3' (SEQ ID NO: 63) that recognizes human p40 mRNA nucleotide positions 333-357 and 5'-UCCGCCCCCAAACUGAUGAGUCCGUGAGGACGAAACAUAUAGCUGG-3' (SEQ ID NO: 64) that recognizes human p40 mRNA nucleotide positions 450-474.

Exemplary TIP47 ribozyme RNAs include, but are not limited to 5'-GACACCUUAGACCUGAUGAGUCCGUGAGGACGAAACACAAGCUCCU-3' (SEQ ID NO: 65) that recognizes human TIP47 mRNA nucleotide positions 365-389 and 5'-ACAGGGAGCCCACUGAUGAGUCCGUGAGGACGAAACGUACGAAGUA -3' (SEQ ID NO: 66) that recognizes human TIP47 mRNA nucleotide positions 703-727.

### microRNAs

MicroRNAs (miRs) that recognize a Rab9A mRNA, Rab11A mRNA, a Rab9A modulator mRNA, or a Rab11A modulator mRNA, can be used to decrease the amount of such mRNAs in a cell. miRNAs silence at the post-transcriptional level by virtue of their sequence complementarity to target mRNAs. In particular examples, miRs are about 18-26 nucleotides in length, such as 21-26 nucleotides, such as at least 18 nucleotides. Animal miRNAs are generally thought to recognize their mRNA targets by incomplete base-pairing, leading to translational inhibition of the target.

Methods of generating or identifying microRNAs are known in the art (for example see Lagos-Quintana et al., Science, 294:853-8, 2001; Lagos-Quintana et al., Curr. Biol. 12:735-9, 2002; and Lagos-Quintana et al., RNA 9:175-9, 2003).

### Triple helix molecules

Nucleic acid molecules used in triplex helix formation for Rab9A, Rab11A, or a modulator thereof, are ideally single stranded and composed of deoxynucleotides. The base composition of these oligonucleotides is designed to promote triple helix formation via Hoogsteen base pairing rules, which generally require sizeable stretches of either purines or pyrimidines to be present on one strand of a duplex. Nucleotide sequences can be pyrimidine-based, which will result in TAT and CGC+ triplets across the three associated strands of the resulting triple helix. The pyrimidine-rich molecules provide base complementarity to a purine-rich region of a single strand of the duplex in a parallel orientation to that strand. In addition, nucleic acid molecules can be chosen that are purine-rich, for example contain a stretch of guanidine residues. These molecules will form a triple helix with a DNA duplex that is rich in GC pairs, in which the majority of the purine residues are located on a single strand of the targeted duplex, resulting in GGC triplets across the three strands in the triplex.

Alternatively, Rab9A, Rab11A, Rab9A modulator, and Rab11A modulator sequences targeted for triple helix formation are increased by creating "switchback" nucleic acid molecule. Switchback molecules are synthesized in an alternating 5'-3', 3'-5' manner, such that they base pair with one strand of a duplex first and then the other, eliminating the necessity for a sizeable stretch of either purines or pyrimidines to be present on one strand of a duplex.

### Example 7

### Methods of Treatment and Prophylaxis

This example provides methods that can be used to treat a subject having a pathogen infection, or to prevent or reduce the incidence of a future infection. Methods of treatment include methods that reduce one or more symptoms in the subject due to the infection, such as fever or increased white blood cell count. However, a complete elimination of symptoms is not required. Treatment methods can also include reducing the presence of the pathogen, such as reducing viral titer in a subject. Prophylactic methods include reducing the incidence of a future pathogen infection, for example in a subject who is susceptible to infection by the pathogen (such as children, the elderly, and medical workers).

In particular examples, the method includes administering to the subject a therapeutically effective amount of an agent that decreases the biological activity of Rab9A or Rab 11A (for example by decreasing the activity of a modulator thereof that increases Rab9A or Rab11A activity, respectively). When the activity of Rab9A, Rab11A, or a modulator thereof that increases Rab9A or Rab11A activity, respectively, is decreased, for example by prematurely downregulating their protein or nucleic acid molecule levels, a reduction in pathogen infection is achieved. For example, antisense oligonucleotides, ribozymes, miRs, siRNA, and triple helix molecules that recognize a nucleic acid that encodes Rab9A, Rab11A, or a modulator thereof that increases Rab9A or Rab11A activity, respectively, (for example those disclosed in the Examples above) can therefore be used to disrupt cellular expression of Rab9A, Rab11A, a Rab9A modulator, or a Rab11A modulator protein, respectively. The disclosed antisense, ribozyme, triple helix, miRs, and siRNA molecules (for example at a concentration of 1-10 mg nucleic acid molecule/kg of subject, such as 1-5 mg/kg, or 3-7 mg/kg) can be administered to a subject alone, or in combination with other agents, such as a pharmaceutical carrier, other therapeutic agents (such as anti-viral compounds), or combinations thereof. In on example, the subject is a mammal, such as mice, non-human primates, and humans.

In one example, an siRNA, ribozyme, triple helix, miR, or antisense molecule is part of a vector, and the vector administered ex vivo or in vitro to a cell, or administered directly to a subject. For example, a U6 promotor that controls the expression of 21 nucleotides, followed by a stem-loop of 8 bp, followed by an additional complementary 21 bp that anneals to the first 21 nucleotides transcribed. The 21 nucleotide sequences are the siRNA that recognize Rab9A, Rab11A, or a modulator thereof. Transcription is halted using a stretch of 5 T's in the plasmid immediately downstream of the last desired transcribed nucleotide. In another example, the vector is the pSilencer^{™} 4.1-CMV vector (Ambion)

In particular examples, a subject susceptible to or suffering from an infection, wherein decreased amounts of infection by the pathogen is desired, is treated with a therapeutically effective amount of antisense, ribozyme, triple helix, miR, or siRNA molecule (or combinations thereof) that recognizes a Rab9A or Rab11A nucleic acid sequence (or the nucleic acid sequence of a modulator thereof that increases Rab9A or Rab11A activity, respectively). Similarly, other agents, such as an agent that specifically recognizes and interacts with (such as binds to) Rab9A, Rab11A, a Rab9A modulator, or a Rab11A modulator protein, thereby decreasing the ability of the protein to interact with a pathogen, can also be used to decrease or inhibit infection. Other exemplary agents are those identified using the methods described in the Examples below. These agents, such as antibodies, peptides, nucleic acid molecules, organic or inorganic compounds, can be administered to a subject in a therapeutically effective amount. After the agent has produced an effect (a decreased level of pathogen infection is observed, or symptoms associated with infection decrease), for example after 24-48 hours, the subject can be monitored for diseases associated with the infection.

In particular examples, the subject is first screened to determine the type of pathogen infection present. If the pathogen is one that can be decreased by the disclosed therapies, such as a pathogen that hijacks a lipid raft, the subject is then administered the therapy.

The treatments disclosed herein can also be used prophylactically, for example to inhibit or prevent infection by a pathogen. Such administration is indicated where the treatment is shown to have utility for treatment or prevention of the disorder. The prophylactic use is indicated in conditions known or suspected of progressing to disorders associated with a pathogen infection.

### Example 8

### Pharmaceutical Compositions and Modes of Administration

Methods for administering the therapies disclosed herein are known. Particular examples include encapsulation in liposomes, microparticles, microcapsules, expression by recombinant cells, receptor-mediated endocytosis (Wu and Wu, J. Biol. Chem. 1987, 262:4429-32), and construction of therapeutic nucleic acid molecules as part of a retroviral or other vector. Methods of introduction include, but are not limited to, topical, intradermal, intramuscular, intraperitoneal, intravenous, subcutaneous, intranasal, and oral routes. The disclosed therapeutic agents can be administered by any convenient route, for example by infusion or bolus injection, by absorption through epithelial or mucocutaneous linings (for example, oral mucosa, rectal, vaginal and intestinal mucosa) and can be administered together with other biologically active agents. Administration can be systemic or local. Pharmaceutical compositions can be delivered locally to the area in need of treatment, for example by topical application.

Pharmaceutical compositions are disclosed that include a therapeutically effective amount of an agent that decreases the expression or activity of Rab9A or Rab 11A (for example by decreasing the activity of a modulator thereof that increases Rab9A or Rab11A activity, or by increasing the activity of a modulator thereof that decreases Rab9A or Rab11A activity). Particular examples include antisense, ribozyme, triple helix, miR, and siRNA molecules, as well as specific-binding agents such as antibodies. Such agents can be administered alone, or with a pharmaceutically acceptable carrier. Furthermore, the pharmaceutical compositions or methods of treatment can be administered in combination with (such as before, during, or following) other therapeutic treatments, such as other antiviral or anti-bacterial agents.

In an example in which a nucleic acid molecule is employed to reduce infection by a pathogen, such as an antisense, ribozyme, triple helix, miR, or siRNA molecule, any method known in the art can be used. Particular examples include delivering the nucleic acid molecule intracellularly (for example by receptor-mediated mechanisms), by an expression vector administered so that it becomes intracellular (for example by use of a retroviral vector, see U.S. Patent No. 4,980,286), by injection of the nucleic acid molecule to a cell, by use of microparticle bombardment (such as a gene gun; Biolistic, Dupont), coating the nucleic acid molecule with lipids or cell-surface receptors or transfecting agents, or by administering it in linkage to a homeobox-like peptide known to enter the nucleus (for example Joliot et al., Proc. Natl. Acad. Sci. USA 1991, 88:1864-8). The present disclosure includes all forms of nucleic acid molecule delivery, including synthetic oligos, naked DNA, plasmid and viral, integrated into the genome or not.

The pharmaceutically acceptable carriers useful herein are conventional. Remington's Pharmaceutical Sciences, by Martin, Mack Publishing Co., Easton, PA, 15th Edition (1975), describes compositions and formulations suitable for pharmaceutical delivery of the therapeutic agents herein disclosed. In general, the nature of the carrier will depend on the mode of administration employed. For instance, parenteral formulations usually include injectable fluids that include pharmaceutically and physiologically acceptable fluids such as water, physiological saline, balanced salt solutions, aqueous dextrose, sesame oil, glycerol, ethanol, combinations thereof, or the like, as a vehicle. The carrier and composition can be sterile, and the formulation suits the mode of administration. In addition to biologically-neutral carriers, pharmaceutical compositions to be administered can contain minor amounts of non-toxic auxiliary substances, such as wetting or emulsifying agents, preservatives, and pH buffering agents and the like, for example sodium acetate or sorbitan monolaurate.

The composition can be a liquid solution, suspension, emulsion, tablet, pill, capsule, sustained release formulation, or powder. For solid compositions (for example powder, pill, tablet, or capsule forms), conventional non-toxic solid carriers can include, for example, pharmaceutical grades of mannitol, lactose, starch, sodium saccharine, cellulose, magnesium carbonate, or magnesium stearate. The composition can be formulated as a suppository, with traditional binders and carriers such as triglycerides.

The amount of therapeutic agent effective in decreasing or inhibiting infection by a pathogen can depend on the nature of the pathogen and its associated disorder or condition, and can be determined by standard clinical techniques. In addition, *in vitro* and *in vivo* assays can be employed to identify optimal dosage ranges. For example, effective doses can be extrapolated from dose-response curves derived from *in vitro* or animal model test systems. The precise dose to be employed in the formulation will also depend on the route of administration, and the seriousness of the disease or disorder, and should be decided according to the judgment of the practitioner and each subject's circumstances.

The disclosure also provides a pharmaceutical pack or kit comprising one or more containers filled with one or more of the ingredients of the pharmaceutical compositions. In one example, the kit includes an agent that can decrease the biological activity of Rab9A, Rab 11A, or a modulator thereof, and another therapeutic agent, such as an anti-viral composition or an anti-bacterial composition.

### Example 9

### Recombinant Expression of Nucleic Acid Molecules

Agents that decrease expression of Rab9A or Rab11A (for example by decreasing expression of a modulator thereof that increases Rab9A or Rab11A activity, or by increasing expression of a modulator thereof that decreases Rab9A or Rab11A activity) can be produced recombinantly using standard molecular biology methods. Recombinant expression can be used to express the agent in a cell in which decreased pathogen infection is desired, or to produce the therapeutic agent (which can then be purified prior to use).

For example, a therapeutic nucleic acid, such as an antisense, ribozyme, triple helix molecule, miR, or siRNA that specifically recognizes a Rab9A, Rab11A, a Rab9A modulator, or a Rab11A modulator nucleic acid, can be ligated into an expression vector. Exemplary vectors include, but are not limited to, pKC30 (Shimatake and Rosenberg, 1981, Nature 292:128), pKK177-3 (Amann and Brosius, 1985, Gene 40:183) and pET-3 (Studiar and Moffatt, 1986, J. Mol. Biol. 189:113). In addition, a nucleic acid sequence can be ligated into other vehicles, such as a bacteriophage, cosmid, animal virus, or yeast artificial chromosome (YAC) (Burke et al., 1987, Science 236:806-12).

Expression of the antisense or siRNA molecule can be controlled by placing a promoter upstream of the antisense or siRNA sequence. The promoter can be constitutive or inducible. Exemplary promoters include, but are not limited to *lac, trp, tac, trc,* major operator and promoter regions of phage lambda, the control region of fd coat protein, the early and late promoters of SV40, promoters derived from polyoma, adenovirus, retrovirus, baculovirus and simian virus, the promoter in the pSV2 vector (Mulligan and Berg, 1981, Proc. Natl. Acad Sci. USA 78:2072-6), the promoter for 3-phosphoglycerate kinase, the promoters of yeast acid phosphatase, the promoter of the yeast alpha-mating factors and combinations thereof.

The transfer of nucleic acid molecules into eukaryotic, such as human or other mammalian cells is a conventional technique. In one example, a nucleic acid molecule (such as an antisense, ribozyme, triple helix, miR, or siRNA molecule) is introduced into a cell, such as the cell of a subject as pure DNA, for example by precipitation with calcium phosphate (Graham and vander Eb, 1973, Virology 52:466), strontium phosphate (Brash et al., 1987, Mol. Cell Biol. 7:2013), electroporation (Neumann et al., 1982, EMBO J. 1:841), lipofection (Felgner et al., 1987, Proc. Natl. Acad. Sci USA 84:7413), DEAE dextran (McCuthan et al., 1968, J. Natl. Cancer Inst. 41:351), microinjection (Mueller et al., 1978, Cell 15:579*),* protoplast fusion (Schafner, 1980, Proc. Natl. Acad Sci. USA 77:2163-7), or pellet guns (Klein et al., 1987, Nature 327:70). In another example, a nucleic acid molecule is introduced into a cell (such as the cell of a subject) by infection with virus vectors, for example retroviruses (Bernstein et al., 1985, Gen. Engrg. 7:235), adenoviruses (Ahmad et al., J. Virol. 57:267, 1986), adenoassociated viral vectors such as AAV 1, AAV2, AAV3, AAV4, AAV5 or AAV6 (Goodman et al. Blood 84:1492, 1994), lentiviral vectors (Naldini et al., Science 272:263, 1996), pseudotyped retroviral vectors (Agrawal et al., Exp. Hematol. 24:738, 1996), vaccinia vectors (Guo and Bartlett, Expert Opin Biol Ther. 4(6):901, 2004) or Herpes (Spaete et al., Cell 30:295, 1982).

### Example 10

### in vitro Screening Assay for Agents that Decrease Infection

This example describes *in vitro* methods that can be used to screen test agents for their ability to interfere with or even inhibit infection of a host cell by a pathogen. As disclosed in the Examples above, Rab9A, Rab11A, and modulators thereof, are involved in pathogen infection (such as infection by a pathogen that uses a lipid raft, for example HIV, Ebola, Marburg, RSV, and measles) and the Rab9A, Rab11A, Rab9A modulator, or Rab11A modulator protein/pathogen protein interaction is a component in the ability of a pathogen to infect a cell. Therefore, screening assays can be used to identify and analyze agents that decrease or interfere with this interaction. For example, the following assays can be used to identify agents that interfere with the interaction of Rab9A, Rab11A, or a modulator thereof, with a pathogen protein sequence. However, the present disclosure is not limited to the particular methods disclosed herein.

Agents identified via the disclosed assays can be useful, for example, in decreasing or even inhibiting pathogen infection by more than an amount of infection in the absence of the agent, such as a decrease of at least about 10%, at least about 20%, at least about 50%, or even at least about 90%. This decrease in infection can serve to ameliorate symptoms associated with infection, such as fever. Assays for testing the effectiveness of the identified agents, are discussed below.

Exemplary test agents include, but are not limited to, any peptide or non-peptide composition in a purified or non-purified form, such as peptides made of D-and/or L-configuration amino acids (in, for example, the form of random peptide libraries; see Lam et al., Nature 354:82-4, 1991), phosphopeptides (such as in the form of random or partially degenerate, directed phosphopeptide libraries; see, for example, Songyang et al., Cell 72:767-78, 1993), antibodies, and small or large organic or inorganic molecules. A test agent can also include a complex mixture or "cocktail" of molecules.

The basic principle of the assay systems used to identify agents that interfere with the interaction between a Rab9A, Rab11A, Rab9A modulator, or Rab11A modulator protein, and a pathogen protein binding partner or partners, involves preparing a reaction mixture containing the Rab9A, Rab11A, Rab9A modulator, or Rab11A modulator protein and one or more pathogen proteins under conditions and for a time sufficient to allow the proteins to interact and bind, thus forming a complex. To test an agent for inhibitory activity, the reaction is conducted in the presence and absence of the test agent. The test agent can be initially included in the reaction mixture, or added at a time subsequent to the addition of Rab9A, Rab11A, Rab9A modulator, or Rab 11A modulator protein and a pathogen protein. Controls can be are incubated without the test agent or with a placebo. Exemplary controls include agents known not to bind to pathogen proteins, Rab9A protein, Rab11A protein, a Rab9A modulator protein, or a Rab11A modulator protein. The formation of any complexes between the Rab9A, Rab11A, Rab9A modulator, or Rab11A modulator protein and the pathogen protein is then detected. The formation of a complex in the control reaction, but not in the reaction mixture containing the test agent, indicates that the agent interferes with the interaction of the Rab9A, Rab11A, Rab9A modulator, or Rab11A modulator and the pathogen protein, and is therefore possibly an agent that can be used to decrease infection by a pathogen, for example to treat a subject having an infection or to prevent an infection in the future.

The assay for agents that interfere with the interaction of a Rab9A, Rab11A, Rab9A modulator, or Rab11A modulator protein and pathogen proteins can be conducted in a heterogeneous or homogeneous format. Heterogeneous assays involve anchoring the Rab9A, Rab11A, Rab9A modulator, or Rab11A modulator protein or the pathogen protein onto a solid phase and detecting complexes anchored on the solid phase at the end of the reaction. In some examples, the method further involves quantitating the amount of complex formation or inhibition. Exemplary methods that can be used to detect the presence of complexes, when one of the proteins is labeled, include ELISA, spectrophotometry, flow cytometry, and microscopy. In homogeneous assays, the entire reaction is performed in a liquid phase. In either method, the order of addition of reactants can be varied to obtain different information about the agents being tested. For example, test agents that interfere with the interaction between the proteins, such as by competition, can be identified by conducting the reaction in the presence of the test agent, for example by adding the test agent to the reaction mixture prior to or simultaneously with the Rab9A, Rab11A, Rab9A modulator, or Rab11A modulator protein and pathogen protein. On the other hand, test agents that disrupt preformed complexes, such as agents with higher binding constants that displace one of the proteins from the complex, can be tested by adding the test agent to the reaction mixture after complexes have been formed. The various formats are described briefly below.

Once identified, test agents found to inhibit or decrease the interaction between a Rab9A, Rab11A, Rab9A modulator, or Rab11A modulator protein and a pathogen protein can be formulated in therapeutic products (or prophylactic products) in pharmaceutically acceptable formulations, and used for specific treatment or prevention of a disease associated with a pathogen, such as HIV, Ebola, Marburg, RSV, or measles.

### Heterogeneous assay system

In a heterogeneous assay system, one binding partner, either the Rab9A, Rab 11A, Rab9A modulator, or Rab11A modulator protein, or the pathogen protein (such as an HIV, Ebola, Marburg, RSV, or measles virus preparation) is anchored onto a solid surface (such as a microtiter plate), and its binding partner, which is not anchored, is labeled, either directly or indirectly. Exemplary labels include, but are not limited to, enzymes, fluorophores, ligands, and radioactive isotopes. The anchored protein can be immobilized by non-covalent or covalent attachments. Non-covalent attachment can be accomplished simply by coating the solid surface with a solution of the protein and drying. Alternatively, an immobilized antibody (such as a monoclonal antibody) specific for the protein can be used to anchor the protein to the solid surface. The surfaces can be prepared in advance and stored.

To conduct the assay, the binding partner of the immobilized species is added to the coated surface with or without the test agent. After the reaction is complete, unreacted components are removed (such as by washing) and any complexes formed will remain immobilized on the solid surface. The detection of complexes anchored on the solid surface can be accomplished in a number of ways. Where the binding partner was pre-labeled, the detection of label immobilized on the surface indicates that complexes were formed. Where the binding partner is not pre-labeled, an indirect label can be used to detect complexes anchored on the surface; for example by using a labeled antibody specific for the binding partner (the antibody, in turn, can be directly labeled or indirectly labeled with a labeled anti-Ig antibody). Depending upon the order of addition of reaction components, test compounds which inhibit complex formation or which disrupt preformed complexes can be detected.

Alternatively, the reaction can be conducted in a liquid phase in the presence or absence of the test agent, the reaction products separated from unreacted components, and complexes detected; for example by using an immobilized antibody specific for one binding partner to anchor any complexes formed in solution, and a labeled antibody specific for the other binding partner to detect anchored complexes. Again, depending upon the order of addition of reactants to the liquid phase, test agents which inhibit complex or which disrupt preformed complexes can be identified.

### Homogenous assays

In an alternate example, a homogeneous assay can be used. In this method, a preformed complex of the Rab9A, Rab11A, Rab9A modulator, or Rab11A modulator protein and the pathogen protein is prepared in which one of the proteins is labeled, but the signal generated by the label is quenched due to complex formation (for example, see U.S. Pat. No. 4,109,496 by Rubenstein which utilizes this approach for immunoassays). The addition of a test substance that competes with and displaces one of the binding partners from the preformed complex will result in the generation of a signal above background. In this way, test agents that disrupt Rab9A, Rab11A, Rab9A modulator, or Rab11A modulator protein-pathogen protein interactions are identified.

### Immobilization of Proteins

In a particular example, a Rab9A, Rab11A, Rab9A modulator, or Rab11A modulator protein can be prepared for immobilization using recombinant DNA techniques. For example, a coding region of Rab9A, Rab11A, a Rab9A modulator, or a Rab11A modulator protein can be fused to a glutathione-S-transferase (GST) gene using the fusion vector pGEX-5X-1, in such a manner that its binding activity is maintained in the resulting fusion protein.

In a heterogeneous assay, for example, the GST-Rab9A, GST-Rab11A, GST-Rab9A modulator, or GST-Rab11A modulator fusion protein can be anchored to glutathione-agarose beads. The pathogen protein preparation can then be added in the presence or absence of the test agent in a manner that allows interaction and binding to occur. At the end of the reaction period, unbound material can be washed away, and a labeled antibody (such as labeled with the radioactive isotope ¹²⁵I) that is specific for Rab9A, Rab11A, Rab9A modulator, or Rab11A modulator protein (or for the pathogen protein) can be added to the system and allowed to bind to the complexed binding partners. The interaction between the Rab9A, Rab11A, Rab9A modulator, or Rab11A modulator protein and the pathogen protein can be detected by measuring the amount of label that remains associated with the glutathione-agarose beads. A successful reduction or inhibition of the interaction by the test compound will result in a decrease in detectable label.

Alternatively, the GST-Rab9A, GST-Rab11A, GST-Rab9A modulator, or GST-Rab11A modulator fusion protein, and the pathogen protein can be mixed together in liquid in the absence of the solid glutathione agarose beads. The test agent can be added either during or after the binding partners are allowed to interact. This mixture can then be added to the glutathione-agarose beads and unbound material is washed away. Again, the extent of inhibition of the binding partner interaction can be detected by adding the labeled antibody and measuring the label (such as the radioactivity) associated with the beads.

In another example, these same techniques can be employed using peptide fragments that correspond to the binding domain of the Rab9A protein, Rab11A, Rab9A modulator, the Rab11A modulator protein, or the pathogen protein, in place of one or more of the full length proteins. Any number of methods routinely practiced in the art can be used to identify and isolate a protein's binding site. These methods include, but are not limited to, mutagenesis of one of the genes encoding the proteins and screening for disruption of binding in a coimmunoprecipitation assay. Compensating mutations in a host gene can be selected. Sequence analysis of the genes encoding the respective proteins will reveal the mutations that correspond to the region of the protein involved in interactive binding. Alternatively, one protein can be anchored to a solid surface using methods described in above, and allowed to interact with and bind to its labeled binding partner, which has been treated with a proteolytic enzyme, such as trypsin. After washing, a short, labeled peptide including the binding domain may remain associated with the solid material, which can be isolated and identified by amino acid sequencing. Also, once the gene coding for the for the protein is obtained, short gene segments can be engineered to express peptide fragments of the protein, which can then be tested for binding activity and purified or synthesized.

For example, a Rab9A, Rab11A, a Rab9A modulator, or a Rab11A modulator protein can be anchored to a solid material as described above by making a GST-Rab9A, GST-Rab11A, GST-Rab9A modulator, or GST-Rab 11A modulator fusion protein and allowing it to bind to glutathione agarose beads. The pathogen protein can be labeled with a radioactive isotope, such as ³⁵S, and cleaved with a proteolytic enzyme such as trypsin. Cleavage products can then be added to the anchored GST-fusion protein and allowed to bind. After washing away unbound peptides, labeled bound material, representing the cellular or extracellular protein binding domain, can be eluted, purified, and analyzed for amino acid sequence. Peptides so identified can be produced synthetically or fused to appropriate facilitative proteins using recombinant DNA technology.

### Example 11

### Cell-Based Screening Assay for Agents that Decrease Infection

This example describes methods using intact cells that can be used to screen test agents for their ability to interfere with or even inhibit infection of a host cell by a pathogen, such as a pathogen that hijacks a lipid raft. For example, a yeast two-hybrid assay or the inverse two-hybrid assay method of Schreiber and coworkers (Proc. Natl. Acad Sci., USA 94:13396, 1977) can be used to screen for an agent that disrupts the association between a Rab9A, Rab11A, a Rab9A modulator, or a Rab11A modulator protein and a pathogen protein. Similar to Example 10, therapeutic agents identified by these approaches are tested for their ability to decrease or inhibit infection of a host cell, such as a human cell, by a pathogen.

In one example, the yeast two-hybrid system is used to identify anti-viral agents. One version of this system has been described (Chien et al., Proc. Natl. Acad. Sci. USA, 88:9578-82, 1991) and is commercially available from Clontech (Palo Alto, CA). Briefly, utilizing such a system, plasmids are constructed that encode two hybrid proteins: one includes the DNA-binding domain of a transcription activator protein fused to one test protein "X" and the other includes the activator protein's activation domain fused to another test protein "Y". Thus, either "X" or "Y" in this system can be a Rab9A, Rab11A, a Rab9A modulator, or a Rab11A modulator protein, while the other can be a test protein. The plasmids are transformed into a strain of *S. cerevisiae* that contains a reporter gene (such as lacZ) whose regulatory region contains the activator's binding sites. Either hybrid protein alone cannot activate transcription of the reporter gene, the DNA-binding domain hybrid because it does not provide activation function and the activation domain hybrid because it cannot localize to the activator's binding sites. Interaction of the two proteins reconstitutes the functional activator protein and results in expression of the reporter gene, which is detected by an assay for the reporter gene product.

The two-hybrid system or related methodology can be used to screen activation domain libraries for proteins that interact with a Rab9A, Rab11A, a Rab9A modulator, or a Rab11A modulator protein involved in pathogen infection. Total genomic or cDNA sequences are fused to the DNA encoding an activation domain. This library and a plasmid encoding a hybrid of the host protein involved in viral infection fused to the DNA-binding domain are cotransformed into a yeast reporter strain, and the resulting transformants are screened for those that express the reporter gene. These colonies are purified and the plasmids responsible for reporter gene expression are isolated. DNA sequencing is then used to identify the proteins encoded by the library plasmids.

For example, and not by way of limitation, a host gene encoding a Rab9A, Rab 11A, a Rab9A modulator, or a Rab11A modulator protein, can be cloned into a vector such that it is translationally fused to the DNA encoding the DNA-binding domain of the GAL4 protein. A cDNA library of the cell line from which proteins that interact with the Rab9A, Rab11A, a Rab9A modulator, or a Rab11A modulator protein are to be detected can be made using methods routinely practiced in the art. In this particular system, the cDNA fragments can be inserted into a vector such that they are translationally fused to the activation domain of GAL4. This library can be co-transformed along with the host-GAL4 DNA binding domain fusion plasmid into a yeast strain which contains a lacZ gene driven by a promoter which contains GAL4 activation sequences. A cDNA encoded protein, fused to GAL4 activation domain, that interacts with the Rab9A, Rab11A, a Rab9A modulator, or a Rab11A modulator protein will reconstitute an active GAL4 protein and thereby drive expression of the lacZ gene. Colonies which express lacZ can be detected by their blue color in the presence of X-gal. The cDNA can then be extracted from strains derived from these and used to produce and isolate the Rab9A, Rab11A, a Rab9A modulator, or a Rab11A modulator protein-interacting protein using techniques routinely practiced in the art.

### Example 12

### Rapid Screening Assays

Prior to performing any assays to detect interference with the association of a Rab9A, Rab11A, a Rab9A modulator, or a Rab11A modulator protein and a pathogen protein, rapid screening assays can be used to screen a large number of agents to determine if they bind to Rab9A, Rab11A, a Rab11A modulator, a Rab9A modulator or a pathogen protein. Rapid screening assays for detecting binding to HIV proteins have been disclosed, for example in U.S. Patent No. 5,230,998, which is incorporated by reference. In that assay, a Rab9 or Rab9 modulator protein or a pathogen protein, such as an HIV protein, is incubated with a first antibody capable of binding to Rab9 protein, Rab11A protein, a Rab9A modulator protein, a Rab11A modulator protein, or a pathogen protein, and the agent to be screened. Excess unbound first antibody is washed and removed, and antibody bound to the Rab9A, Rab11A, a Rab9A modulator, or a Rab11A modulator protein, or pathogen protein, is detected by adding a second labeled antibody which binds the first antibody. Excess unbound second antibody is then removed, and the amount of the label is quantitated. The effect of the binding effect is then determined in percentages by the formula: (quantity of the label in the absence of the test agent) - (quantity of the label in the presence of the test agent /quantity of the label in the absence of the test agent) x 100.

Agents that are found to have a high binding affinity to the Rab9A, Rab11A, a Rab9A modulator, a Rab11A modulator protein, or pathogen protein can then be used in other assays more specifically designed to test inhibition of the Rab9A, Rab11A, a Rab9A modulator, or a Rab11A modulator protein/pathogen protein interaction, or inhibition of pathogen infection.

### Example 13

### Identification of Modulators of Rab9A and Rab11

This example describes methods that can be used to identify a Rab9A modulator or Rab 11A modulator that is involved in infection of a cell by a pathogen, such as a pathogen that hijacks a lipid raft. Such identified modulators can be used to treat or prevent a pathogen infection, by administration of such modulators to a cell, such as administration to a subject.

In one example, expression of a putative Rab9A modulator or Rab11A modulator is decreased (such as inhibited) in a cell that expresses Rab9A, Rab11A, or both. Alternatively, the biological activity of the putative Rab9A or Rab11A modulator is decreased (such as inhibited) in a cell that expresses Rab9A, Rab11A, or both. Methods of decreasing expression or biological activity of a Rab9A modulator or Rab11A modulator are described throughout this application. The cell expressing Rab9A (or Rab11A, or both) but wherein at least one (such as at least two, at least three, or at least four) Rab9A modulator or Rab11A modulator is functionally deleted, is contacted with a desired pathogen, and an amount of infection by the pathogen determined. A decrease (and in some examples elimination) in infection indicates that the putative modulator of Rab9A or Rab11A is a Rab9A modulator or Rab11A modulator, respectively, involved in infection by a pathogen. The amount of infection can be compared to a control, such as an amount of infection by the pathogen of a cell not having reduced expression of a putative Rab9A modulator or Rab11A modulator. In one example, the pathogen is a pathogen that can hijack a lipid raft.

Any cell that can be infected with a virus or other pathogen, such as bacteria, protozoa, or fungi can be utilized. The cell can be prokaryotic or eukaryotic, such as a cell from an insect, crustacean, mammal, bird, reptile, yeast or a bacterium, such as *E. coli.* The cell can be part of an organism, or part of a cell culture, such as a culture of mammalian cells or a bacterial culture.

Methods for decreasing expression of the putative Rab9A modulator or Rab11A modulator are known in the art, and include, methods described above, such as functionally deleting the putative Rab9A modulator or Rab11A modulator. A decrease in expression need not be complete; a reduction of at least 10%, at least 50%, at least 90% or even at least 99% can be obtained. In one example, expression of a Rab9A or Rab11A modulator is inhibited by knocking out the Rab9A modulator or Rab11A modulator gene. Functional deletions can also be accomplished by mutation, partial or complete deletion, insertion or other variation to Rab9A modulator or Rab11A modulator gene sequence that reduces production of the gene product, or renders the gene product non-functional.

In one example, expression of a Rab9A modulator or Rab11A modulator is decreased by decreasing or disrupting transcription or translation of mRNA encoding the Rab9A modulator or Rab11A modulator, respectively. In one example, an siRNA, ribozyme, triple helix, or antisense molecule that is specific for the putative Rab9A modulator or Rab11A modulator mRNA is contacted with the cell and allowed to enter the cell, thereby reducing expression of the putative Rab9A modulator or Rab11A modulator and the amount of putative Rab9A or Rab11A modulator mRNA, respectively, in the cell.

The biological activity of the putative Rab9A modulator or Rab11A modulator can be reduced using methods known in the art, such as those described herein. For example, the biological activity of a Rab9A modulator or Rab11A modulator can be reduced by contacting the Rab9A modulator or Rab11A modulator in the cell with an agent that reduces the interaction (for example, a binding interaction) between the Rab9A modulator and Rab9A, an interaction between the Rab11A modulator and Rab11A, an interaction between a Rab11A modulator and a pathogen protein, or an interaction between a Rab9A modulator and a pathogen protein.

The method can further include associating the amount of infection with an amount of Rab9A (such as an amount of Rab9A nucleic acid molecule, for example cDNA or mRNA, or protein in the cell) or an amount of Rab9A biological activity in the cell, wherein the presence of a decrease in infection and a decrease in the amount of Rab9A or Rab9A activity indicates that the putative modulator of Rab9A is a modulator of Rab9A involved in infection by the pathogen. Similarly, the method can further include associating the amount of infection with an amount of Rab11A (such as an amount of Rab11A nucleic acid molecule, for example cDNA or mRNA, or protein in the cell) or an amount of Rab11A biological activity in the cell, wherein the presence of a decrease in infection and a decrease in the amount of Rab11A or Rab11A activity indicates that the putative modulator of Rab11A is a modulator of Rab11A involved in infection by the pathogen.

Methods for determining an amount of Rab9A or Rab11A protein in a cell are known in the art. Particular examples of methods that can be used to quantitate an amount of Rab9A or Rab11A protein in a cell include, but are not limited to, Western blotting, ELISA, ELISPOT, immunoprecipitation, immunofluorescence (such as flow cytometry or microscopy), immunohistochemistry, and immunocytochemistry. For example, cells can be lysed, and the resulting lysate incubated with a labeled anti-Rab9A antibody to permit binding of Rab9A proteins in the lysate with the antibody or with a labeled anti-Rab11A antibody to permit binding of Rab11A proteins in the lysate with the antibody. The label can be direct or indirect, such as through the use of a secondary antibody. Unbound anti-Rab9A or anti-Rab11A antibody is removed, and the remaining label detected.

Similarly, methods for determining an amount of Rab9A or Rab11A nucleic acid molecule in a cell are known in the art. Particular examples of methods that can be used to quantitate an amount of nucleic acid molecule in a cell include, but are not limited to, *in situ* hybridization, quantitative PCR, RT-PCR, Northern blotting, ELISPOT, and dot blotting. For example, nucleic acid molecules can be isolated from the cells, and incubated with Rab9A- or Rab11A- specific PCR primers and incubated under the appropriate PCR conditions. The resulting Rab9A- or Rab11A-amplicons, if any, can be detected and quantitated.

One of skill in the art can also assess the effect(s) of inhibiting a Rab9A modulator on Rab9A biological activity, for example by observing differences in vesicle transport from late endosomes to the trans-Golgi. Methods for visualizing this process are known in the art (for example, see Barbero et al. J. Cell. Biol. 156:511-8, 2002, herein incorporated by reference). Similarly, one of skill in the art can also assess the effect(s) of inhibiting a Rab11A modulator on Rab11A biological activity, for example by observing differences in vesicle transport from trans-Golgi to the plasma membrane. The interaction between Rab9A or Rab11A and other proteins can be direct or indirect. An example of a direct interaction is the binding of Rab9A or Rab11A to another protein. An example of an indirect interaction is the interaction between two or more proteins involved in vesicle transport or viral infection, either upstream or downstream of Rab9A or Rab11A that affect an activity of Rab9A or Rab11A, respectively, but do not bind directly to Rab9A or Rab11A, respectively.

### Example

### Assays for Measuring Inhibition of Infection

### Any of the test agents identified in the foregoing assay systems can be tested for their ability to decrease or inhibit infection by a pathogen, such as a pathogen that uses a lipid raft.

### Cell-based assays

Exemplary methods are provided in Examples 1 and 3 above. Briefly, cells (20,000 to 250,000) are infected with the desired pathogen (such as a pathogen that can hijack a lipid raft), and the incubation continued for 3-7 days. The test agent can be applied to the cells before, during, or after infection with the pathogen. The amount of pathogen and agent administered can be determined by skilled practitioners. In some examples, several different doses of the potential therapeutic agent can be administered, to identify optimal dose ranges. Following administration of the test agent, assays are conducted to determine the resistance of the cells to infection.

For example, if analyzing viral infection, the presence of a viral antigen can be determined by using antibody specific for the viral protein then detecting the antibody. In one example, the antibody that specifically binds to the viral protein is labeled, for example with a detectable marker such as a flurophore. In another example, the antibody is detected by using a secondary antibody containing a label. The presence of bound antibody is then detected, for example using microscopy, flow cytometry, or ELISA. Similar methods can be used to monitor bacterial, protozoal, or fungal infection (except that the antibody would recognize a bacterial, protozoal, or fungal protein, respectively).

The ability of the cells to survive in the presence of a pathogen can also be used as a measure of pathogen infection. For example, a cell viability assay, such as trypan blue exclusion, can be performed, wherein a decrease in cell viability indicates the presence of pathogen infection, and an increase in cell viability indicates a decrease in viral infection.

### Animal model assays

The ability of an agent, such as those identified using the methods provided above, to prevent or decrease infection by a pathogen, can be assessed in animal models. Several animal models for pathogen infection are known in the art. For example, mouse HIV models are disclosed in Sutton et al. (Res. Initiat Treat. Action, 8:22-4, 2003) and Pincus et al. (AIDS Res. Hum. Retroviruses 19:901-8, 2003); guinea pig models for Ebola infection are disclosed in Parren et al. (J. Virol. 76:6408-12, 2002) and Xu et al. (Nat. Med. 4:37-42, 1998); mouse models for Marburg infection are disclosed in (Ruchko et al., Vopr Virusol. 46: 21-4, 2001); and macaque models for measles infection are disclosed in Premenko-Lanier et al. (J. Infect. Dis. 189:2064-71, 2004). Such animal models can also be used to test agents for an ability to ameliorate symptoms associated with a pathogen infection. In addition, such animal models can be used to determine the LD50 and the ED50 in animal subjects, and such data can be used to determine the *in vivo* efficacy of potential agents.

Animals of any species, including, but not limited to, mice, rats, rabbits, guinea pigs, pigs, micro-pigs, goats, and non-human primates, such as baboons, monkeys, and chimpanzees, can be used to generate an animal model of pathogen infection if needed.

The appropriate animal is inoculated with the desired pathogen, in the presence or absence of the test agents identified in the examples above. The amount of pathogen and agent administered can be determined by skilled practitioners. In some examples, several different doses of the potential therapeutic agent can be administered to different test subjects, to identify optimal dose ranges. The therapeutic agent can be administered before, during, or after infection with the pathogen. Subsequent to the treatment, animals are observed for the development of the appropriate pathogen infection and symptoms associated therewith. A decrease in the development of the appropriate pathogen infection, or symptoms associated therewith, in the presence of the test agent provides evidence that the test agent is a therapeutic agent that can be used to decrease or even inhibit pathogen infection in a subject.

In view of the many possible embodiments to which the principles of the disclosed invention may be applied, it should be recognized that the illustrated embodiments are only preferred examples of the invention and should not be taken as limiting the scope of the invention. Rather, the scope of the invention is defined by the following claims. We therefore claim as our invention all that comes within the scope and spirit of these claims.

### Additional Statements of Invention

1. A method of decreasing infection of a host cell by a pathogen, comprising decreasing activity of Rab9A or Rab11A in the host cell, wherein decreasing activity of Rab9A or Rab11A decreases infection of the host cell by the pathogen.
2. The method of 1, wherein decreasing activity of Rab9A comprises decreasing activity of a Rab9A modulator that increases Rab9A activity or increasing activity of a Rab9A modulator that decreases Rab9A activity.
3. The method of 1, wherein decreasing activity of Rab11A comprises decreasing activity of a Rab11A modulator that increases Rab11A activity, or increasing activity of a Rab11A modulator that decreases Rab11A activity.
4. The method of 1, wherein the pathogen hijacks a lipid raft.
5. The method of 4, wherein the pathogen that pathogen hijacks a lipid raft is *Campylobacter jujuni, Vibrio cholerae,* SV40, *Legionella pneumophila, Aeromonas hydrophilia,* Echovirus 1, Echovirus 11, *Brucella* spp, *Clostridium* spp., Avian sarcoma and leukosis virus, *Escherichia coli, Streptcoccus pyogenes,* Semiliki forest virus, *Salmonella typhimurium, Bacillus anthracis,* Ecotropic mouse leukaemia virus, *Shigella flexneri, Bacillus thuringiensis,* HTLV-1, *Chlamydia* spp., *Helicobacter pylori,* HIV-1, *Mycobacterium* spp., *Lysteria monocytogenes, Ebola, Marburg,* Measles, Herpes Simplex virus, influenza virus, respiratory syncytia virus (RSV ), or Epstein-Barr virus.
6. The method of 4, wherein the pathogen that hijacks a lipid raft comprises an enveloped RNA virus.
7. The method of 6, wherein the enveloped RNA virus comprises human immunodeficiency virus (HIV)-1, HIV-2, Ebola virus, Marburg virus, RSV, or measles virus.
8. The method of 1, wherein decreasing activity of Rab9A or Rab11A comprises decreasing an amount of mRNA encoding Rab9A or Rab11A.
9. The method of 8, wherein decreasing an amount of mRNA encoding Rab9A comprises contacting the mRNA with any of SEQ ID NOS: 5-9, 16-32, or 47-66.
10. The method of 2, wherein the Rab9A modulator that increases Rab9A activity is a v-SNARE protein, a t-SNARE protein, a GDP dissociation inhibitor, a guanine-nucleotide exchange factor (GEF), a guanine-nucleotide displacement factor (GDF), mannose-6-phosphate receptor, TIP47, or a Rab9A effector.
11. The method of 10, wherein the Rab9 effector is p40.
12. The method of 10, wherein the GDF is Yip3.
13. The method of 2, wherein the Rab9A modulator that increases Rab9A activity is PIKfyve, TIP47, or p40.
14. The method of 3, wherein the Rab11A modulator that increases Rab11A activity is Sec15, Rip11, FIP1, FIP2, FIP3/eferin, FIP4, or Rab-coupling protein (RCP).
15. The method of 1, wherein Rab9A is encoded by a host nucleic acid comprising at least 90% sequence identity to a target nucleic acid sequence associated with SEQ ID NO: 1 or 2.
16. The method of 15, wherein the host nucleic acid comprises a target nucleic acid sequence associated with SEQ ID NO: 1 or 2.
17. The method of 2, wherein decreasing activity of Rab9A comprises decreasing activity of one or more Rab9A modulators that enhance Rab9A activity.
18. The method of 3, wherein decreasing activity of Rab11A comprises decreasing activity of one or more Rab11A modulators that enhance Rab11A activity.
19. The method of 1, wherein decreasing activity of Rab9A comprises decreasing activity of Rab9A and one or more Rab9A modulators that enhance Rab9A activity.
20. The method of 1, wherein decreasing activity of Rab11A comprises decreasing activity of Rab11A and one or more Rab11A modulators that enhance Rab11A activity.
21. The method of 1, wherein decreasing the activity of Rab9A or Rab11A comprises decreasing an interaction of a pathogen protein and a Rab9A protein, decreasing an interaction of a pathogen protein and a Rab11A protein, decreasing an interaction of a pathogen protein and a Rab9A modulator protein, or decreasing an interaction of a pathogen protein and a Rab11A modulator protein, by decreasing expression of the Rab9A protein, the Rab11A protein, the Rab9A modulator protein, or the Rab11A modulator protein.
22. The method of 21, wherein the pathogen protein comprises a virus and decreasing the interaction of the virus and the Rab9A protein, the Rab11A protein, the Rab9A modulator protein, or the Rab11A modulator protein, decreases infection of a host cell by the virus.
23. The method of 21, wherein decreasing expression of the Rab9A protein, the Rab11A protein, the Rab9A modulator protein, or the Rab11A modulator protein, comprises decreasing transcription of an mRNA encoding the Rab9A protein, the Rab11A protein, the Rab9A modulator protein, or the Rab11A modulator protein.
24. The method of 23, wherein decreasing transcription of the mRNA comprises inserting a transposon or insertional vector into a coding region of a nucleic acid sequence encoding the Rab9A protein, the Rab11A protein, the Rab9A modulator protein, or the Rab11A modulator protein.
25. The method of 23, wherein decreasing the transcription of the mRNA comprises contacting the mRNA with an antisense RNA, triple helix molecule, ribozyme, microRNA, or siRNA that recognizes the mRNA.
26. The method of 25, wherein the siRNA sequence comprises one or more of SEQ ID NOS: 5-9 and 16-32.
27. The method of 25, wherein the antisense RNA sequence comprises one or more of SEQ ID NOS: 47-56.
28. The method of 25, wherein the ribozyme RNA sequence comprises one or more of SEQ ID NOS: 57-66.
29. The method of 25, wherein the host cell is present in a subject, and contacting the mRNA with an antisense RNA, triple helix molecule, ribozyme, microRNA, or siRNA that recognizes the mRNA comprises administering the antisense RNA, triple helix molecule, ribozyme, microRNA, or siRNA to the subject.
30. The method of 21, wherein decreasing an interaction of a pathogen protein and the Rab9 protein, the Rab11A protein, the Rab9A modulator protein, or the Rab11A modulator protein, comprises contacting the host cell with an agent that decreases the activity of the Rab9A protein, the Rab11A protein, the Rab9A modulator protein, or the Rab11A modulator protein.
31. The method of 30, wherein the host cell is present in a host subject and wherein contacting the host cell with the agent comprises administering the agent to the subject.
32. The method of 30, wherein the agent is an anti-protein binding agent that specifically binds to the Rab9A protein, the Rab11A protein, the Rab9A modulator protein, or the Rab11A modulator protein, wherein the anti-protein binding agent decreases an interaction between the Rab9 protein, the Rab11A protein, the Rab9A modulator protein, or the Rab11A modulator protein, and the pathogen.
33. The method of 32, wherein the anti-protein binding agent is an antibody or chemical compound.
34. The method of 1, wherein the host cell is a mammalian host cell.
35. A method of treating a pathogen infection in a host subject, comprising:
   administering to a subject having a pathogen infection an effective amount of an agent that interferes with the interaction of a pathogen and host protein, wherein the host protein is Rab9A, Rab11A, a Rab9A modulator that enhances Rab9A activity, or a Rab11A modulator that enhances Rab11A activity.
36. The method of, 35, wherein the agent decreases expression of a nucleic acid sequence encoding Rab9A, Rab11A, a Rab9A modulator that enhances Rab9A activity, or a Rab11A modulator that enhances Rab11A activity.
37. The method of 35, wherein the agent is an antisense, triple helix molecule, ribozyme, microRNA, or siRNA molecule that recognizes a nucleic acid sequence encoding Rab9A, Rab11A, a Rab9A modulator that enhances Rab9A activity, or a Rab11A modulator that enhances Rab11A activity.
38. The method of claim 35, wherein the effective amount induces a prophylactic effect in the host, which decreases infection of the host by a pathogen.
39. The method of 35, wherein the host was previously infected by a pathogen and the effective amount induces a therapeutic effect in the host.
40. A method of identifying an agent that decreases pathogenicity of a pathogen, the method comprising:
   contacting a test agent with a Rab9A target or a Rab 11A target; and
   determining whether the test agent decreases Rab9A activity or Rab11A activity, wherein a decrease in Rab9A activity or Rab11A activity, indicates the agent decreases pathogenicity of the pathogen.
41. The method of 40, wherein the pathogen is a pathogen that hijacks a lipid raft.
42. The method of 41, wherein the pathogen that hijacks a lipid raft comprises *Campylobacter jujuni, Vibrio cholerae,* SV40, *Legionella pneumophila, Aeromonas hydrophilia,* Echovirus 1, Echovirus 11, *Brucella* spp, *Clostridium* spp., Avian sarcoma and leukosis virus, *Escherichia coli, Streptcoccus pyogenes,* Semiliki forest virus, *Salmonella typhimurium, Bacillus anthracis,* Ecotropic mouse leukaemia virus, *Shigella flexneri, Bacillus thuringiensis,* HTLV-1, *Chlamydia* spp., *Helicobacter pylori,* HIV-1, *Mycobacterium* spp., *Lysteria monocytogenes, Ebola, Marburg,* Measles, Herpes Simplex virus, influenza virus, respiratory syncytia virus (RSV ), or Epstein-Barr virus.
43. The method of 40, wherein determining whether the test agent decreases Rab9A activity or Rab11A activity comprises determining whether the test agent decreases expression in a cell of Rab9A, Rab11A, a Rab9A modulator that affects Rab9A activity, or a Rab11A modulator that affects Rab11A activity.
44. The method of 43, wherein determining whether the test agent decreases expression of Rab9A, Rab11A, a Rab9A modulator that affects Rab9A activity, or a Rab11A modulator that affects Rab11A activity, comprises determining whether the test agent decreases a level of Rab9A mRNA, Rab11A mRNA, a Rab9A modulator mRNA that affects Rab9A activity, or a Rab11A mRNA modulator that affects Rab11A activity in the cell.
45. The method of 40, wherein the pathogen comprises a virus, bacterium or protozoa.
46. The method of 45, wherein the pathogen comprises a virus.
47. The method of 46, wherein the virus includes a viral envelope protein.
48. The method of 46 wherein the virus comprises and enveloped RNA virus.
49. A cell comprising a functional deletion of a Rab9A gene, Rab11A gene, or a modulator gene thereof that increases Rab9A or Rab11A activity, wherein the cell has a decreased susceptibility to infection by a pathogen that hijacks a lipid raft.
50. A non-human transgenic mammal comprising a functional deletion of a Rab9A gene, a Rab11A gene, or a modulator gene thereof that increases Rab9A or Rab11A activity, wherein the mammal has decreased suseptibility to infection by a pathogen that hijacks a lipid raft.
51. A method of identifying a modulator of Rab9A or Rab11A involved in infection by a pathogen that hijacks a lipid raft, comprising:
   reducing expression of a putative Rab9A or Rab11A modulator in a cell that expresses Rab9A or Rab11A;
   contacting the cell with a pathogen that hijacks a lipid raft; and
   detecting an amount of infection by the pathogen that hijacks a lipid raft, wherein a decrease in infection indicates that the putative Rab9A or Rab 11A modulator is a Rab9A or Rab11A modulator, respectively, involved in infection by the pathogen that hijacks a lipid raft.
52. The method of 51, further comprising associating the amount of infection with an amount of Rab9A or Rab11A nucleic acid molecule, protein, or activity, wherein the presence of a decrease in infection and a decrease in the amount of Rab9A or Rab11A indicates that the putative modulator of Rab9A or Rab11A is a modulator of Rab9A or Rab11A, respectively, involved in infection by the pathogen that hijacks a lipid raft.
53. The method of 51, wherein decreasing expression of the putative modulator of Rab9A or Rab11A comprises decreasing an amount of mRNA encoding the putative modulator of Rab9A or Rab11A.
54. A method of determining resistance or susceptibility to pathogen infection in a subject, comprising comparing a first nucleic acid sequence of a subject to a second nucleic acid sequence comprising a Rab9A, Rab11A, a Rab9A modulator, or a Rab11A modulator sequence, wherein a higher similarity between the first and second nucleic acid sequence indicates the subject is more susceptible to pathogen infection, and wherein a lesser similarity between the first and second nucleic acid sequence indicates the subject is more resistant to pathogen infection.
55. The method of 54, wherein the first nucleic acid sequence is obtained from a biological sample of the subject.
56. The method of 54, wherein the first nucleic acid sequence comprises a plurality of nucleic acid sequences, wherein each nucleic acid sequence is obtained from a different subject.
57. The method of 56, further comprising determining a polymorphic variation within a population.
58. An isolated nucleic acid sequence comprising any nucleic acid sequence shown in SEQ ID NOS: 5-9, 16-32, or 47-66.
59. A vector comprising the isolated nucleic acid sequence of 58.

## Claims

1. A composition that decreases activity or expression of Rab11A for use in a method of decreasing infection of a host cell by a pathogen, comprising decreasing activity or expression of Rab11A in the host cell, wherein decreasing activity or expression of Rab11A decreases infection of the host cell by the pathogen.

2. Use of a composition that decreases activity or expression of Rab11A in the manufacture of a medicament for decreasing infection of a host cell by a pathogen by decreasing activity or expression of Rab 11 A in the host cell, wherein decreasing activity or expression of Rab11A decreases infection of the host cell by the pathogen.

3. The composition of claim 1 or use according to claim 2, wherein the composition decreases activity or expression of a Rab11A modulator that increases Rab11A activity, or increases activity or expression of a Rab11A modulator that decreases Rab11A activity.

4. The composition of claim 1 or use according to claim 2, wherein the pathogen hijacks a lipid raft, such as a lipid raft comprising an enveloped RNA virus.

5. The composition of claim 1 or use according to claim 2, wherein the composition decreases an amount of mRNA encoding Rab11A.

6. The composition of claim 1 or use according to claim 2, wherein the composition decreases an interaction of a pathogen protein and a Rab11A protein, or decreases an interaction of a pathogen protein and a Rab11A modulator protein, by decreasing expression of the Rab11A protein or the Rab11A modulator protein, wherein the pathogen comprises a virus.

7. The composition or use according to claim 6, wherein decreasing expression of the Rab11A protein or the Rab11A modulator protein comprises decreasing or disrupting transcription of an mRNA encoding the Rab 11A protein or the Rab 11A modulator protein, for example by decreasing or disrupting transcription of the mRNA with a transposon or insertional vector inserted into a coding region of a nucleic acid sequence encoding the Rab11A protein or the Rab11A modulator protein, such as wherein decreasing or disrupting the transcription of the mRNA comprises contacting the mRNA with an antisense RNA, triple helix molecule, ribozyme, microRNA, or siRNA that recognizes the mRNA.

8. The composition or use according to claim 7, wherein the siRNA sequence comprises one or more of SEQ ID NOS: 16-19, the antisense RNA sequence comprises one or more of SEQ ID NOS: 49-50, and the ribozyme RNA sequence comprises one or more of SEQ ID NOS: 59-60.

9. The composition or use according to claim 7, wherein the host cell is present in a subject, and the mRNA is contacted with an antisense RNA, triple helix molecule, ribozyme, microRNA, or siRNA that recognizes the mRNA comprises administering the antisense RNA, triple helix molecule, ribozyme, microRNA, or siRNA to the subject.

10. The composition or use according to any of claims 1-9, wherein the host cell is present in a host subject.

11. A method of identifying an agent that decreases pathogenicity of a pathogen, the method comprising:
contacting a test agent with a Rab11A target; and
determining whether the test agent decreases Rab 11 A activity or expression, wherein a decrease in Rab11A activity or expression as compared to Rab11A activity or expression in the absence of the test agent, indicates the agent decreases pathogenicity of the pathogen.

12. The method of claim 12, wherein the pathogen comprises a bacterium protozoan, virion or viral protein, such as a viral envelope protein or an enveloped RNA virus.

13. A non-human transgenic mammal comprising a functional deletion of a Rab11A gene or a Rab11A modulator gene thereof that increases Rab11A activity, wherein the mammal has decreased susceptibility to infection by a pathogen that hijacks a lipid raft.

14. A method of identifying a modulator of Rab11A involved in infection by a pathogen that hijacks a lipid raft, comprising:
reducing expression of a putative Rab11A modulator in a cell that expresses Rab11A;
contacting the cell with a pathogen that hijacks a lipid raft; and
detecting an amount of infection by the pathogen that hijacks a lipid raft, wherein a decrease in infection indicates that the putative Rab11A modulator is a Rab11A modulator involved in infection by the pathogen that hijacks a lipid raft.

15. The method of claim 14, further comprising associating the amount of infection with an amount of Rab11A nucleic acid molecule, protein, or activity, wherein the presence of a decrease in infection and a decrease in the amount of Rab11A indicates that the putative modulator of Rab11A is a modulator of Rab11A involved in infection by the pathogen that hijacks a lipid raft.

16. A method of determining resistance or susceptibility to pathogen infection in a subject, comprising comparing a first nucleic acid sequence of a subject to a second nucleic acid sequence comprising Rab11A or a Rab11A modulator sequence, wherein a higher similarity between the first and second nucleic acid sequence indicates the subject is more susceptible to pathogen infection, and wherein a lesser similarity between the first and second nucleic acid sequence indicates the subject is more resistant to pathogen infection.

17. A composition that decreases activity or expression of a Rab9A modulator for use in a method of decreasing infection of a host cell by a pathogen, by decreasing activity or expression of a Rab9A modulator in the host cell, wherein decreasing activity or expression of Rab9A modulator decreases infection of the host cell by the pathogen.

18. The composition of claim 17, wherein the Rab9A modulator is selected from the group consisting of PIKfyve, p40, TIP47, GDF, Yip3, TSG101, v-SNARE, mannose-6-phosphate receptor, mapmodulin, syntaxin 11, a t-SNARE protein, a GDP dissociation inhibitor and a GEF.

19. An isolated nucleic acid sequence consisting of any nucleic acid sequence shown in SEQ ID NOS: 16-32, 49-56, or 59-66.

20. A vector comprising the isolated nucleic acid sequence of claim 19.
